# EUROPEAN PATENT APPLICATION

(11) **EP 4 458 968 A1**
(43) Date of publication of application: **06.11.2024**
(21) Application number: 23171754.7
(22) Date of filing: 05.05.2023
(51) Int. Cl.: C12N 15/11, C12N 15/113

(54) **CIRCULAR NUCLEIC ACIDS AND USES THEREOF FOR SHAPING THE CELLULAR PROTEOME**

(71) Applicant: Justus-Liebig-Universität Gießen, 35390 Gießen (DE)
(72) Inventor: Bindereif, Albrecht, 35394 Gießen (DE); Schreiner, Silke, 35510 Butzbach (DE); Pfafenrot, Christina, 55128 Mainz (DE)
(74) Representative: Stumpf, Peter

(57) **Abstract**

The present invention relates to polynucleotides, preferably circular polynucleotides, pharmaceutical compositions and the use thereof in methods for shaping the cellular proteome. The polynucleotides comprise a nucleotide sequence, which sequence comprises a targeting region having a nucleotide sequence complementary to a U1 snRNA or a 5' untranslated region (5'-UTR) or to a respective portion thereof. In particular, the polynucleotides are capable of influencing pre-mRNA splicing or mRNA translation.

## Description

### INTRODUCTION

The present invention relates to polynucleotides, *e.g.*, circular polynucleotides, compositions and the use thereof in methods for shaping the cellular proteome. The polynucleotides comprise a nucleotide sequence, which sequence comprises a targeting region having a nucleotide sequence complementary to a U1 snRNA or a 5' untranslated region (5'-UTR). In particular, the polynucleotides are involved in pre-mRNA splicing or in mRNA translation.

### BACKGROUND OF THE INVENTION

Antisense approaches have been investigated for the last 30 years (for reviews, see Bennett, C.F. and Swayze, E.E., Annu. Rev. Pharmacol. Toxicol. 50 (2010): 259-293; Crooke, S.T., et al., Cell Metab. 27 (2018): 714-739; Bennett, C.F., et al., Annu. Rev. Neurosci, 42 (2019): 385-406). By targeting specific RNA sequences, antisense approaches aim to modulate RNA structure and activity, mRNA splicing, translation, or stability. Best known examples are antisense oligonucleotides (ASO), such as locked nucleic acids (LNA), so-called gapmers, morpholino or other modifications that are introduced to increase RNA base-pairing and/or metabolic stability. As a result of antisense research over several decades, ASO-based therapies have been advanced to the stage of approved drugs used in certain genetic diseases.

Circular RNAs are a novel class of RNAs, which are generated from pre-mRNAs by circular splicing (also termed "back splicing") of one or several adjacent exons (Wilusz, J.E., et al., Wiley Interdiscip Rev RNA 9(2018): e1478). Circular RNAs are expressed in a cell-type specific manner, are much more stable than the corresponding linear mRNA, evolutionarily conserved, and are mostly cytoplasmic. Although present in all eukaryotes investigated so far, circular RNAs are still largely undefined in functional terms, except for development of artificial miRNA sponges (Jost, I., et al., RNA Biol. 15 (2018): 1032-1039), protein sponges for hnRNP L and IMP3 (Schreiner, S., et al., Nucleic Acids Res. 48 (2020): 12326-12335), and designer antisense circRNAs for interfering with SARS-CoV-2 proliferation (Pfafenrot, C., et al., Nucleic Acids Res. 49 (2021): 12502-12516).

Despite impressive benefits associated with antisense approaches, there is still a need for the development of improved antisense polynucleotides capable of shaping the cellular proteome.

### SUMMARY OF THE INVENTION

The present inventors surprisingly found that polynucleotides targeting a U1 snRNA, or a 5'-untranslated region (also referred to as 5'-UTR herein) of an mRNA, in particular polynucleotides targeting a 5'-UTR of a human β-globin (HBB) mRNA or a 5'-UTR of the mouse double minute 2 homolog (MDM2) mRNA, are particularly suitable for improved antisense approaches. Preferably, the targeting region comprising or consisting of a nucleotide sequence complementary to the U1 snRNA or to a portion thereof, or complementary to the 5'-UTR or to a portion thereof is presented by a circular RNA (also referred to as circRNA herein), thereby exploiting the unusual metabolic stability of circular RNAs.

In one aspect, the invention therefore relates to a polynucleotide comprising a nucleotide sequence, which sequence comprises a targeting region comprising or consisting of a nucleotide sequence complementary to a target structure which is involved in shaping the cellular proteome, wherein the target structure is involved in pre-mRNA splicing and the polynucleotide is capable of at least one of:
(i) inhibiting pre-mRNA splicing;
(ii) resulting in intron retention;
(iii) resulting in alternative 5' splice site use; and/or
(iv) resulting in single-exon skipping, and
the nucleotide sequence comprises a targeting region comprising or consisting of a nucleotide sequence complementary to a U1 snRNA or to a continuous or discontinuous portion of said U1 snRNA.

In one embodiment, the polynucleotide is a circular polynucleotide.

In one embodiment of the polynucleotide, the nucleotide sequence comprises a targeting region comprising or consisting of a nucleotide sequence complementary to a single-stranded region in the secondary structure, *e*.*g*., in a loop sequence, of a U1 snRNA or to a continuous or discontinuous portion of said U1 snRNA.

In one embodiment of the polynucleotide, the nucleotide sequence of the targeting region is complementary to at least nucleotides 1 to 20, starting from the 5' end of the U1 snRNA nucleotide sequence.

For increasing specificity, efficiency and/or flexibility of the polynucleotide, in one embodiment, the targeting region of the polynucleotide may be flanked at the 5' end, relative to the 5' terminal nucleotide, of the nucleotide sequence of the targeting region. The flanking region may comprise one or more additional nucleotides, wherein each additional nucleotide may be complementary or non-complementary to the corresponding nucleotide of the nucleotides flanking the portion of the U1 snRNA to which the nucleotide sequence of the targeting region is complementary.

In one embodiment of the polynucleotide, the nucleotide sequence of the targeting region complementary to at least the nucleotides 1 to 20 is flanked 5' by one or more additional nucleotides for targeting the nucleotide at position 21 up to at most to the nucleotide at position 70 of the U1 snRNA nucleotide sequence. In one embodiment of the polynucleotide, the nucleotide sequence of the targeting region complementary to at least the nucleotides 1 to 20 is flanked 5' by one or more additional nucleotides for targeting the nucleotide at position 21 up to at most to the nucleotide at position 50 of the U1 snRNA nucleotide sequence. In one embodiment of the polynucleotide, the nucleotide sequence of the targeting region complementary to at least the nucleotides 1 to 20 is flanked 5' by one or more additional nucleotides for targeting the nucleotide at position 21 up to at most to the nucleotide at position 42 of the U1 snRNA nucleotide sequence. In one embodiment of the polynucleotide, the nucleotide sequence of the targeting region complementary to at least the nucleotides 1 to 20 is flanked 5' by one or more additional nucleotides for targeting the nucleotide at position 21 up to at most to the nucleotide at position 30 of the U1 snRNA nucleotide sequence. Each nucleotide added 5' may be complementary or non-complementary to the corresponding nucleotide of the nucleotides flanking the portion of the U1 snRNA to which the nucleotide sequence of the targeting region is complementary.

In one embodiment of the polynucleotide, the nucleotide sequence complementary to a U1 snRNA or to a continuous or discontinuous portion of said U1 snRNA is complementary to at least one continuous portion of the U1 snRNA, the continuous portion being selected from the group consisting of:
(i) nucleotides 1 to 20 of the nucleotide sequence of the U1 snRNA;
(ii) nucleotides 1 to 22 of the nucleotide sequence of the U1 snRNA;
(iii) nucleotides 1 to 25 of the nucleotide sequence of the U1 snRNA;
(iv) nucleotides 1 to 26 of the nucleotide sequence of the U1 snRNA;
(v) nucleotides 1 to 30 of the nucleotide sequence of the U1 snRNA;
(vi) nucleotides 1 to 40 of the nucleotide sequence of the U1 snRNA;
(vii) nucleotides 1 to 42 of the nucleotide sequence of the U1 snRNA; and
(viii) nucleotides 1 to 50 of the nucleotide sequence of the U1 snRNA,
each starting from the 5' end of the U1 snRNA nucleotide sequence.

In one embodiment of the polynucleotide, the nucleotide sequence of the targeting region is depicted in any one of SEQ ID NO: 3 to SEQ ID: 6. In one embodiment, the nucleotide sequence of the targeting region of the polynucleotide is a variant of any of the sequences depicted in any one of SEQ ID NO: 3 to SEQ ID: 6.

Alternatively, or in addition to the above embodiments of the polynucleotide, in a further embodiment the targeting region of the polynucleotide comprises two or more copies of a nucleotide sequence complementary to a U1 snRNA or to a continuous or discontinuous portion of said U1 snRNA. In one embodiment of the polynucleotide, the targeting region of the polynucleotide comprises two or three copies of the nucleotide sequence complementary to a U1 snRNA or to a continuous or discontinuous portion of said U1 snRNA.

In one embodiment of the polynucleotide, the nucleotide sequence to be copied comprises a targeting region comprising or consisting of a nucleotide sequence complementary to a single-stranded region in the secondary structure, *e*.*g*., in a loop sequence, of a U1 snRNA or to a continuous or discontinuous portion of said U1 snRNA.

In one embodiment of the polynucleotide, the nucleotide sequence to be copied comprises a nucleotide sequence which is complementary to at least nucleotides 1 to 20 of the U1 snRNA nucleotide sequence, starting from the 5' end of the U1 snRNA nucleotide sequence.

In one embodiment of the polynucleotide, the nucleotide sequence complementary to at least the nucleotides 1 to 20 is flanked 5' by one or more additional nucleotides for targeting the nucleotide at position 21 up to at most to the nucleotide at position 50 of the U1 snRNA nucleotide sequence. Each nucleotide added 5' may be complementary or non-complementary to the corresponding nucleotide of the nucleotides flanking the portion of the U1 snRNA to which the nucleotide sequence of the targeting region is complementary.

In one embodiment of the polynucleotide, the nucleotide sequence to be copied comprises a nucleotide sequence which is complementary to at least nucleotides 1 to 25 of the U1 snRNA nucleotide sequence.

In one embodiment of the polynucleotide, the nucleotide sequence complementary to at least the nucleotides 1 to 25 is flanked 5' by one or more additional nucleotides for targeting the nucleotide at position 26 up to at most to the nucleotide at position 50 of the U1 snRNA nucleotide sequence. Each nucleotide added 5' may be complementary or non-complementary to the corresponding nucleotide of the nucleotides flanking the portion of the U1 snRNA to which the nucleotide sequence of the targeting region is complementary.

In one embodiment of the polynucleotide, the nucleotide sequence to be copied is complementary to at least one continuous portion of the U1 snRNA, the continuous portion being selected from the group consisting of:
(i) nucleotides 1 to 20 of the nucleotide sequence of the U1 snRNA;
(ii) nucleotides 1 to 22 of the nucleotide sequence of the U1 snRNA;
(iii) nucleotides 1 to 25 of the nucleotide sequence of the U1 snRNA;
(iv) nucleotides 1 to 26 of the nucleotide sequence of the U1 snRNA;
(v) nucleotides 1 to 30 of the nucleotide sequence of the U1 snRNA;
(vi) nucleotides 1 to 40 of the nucleotide sequence of the U1 snRNA;
(vii) nucleotides 1 to 42 of the nucleotide sequence of the U1 snRNA; and
(viii) nucleotides 1 to 50 of the nucleotide sequence of the U1 snRNA,
each starting from the 5' end of the U1 snRNA nucleotide sequence.

In one embodiment of the polynucleotide, the nucleotide sequence of the continuous portion to be copied is depicted in any one of SEQ ID NO: 3 to SEQ ID: 6. In one embodiment, the nucleotide sequence of the continuous portion to be copied of the polynucleotide is a variant of any of the sequences depicted in any one of SEQ ID NO: 3 to SEQ ID: 6.

In one embodiment of the polynucleotide, the targeting region comprises two or three copies of a continuous portion selected from the group consisting of:
(i) nucleotides 1 to 20 of the nucleotide sequence of the U1 snRNA;
(ii) nucleotides 1 to 22 of the nucleotide sequence of the U1 snRNA;
(iii) nucleotides 1 to 25 of the nucleotide sequence of the U1 snRNA;
(iv) nucleotides 1 to 26 of the nucleotide sequence of the U1 snRNA;
(v) nucleotides 1 to 30 of the nucleotide sequence of the U1 snRNA;
(vi) nucleotides 1 to 40 of the nucleotide sequence of the U1 snRNA;
(vii) nucleotides 1 to 42 of the nucleotide sequence of the U1 snRNA; and
(viii) nucleotides 1 to 50 of the nucleotide sequence of the U1 snRNA,
each starting from the 5' end of the U1 snRNA nucleotide sequence.

In one embodiment of the polynucleotide, between the nucleotides of the copied continuous portions a non-complementary sequence of two or more nucleotides is inserted. The noncomplementary sequence may be independently at least 1, at least 2 or at least 3 nucleotides in length, and/or may be independently at most 20, at most 15, at most 12, at most 10 or at most 8 nucleotides in length. For example, each inserted nucleotide sequence may be 1 to 10 or 2 to 8 nucleotides in length. The inserted nucleotide sequence may be composed of adenine nucleotides or modified adenine nucleotides and thymine/uracil nucleotides or modified thymine/uracil nucleotides.

In one embodiment of the polynucleotide, the nucleotide sequence of the targeting region is depicted in any one of SEQ ID NO: 7 to SEQ ID: 9. In one embodiment, the nucleotide sequence of the targeting region is a variant of any of the sequences depicted in any one of SEQ ID NO: 7 to SEQ ID: 9.

In one embodiment, the nucleotide sequence of the U1snRNA is depicted in SEQ ID NO: 1, which sequence shows a human U1snRNA. In one embodiment, the U1snRNA is a variant of the sequence depicted in SEQ ID NO: 1.

In one aspect, the invention relates to a polynucleotide comprising a nucleotide sequence, which sequence comprises a targeting region comprising or consisting of a nucleotide sequence complementary to a target structure which is involved in shaping the cellular proteome, wherein the target structure is involved in mRNA translation and the polynucleotide is capable of inhibiting mRNA translation, and the nucleotide sequence comprises a targeting region comprising or consisting of a nucleotide sequence complementary to a 5' untranslated region (5'-UTR) or to a continuous or discontinuous portion of said 5'-UTR.

In one embodiment, the polynucleotide is a linear, preferably circular polynucleotide.

In one embodiment of the polynucleotide, the nucleotide sequence of the targeting region is complementary to a 5'-UTR or to a continuous or discontinuous portion of said 5'-UTR of the mouse double minute 2 homolog (MDM2) mRNA.

In one embodiment, the nucleotide sequence of the 5'-untranslated region is depicted in SEQ ID NO: 10, which sequence shows the 5'-UTR of isoforms 1 and 2 of MDM2. In one embodiment, the 5'-UTR as a variant of the sequence depicted in SEQ ID NO: 10.

In one embodiment of the polynucleotide, the nucleotide sequence of the targeting region is complementary to nucleotides 22 to 40 of the sequence depicted in SEQ ID NO: 10 or to a continuous or discontinuous portion of said sequence.

For increasing specificity, efficiency and/or flexibility of the polynucleotide, in one embodiment, the targeting region of the polynucleotide may be flanked on one or both ends by a flanking region. Each flanking region may comprise one or more additional nucleotides, wherein each additional nucleotide may be complementary or non-complementary to the corresponding nucleotide of the nucleotides flanking the portion of the 5'-UTR to which the nucleotide sequence of the targeting region is complementary. Flanking on both ends means 3' and 5', relative to the 3' terminal nucleotide or the 5' terminal nucleotide, respectively, of the nucleotide sequence of the targeting region.

In one embodiment of the polynucleotide, the nucleotide sequence of the targeting region complementary to at least nucleotides 22 to 40 of the sequence depicted in SEQ ID NO: 10 is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.

In one embodiment of the polynucleotide, the nucleotide sequence of the targeting region complementary to at least nucleotides 22 to 40 of the sequence depicted in SEQ ID NO: 10 is flanked 3' by one or more additional nucleotides for targeting the nucleotide at position 21 up to at most to the nucleotide at position 1 of the sequence depicted in SEQ ID NO: 10. Each nucleotide added 3' may be complementary or non-complementary to the corresponding nucleotide of the nucleotides flanking the portion of the 5'-UTR to which the nucleotide sequence of the targeting region is complementary.

In one embodiment, the nucleotide sequence of the targeting region complementary to at least nucleotides 22 to 40 of the sequence depicted in SEQ ID NO: 10 is flanked 5' by one or more additional nucleotides for targeting the nucleotide at position 41 up to at most to the nucleotide at position 50 of the sequence depicted in SEQ ID NO: 10. Each nucleotide added 5' may be complementary or non-complementary to the corresponding nucleotide of the nucleotides flanking the portion of the 5'-UTR to which the nucleotide sequence of the targeting region is complementary.

In one embodiment of the polynucleotide, the nucleotide sequence of the targeting region is complementary to nucleotides 67 to 86 of the sequence depicted in SEQ ID NO: 10 or to a continuous or discontinuous portion of said sequence.

In one embodiment of the polynucleotide, the nucleotide sequence of the targeting region complementary to at least nucleotides 67 to 86 of the sequence depicted in SEQ ID NO: 10 is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.

In one embodiment of the polynucleotide, the nucleotide sequence of the targeting region complementary to at least nucleotides 67 to 86 of the sequence depicted in SEQ ID NO: 10 is flanked 3' by one or more additional nucleotides for targeting the nucleotide at position 66 up to at most to the nucleotide at position 47 of the sequence depicted in SEQ ID NO: 10. In one embodiment of the polynucleotide, the nucleotide sequence of the targeting region complementary to at least nucleotides 67 to 86 of the sequence depicted in SEQ ID NO: 10 is flanked 3' by one or more additional nucleotides for targeting the nucleotide at position 66 up to at most to the nucleotide at position 57 of the sequence depicted in SEQ ID NO: 10. Each nucleotide added 3' may be complementary or non-complementary to the corresponding nucleotide of the nucleotides flanking the portion of the 5'-UTR to which the nucleotide sequence of the targeting region is complementary.

In one embodiment, the nucleotide sequence of the targeting region complementary to at least nucleotides 67 to 86 of the sequence depicted in SEQ ID NO: 10 is flanked 5' by one or more additional nucleotides for targeting the nucleotide at position 87 up to at most to the nucleotide at position 106 of the sequence depicted in SEQ ID NO: 10. In one embodiment, the nucleotide sequence of the targeting region complementary to at least nucleotides 67 to 86 of the sequence depicted in SEQ ID NO: 10 is flanked 5' by one or more additional nucleotides for targeting the nucleotide at position 87 up to at most to the nucleotide at position 96 of the sequence depicted in SEQ ID NO: 10. Each nucleotide added 5' may be complementary or non-complementary to the corresponding nucleotide of the nucleotides flanking the portion of the 5'-UTR to which the nucleotide sequence of the targeting region is complementary.

In one embodiment of the polynucleotide, the nucleotide sequence of the targeting region is complementary to nucleotides 138 to 157 of the sequence depicted in SEQ ID NO: 10 or to a continuous or discontinuous portion of said sequence.

In one embodiment of the polynucleotide, the nucleotide sequence of the targeting region complementary to at least nucleotides 138 to 157 of the sequence depicted in SEQ ID NO: 10 is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.

In one embodiment of the polynucleotide, the nucleotide sequence of the targeting region complementary to at least nucleotides 138 to 157 of the sequence depicted in SEQ ID NO: 10 is flanked 3' by one or more additional nucleotides for targeting the nucleotide at position 137 up to at most to the nucleotide at position 118 of the sequence depicted in SEQ ID NO: 10. In one embodiment of the polynucleotide, the nucleotide sequence of the targeting region complementary to at least nucleotides 138 to 157 of the sequence depicted in SEQ ID NO: 10 is flanked 3' by one or more additional nucleotides for targeting the nucleotide at position 137 up to at most to the nucleotide at position 128 of the sequence depicted in SEQ ID NO: 10. Each nucleotide added 3' may be complementary or non-complementary to the corresponding nucleotide of the nucleotides flanking the portion of the 5'-UTR to which the nucleotide sequence of the targeting region is complementary.

In one embodiment, the nucleotide sequence of the targeting region complementary to at least nucleotides 138 to 157 of the sequence depicted in SEQ ID NO: 10 is flanked 5' by one or more additional nucleotides for targeting the nucleotide at position 158 up to at most to the nucleotide at position 177 of the sequence depicted in SEQ ID NO: 10. In one embodiment, the nucleotide sequence of the targeting region complementary to at least nucleotides 138 to 157 of the sequence depicted in SEQ ID NO: 10 is flanked 5' by one or more additional nucleotides for targeting the nucleotide at position 158 up to at most to the nucleotide at position 167 of the sequence depicted in SEQ ID NO: 10. Each nucleotide added 5' may be complementary or non-complementary to the corresponding nucleotide of the nucleotides flanking the portion of the 5'-UTR to which the nucleotide sequence of the targeting region is complementary.

In one embodiment of the polynucleotide, the nucleotide sequence of the targeting region is complementary to nucleotides 301 to 318 of the sequence depicted in SEQ ID NO: 10 or to a continuous or discontinuous portion of said sequence.

In one embodiment of the polynucleotide, the nucleotide sequence of the targeting region complementary to at least nucleotides 301 to 318 of the sequence depicted in SEQ ID NO: 10 is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.

In one embodiment of the polynucleotide, the nucleotide sequence of the targeting region complementary to at least nucleotides 301 to 318 of the sequence depicted in SEQ ID NO: 10 is flanked 3' by one or more additional nucleotides for targeting the nucleotide at position 300 up to at most to the nucleotide at position 269 of the sequence depicted in SEQ ID NO: 10. In one embodiment of the polynucleotide, the nucleotide sequence of the targeting region complementary to at least nucleotides 301 to 318 of the sequence depicted in SEQ ID NO: 10 is flanked 3' by one or more additional nucleotides for targeting the nucleotide at position 300 up to at most to the nucleotide at position 279 of the sequence depicted in SEQ ID NO: 10. Each nucleotide added 3' may be complementary or non-complementary to the corresponding nucleotide of the nucleotides flanking the portion of the 5'-UTR to which the nucleotide sequence of the targeting region is complementary.

In one embodiment, the nucleotide sequence of the targeting region complementary to at least nucleotides 301 to 318 of the sequence depicted in SEQ ID NO: 10 is flanked 5' by one or more additional nucleotides for targeting the nucleotide at position 319 up to at most to the nucleotide at position 324 of the sequence depicted in SEQ ID NO: 10. Each nucleotide added 5' may be complementary or non-complementary to the corresponding nucleotide of the nucleotides flanking the portion of the 5'-UTR to which the nucleotide sequence of the targeting region is complementary.

In one embodiment of the polynucleotide, the nucleotide sequence of the targeting region is complementary to at least one continuous portion of the 5'-UTR, the continuous portion being selected from the group consisting of:
(i) nucleotides 1 to 40 of the sequence depicted in SEQ ID NO: 10;
(ii) nucleotides 57 to 96 of the sequence depicted in SEQ ID NO: 10;
(iii) nucleotides 128 to 167 of the sequence depicted in SEQ ID NO: 10
(iv) nucleotides 279 to 318 of the sequence depicted in SEQ ID NO: 10; and
(v) nucleotides 301 to 324 of the sequence depicted in SEQ ID NO: 10.

In one embodiment, the continuous portion of the 5'-UTR is variant of any one of these portions under (i) to (v).

In one embodiment of the polynucleotide, the nucleotide sequence of the targeting region is depicted in any one of SEQ ID NO: 11 to SEQ ID NO: 31. In one embodiment, the nucleotide sequence of the targeting region of the polynucleotide is a variant of any of the sequences depicted in any one of SEQ ID NO: 11 to SEQ ID NO: 31.

In one embodiment of the polynucleotide, the nucleotide sequence of the targeting region comprises the sequences as depicted in SEQ ID NO: 31, wherein the sequence is additionally flanked 5' in a contiguous manner by one or more nucleotides for targeting nucleotide +1 up to at most nucleotide +26, preferably up to at most nucleotide +16 of the main ORF.

In one embodiment, the nucleotide sequence of the 5'-untranslated region is depicted in SEQ ID NO: 32, which sequence shows 5'-UTR of isoforms 3 to 6 of MDM2 (mouse double minute 2 homolog) mRNA. In one embodiment, the 5'-UTR as a variant of the sequence depicted in SEQ ID NO: 32.

In one embodiment of the polynucleotide, the nucleotide sequence of the targeting region is complementary to nucleotides 22 to 40 of the sequence depicted in SEQ ID NO: 32 or to a continuous or discontinuous portion of said sequence.

In one embodiment of the polynucleotide, the nucleotide sequence of the targeting region complementary to at least nucleotides 22 to 40 of the sequence depicted in SEQ ID NO: 32 is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.

In one embodiment of the polynucleotide, the nucleotide sequence of the targeting region complementary to at least nucleotides 22 to 40 of the sequence depicted in SEQ ID NO: 32 is flanked 3' by one or more additional nucleotides for targeting the nucleotide at position 21 up to at most to the nucleotide at position 1 of the sequence depicted in SEQ ID NO: 32. In one embodiment of the polynucleotide, the nucleotide sequence of the targeting region complementary to at least nucleotides 22 to 40 of the sequence depicted in SEQ ID NO: 32 is flanked 3' by one or more additional nucleotides for targeting the nucleotide at position 21 up to at most to the nucleotide at position 10 of the sequence depicted in SEQ ID NO: 32. Each nucleotide added 3' may be complementary or non-complementary to the corresponding nucleotide of the nucleotides flanking the portion of the 5'-UTR to which the nucleotide sequence of the targeting region is complementary.

In one embodiment, the nucleotide sequence of the targeting region complementary to at least nucleotides 22 to 40 of the sequence depicted in SEQ ID NO: 32 is flanked 5' by one or more additional nucleotides for targeting the nucleotide at position 41 up to at most to the nucleotide at position 61 of the sequence depicted in SEQ ID NO: 32. In one embodiment, the nucleotide sequence of the targeting region complementary to at least nucleotides 22 to 40 of the sequence depicted in SEQ ID NO: 32 is flanked 5' by one or more additional nucleotides for targeting the nucleotide at position 41 up to at most to the nucleotide at position 50 of the sequence depicted in SEQ ID NO: 32. Each nucleotide added 5' may be complementary or non-complementary to the corresponding nucleotide of the nucleotides flanking the portion of the 5'-UTR to which the nucleotide sequence of the targeting region is complementary.

In one embodiment of the polynucleotide, the nucleotide sequence of the targeting region is complementary to nucleotides 44 to 67 of the sequence depicted in SEQ ID NO: 32 or to a continuous or discontinuous portion of said sequence.

In one embodiment of the polynucleotide, the nucleotide sequence of the targeting region complementary to at least nucleotides 44 to 67 of the sequence depicted in SEQ ID NO: 32 is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.

In one embodiment of the polynucleotide, the nucleotide sequence of the targeting region complementary to at least nucleotides 44 to 67 of the sequence depicted in SEQ ID NO: 32 is flanked 3' by one or more additional nucleotides for targeting the nucleotide at position 43 up to at most to the nucleotide at position 32 of the sequence depicted in SEQ ID NO: 32. Each nucleotide added 3' may be complementary or non-complementary to the corresponding nucleotide of the nucleotides flanking the portion of the 5'-UTR to which the nucleotide sequence of the targeting region is complementary.

In one embodiment of the polynucleotide, the nucleotide sequence of the targeting region is complementary to at least one continuous portion of the 5'-UTR, the continuous portion being selected from the group consisting of:
(i) nucleotides 1 to 40 of the sequence depicted in SEQ ID NO: 32;
(ii) nucleotides 22 to 61 of the sequence depicted in SEQ ID NO: 32; and
(iii) nucleotides 44 to 67 of the sequence depicted in SEQ ID NO: 32.

In one embodiment, the continuous portion of the 5'-UTR is variant of any one of these portions under (i) to (iii).

In one embodiment of the polynucleotide, the nucleotide sequence of the targeting region is depicted in any one of SEQ ID NO: 33 to 39. In one embodiment, the nucleotide sequence of the targeting region of the polynucleotide is a variant of any of the sequences depicted in any one of SEQ ID NO: 33 to 39.

In one embodiment of the polynucleotide, the nucleotide sequence of the targeting region comprises the sequences as depicted in SEQ ID NO: 38, wherein the sequence is additionally flanked 5' in a contiguous manner by one or more nucleotides for targeting nucleotide +1 up to at most nucleotide +26, preferably up to at most nucleotide +16 of the ORF.

In one embodiment of the polynucleotide, the nucleotide sequence of the targeting region, preferably the targeting regions as specified above or below, is at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% complementary to the U1 snRNA or to a continuous or discontinuous portion thereof. In one embodiment, the nucleotide sequence of the targeting region is fully complementary, *i.e.,* 100% complementary, to the U1 snRNA or to a continuous or discontinuous portion thereof.

In one embodiment of the polynucleotide, the nucleotide sequence of the targeting region, preferably the targeting regions as specified above or below, is at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% complementary to the 5'-UTR or to a continuous or discontinuous portion thereof. In one embodiment, the nucleotide sequence of the targeting region is fully complementary, *i.e.,* 100% complementary, to the 5'-UTR or to a continuous or discontinuous portion thereof.

In one embodiment of the polynucleotide, the nucleotide sequence of the targeting region, preferably the targeting regions as specified above or below, is at least 15 nucleotides, at least 20 nucleotides, at least 25 nucleotides, at least 30 nucleotides, at least 35 nucleotides, or at least 40 nucleotides in length. In one embodiment, the nucleotide sequence of the targeting region is at most 300 nucleotides, at most 265 nucleotides, at most 250 nucleotides, at most 200 nucleotides, at most 150 nucleotides, at most 125 nucleotides, at most 100 nucleotides, at most 90 nucleotides, at most 80 nucleotides, at most 70 nucleotides, at most 60 nucleotides, or at most 50 nucleotides in length. For example, the nucleotide sequence of the targeting region may be 15 to 300 or may be 20 to 250 or may be 15 to 100 nucleotides in length. As will be easily understood by a person skilled in the art any of the lower limits disclosed can be combined with any of the upper limits mentioned.

In one embodiment of all aspects, the polynucleotide comprises at least one ribonucleotide. In one embodiment of all aspects, the polynucleotide consists of ribonucleotides. In one embodiment of all aspects, the polynucleotide comprises at least one deoxyribonucleotide. In one embodiment of all aspects, the polynucleotide comprises a mixture of ribonucleotides and deoxyribonucleotides. For example, the polynucleotide of the present invention may be a DNA polynucleotide, a cDNA polynucleotide, an RNA polynucleotide or a DNA/RNA hydrid polynucleotide. A DNA/RNA hydrid polynucleotide comprises at least one deoxyribonucleotide and at least one ribonucleotide, *e.g.*, such a hybrid may comprise one or more portions being DNA and one or more portions being RNA. In a further example or preferred embodiment, the polynucleotide of the invention may be a circular DNA polynucleotide, a circular cDNA polynucleotide, a circular RNA polynucleotide or a circular DNA/RNA hydrid polynucleotide.

In one embodiment of all aspects, the polynucleotide comprises at least one modified nucleotide. For example, a nucleotide may be modified with regard to the nucleobase and/or the sugar compound. Within the present invention such modifications may further comprise or be backbone modifications.

In one embodiment of all aspects, the polynucleotide comprises in addition to (i) the targeting region, preferably a targeting region as specified above or below, (ii) a region comprising or consisting of a nucleotide sequence capable of forming a stem-loop, which region is linked to the targeting region of (i), and (iii) two linker nucleotide sequences flanking the nucleotide sequence of the targeting region at both sides, *i.e.,* 5' and 3'. That is, when the polynucleotide is in a circularized form, the targeting region is on both sides separated from region capable of forming a stem-loop of (ii) by the one linker sequence. The linkers allow a more flexible presentation of the targeting region. In one embodiment, the nucleotide sequence capable of forming a stem-loop comprises at least 2, at least 3, at least 4, at least 5 or at least 6, and/or at most 15, at most 14, at most 13, at most 12, at most 11, at most 10, at most 9 or at most 8 perfect base-pairs with two overhanging ends. For example, the nucleotide sequence capable of forming a stem-loop may comprise 4 to 10 or 5 to 8 perfect base-pairs. As will be easily understood by a person skilled in the art any of the lower limits disclosed can be combined with any of the upper limits mentioned. The overhanging ends may be each at least 2, at least 3, at least 4, at least 5, at least 6, at least 7, at least 8, at least 9 or at least 10 nucleotides in length, and/or the overhanging ends may be each at most 40, at most 35, at most 30, at most 20 or at most 15 nucleotides in length. For example, the overhanging ends may be each 2 to 30 or 3 to 20 nucleotides in length. As will be easily understood by a person skilled in the art any of the lower limits disclosed can be combined with any of the upper limits mentioned. In one embodiment, each linker nucleotide sequence may be independently at least 1, at least 2 or at least 3 nucleotides in length, and/or may be independently at most 20, at most 15, at most 12, at most 10 or at most 8 nucleotides in length. For example, each linker may be 1 to 20 or 2 to 10 nucleotides in length. As will be easily understood by a person skilled in the art any of the lower limits disclosed can be combined with any of the upper limits mentioned. In one embodiment, the nucleotide sequence of the polynucleotide is depicted in SEQ ID NO: 48 to 53, 55 to 58, or 61 to 70, respectively. In one embodiment, the nucleotide sequence of the polynucleotide is a variant of any of the sequences depicted in SEQ ID NO: 48 to 53, 55 to 58 or 61 to 70, respectively.

In one embodiment of all aspects, the entire length of the polynucleotide is at least 30 nucleotides, at least 35 nucleotides, at least 40 nucleotides, at least 45 nucleotides, at least 50 nucleotides, at least 55 nucleotides, or at least 60 nucleotides in length. In one embodiment, the nucleotide sequence of the targeting region is at most 500 nucleotides, at most 400 nucleotides, at most 300 nucleotides, at most 200 nucleotides, at most 150 nucleotides, at most 125 nucleotides, at most 100 nucleotides, at most 90 nucleotides, or at most 80 nucleotides in length. For example, the nucleotide sequence of the targeting region may be 30 to 500 or may be 35 to 300 in length. As will be easily understood by a person skilled in the art any of the lower limits disclosed can be combined with any of the upper limits mentioned.

In a further aspect, the invention relates to a pharmaceutical composition comprising one or more of the polynucleotides of the invention, and at least one pharmaceutically acceptable carrier. The pharmaceutical composition may also comprise one or more excipients and/or one or more diluents.

In one embodiment, the composition is an aqueous composition. The aqueous composition may optionally comprise solutes, *e*.*g*., salts. In one embodiment, the composition is in the form of a freeze-dried composition. A freeze-dried composition is obtainable by freeze-drying a respective aqueous composition. In one embodiment, the composition is an injectable composition, optionally comprising sodium, potassium and calcium salts, such as Ringer's solution or Ringer's Lactate.

In one embodiment of the pharmaceutical composition, the polynucleotide, *e*.*g*., the circular RNA molecule, to be administered is naked, *i.e.,* not complexed with any kind of delivery material or proteins. In one embodiment of the pharmaceutical composition, the polynucleotide, *e*.*g*., the RNA molecule, to be administered is formulated in a delivery vehicle. In one embodiment, the delivery vehicle comprises particles. In one embodiment, the delivery vehicle comprises a lipid. In one embodiment, the lipid comprises a cationic lipid. In one embodiment, the lipid forms a complex with and/or encapsulates the polynucleotide, *e*.*g*., the RNA molecule. In one embodiment, the polynucleotide, *e*.*g*., the RNA molecule, is formulated in liposomes.

The pharmaceutical composition according to the present invention is generally to be applied in a "pharmaceutically effective amount" and in "a pharmaceutically acceptable preparation".

In one embodiment of the pharmaceutical composition, the one or more polynucleotides of the present invention is/are comprised in the pharmaceutical composition in an amount of at least 20 ng, of at least 50 ng, of at least 100 ng, of at least 200 ng, of at least 500 ng, of at least 1,000 ng or of at least 2,000 ng, and/or at most 20 mg, at most 15 mg, at most 10 mg, at most 9 mg, at most 8 mg, at most 7 mg, or at most 6 mg. For example, the one or more polynucleotides may be comprised in an amount of 20 ng to 20 mg or in an amount of 50 ng to 15 mg. As will be easily understood by a person skilled in the art any of the lower limits disclosed can be combined with any of the upper limits mentioned.

In one embodiment of the pharmaceutical composition, the one or more polynucleotides of the present invention is/are comprised in the pharmaceutical composition in a concentration of at least 1 nM, of at least 2 nM, of at least 3 nM, of at least 4 nM, of at least 5 nM, of at least 10 nM, of at least 15 nM, or of at least 20 nM, and/or at most 100 nM, at most 90 nM, at most 80 nM, at most 70 nM, at most 60 nM, at most 50 nM, at most 45 nM, or at most 40 nM. For example, the one or more polynucleotides may be comprised in a concentration of 1 nM to 100 nM or in a concentration of 5 nM to 50 nM. As will be easily understood by a person skilled in the art any of the lower limits disclosed can be combined with any of the upper limits mentioned.

In one embodiment, the pharmaceutical composition is formulated to be suitable for local or systemic administration. For example, the pharmaceutical composition can be formulated to be suitable for a parenteral administration.

The pharmaceutical composition of the invention can be used in the field of medicine. Thus, in a further aspect, the invention relates to a pharmaceutical composition of the invention for use as a medicament.

The polynucleotides or the pharmaceutical composition of the invention may be used in a method of treating or preventing a proliferative disorder. The proliferative disorder may be cancer.

In a further aspect, the invention relates to a polynucleotide of the invention or a pharmaceutical composition of the invention for use in a method of treating or preventing a proliferative disorder, said method comprising a step of administering to a patient in need thereof one or more of the polynucleotides of the invention or the pharmaceutical composition of the invention. In one embodiment, the proliferative disorder is cancer.

In another aspect, the invention relates to a method of treating or preventing a proliferative disease comprising administering to a patient in need thereof one or more of the polynucleotides of the invention or the pharmaceutical composition of the invention. In one embodiment, the proliferative disorder is cancer.

In one embodiment of these two aspects, (*i.e*., the medical use and the method of treatment), the one or more polynucleotides or the pharmaceutical composition is administered locally or systemically. In one embodiment, the one or more polynucleotides or the pharmaceutical composition is/are administered parenterally.

In one embodiment of these two aspects, the patient to be treated may be a human.

In one embodiment of these two aspects, the polynucleotide(s) of the invention or the pharmaceutical composition of the invention is/are applied in a pharmaceutically effective amount and/or in a pharmaceutically acceptable preparation. In one embodiment, the one or more polynucleotides of the present invention are administered per treatment in an amount of at least 20 ng, of at least 50 ng, of at least 100 ng, of at least 200 ng, of at least 500 ng, of at least 1,000 ng or of at least 2,000 ng, and/or at most 20 mg, at most 15 mg, at most 10 mg, at most 9 mg, at most 8 mg, at most 7 mg, or at most 6 mg. For example, the one or more polynucleotides may be administered in an amount of 20 ng to 20 mg per treatment or in an amount of 50 ng to 15 mg per treatment. As will be easily understood by a person skilled in the art any of the lower limits disclosed can be combined with any of the upper limits mentioned. In one embodiment, the one or more polynucleotides of the present invention are administered per treatment with a concentration of at least 1 nM, of at least 2 nM, of at least 3 nM, of at least 4 nM, of at least 5 nM, of at least 10 nM, of at least 15 nM, or of at least 20 nM, and/or at most 100 nM, at most 90 nM, at most 80 nM, at most 70 nM, at most 60 nM, at most 50 nM, at most 45 nM, or at most 40 nM. For example, the one or more polynucleotides may be administered in a concentration of 1 nM to 100 nM or in a concentration of 5 nM to 50 nM. As will be easily understood by a person skilled in the art any of the lower limits disclosed can be combined with any of the upper limits mentioned.

In a further aspect, the invention relates to a method for targeting a nucleic acid comprising contacting the nucleic acid to be targeted with a polynucleotide of the invention. In one embodiment, the nucleic acid to be targeted is RNA. The RNA to be targeted may be a portion of a mRNA, *e*.*g*., a 5'-UTR or a portion of the 5'-UTR, or may be a U1 snRNA, *e*.*g*., a human U1 snRNA, or a portion of said U1 snRNA. In one embodiment, the nucleic acid to be targeted is at least partially complementary to the nucleotide sequence of the targeting region of the polynucleotide. In one embodiment, the nucleic acid to be targeted comprises at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence complementarity to the nucleotide sequence of the targeting region of the polynucleotide.

In a further aspect, the invention relates to a method for forming a complex comprising a polynucleotide of the invention, and a single-stranded nucleic acid. The method comprises a step of contacting the polynucleotide with the single-stranded nucleic acid. In one embodiment, the single-stranded nucleic acid is a nucleic acid comprising or consisting of deoxyribonucleotides. For example, the nucleic acid may be a portion of a mRNA, *e*.*g*., a 5'-UTR or a portion of the 5'-UTR, or may be a U1 snRNA, *e*.*g*., a human U1 snRNA, or a portion of said U1 snRNA. In one embodiment, the single-stranded nucleic acid is at least partially complementary to the nucleotide sequence of the targeting region of the polynucleotide. In one embodiment, the single-stranded nucleic acid comprises at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence complementarity to the nucleotide sequence of the targeting region of the polynucleotide.

In a further aspect, the invention relates to a method for interfering with genome expression or for shaping the cellular proteome. The method comprises a step of contacting a polynucleotide of the invention with the nucleic acid of the cell or a patient. The patient may be a patient having a proliferative disease or being at the risk of developing a proliferative disease. In one embodiment, the patient may be a cancer patient.

In one embodiment of the methods of the invention, the respective method is an *in vitro* or an *in vivo* method.

Other features and advantages of the instant invention will be apparent from the following detailed description and claims.

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., (1995) Helvetica Chimica Acta, CH-4010 Basel, Switzerland.

The practice of the present invention will employ, unless otherwise indicated, conventional methods of biochemistry, cell biology, immunology, and recombinant DNA techniques which are explained in the literature in the field (*cf., e*.*g*., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, *i.e.,* the subject-matter consists in the inclusion of a stated member, integer or step or group of members, integers or steps. Thus, the term "comprise", and variations such as "comprises" and "comprising", includes "to comprise at least one", so may be in some embodiments "consisting of". The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by context. The use of any and all examples, or exemplary language (*e.g*., "such as"), provided herein is intended merely to better illustrate the invention and does not pose a limitation on the scope of the invention otherwise claimed. No language in the specification should be construed as indicating any non-claimed element essential to the practice of the invention.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

Terms such as "reducing" or "inhibiting" relate to the ability to cause an overall decrease, preferably of 5% or greater, 10% or greater, 20% or greater, more preferably of 50% or greater, and most preferably of 75% or greater, in the level. The term "inhibit" or similar phrases can include a complete or essentially complete inhibition, *i.e.,* a reduction to zero or essentially to zero.

Terms such as "increasing", "enhancing", "promoting" or "prolonging" preferably relate to an increase, enhancement, promotion or prolongation by about at least 10%, preferably at least 20%, preferably at least 30%, preferably at least 40%, preferably at least 50%, preferably at least 80%, preferably at least 100%, preferably at least 200% and in particular at least 300%. These terms may also relate to an increase, enhancement, promotion or prolongation from zero or a non-measurable or non-detectable level to a level of more than zero or a level which is measurable or detectable.

The terms "polynucleotide" and "nucleic acid", used interchangeably herein, refer to a polymeric form of nucleotides of any length, either ribonucleotides or/and deoxyribonucleotides. Thus, this term includes, but is not limited to, single-, or double-stranded DNA or RNA, genomic DNA, cDNA, DNA-RNA hybrids, or a polymer comprising purine and pyrimidine bases or other natural, chemically or biochemically modified, non-natural, or derivatized nucleotides. In a polynucleotide preferably the 5' end of one nucleotide and the 3' end of another nucleotide being linked to another. A polynucleotide typically varies in length and the term as used herein is, unless specified otherwise, not intended to be limited to a certain length. Polynucleotides can typically vary in length from 15 to several hundred nucleotides, more typically about 20-300 nucleotides in length or shorter, even more typically about 30-200 nucleotides in length.

As disclosed herein, the terms "nucleotide sequence" and "nucleic acid sequence", used interchangeably herein, refer to the sequence of nucleotides in a polynucleotide, *e*.*g*., a ribonucleic acid (RNA) or a deoxyribonucleic acid (DNA). The term may refer to an entire nucleic acid molecule (such as to the single strand of an entire nucleic acid molecule) or to a portion (*e.g*., a fragment) thereof.

By convention, sequences disclosed herein are set forth in the direction 5' to 3' unless other specified. Moreover, a strand containing the sequence of a SEQ ID NO has that sequence from 5' to 3' unless otherwise specified.

The term "3' ", when used directionally, generally refers to a region or position in a polynucleotide or oligonucleotide 3' (toward the 3' end of the nucleotide) from another region or position in the same polynucleotide or oligonucleotide. The term "3' end" generally refers to the 3' terminal nucleotide of the component oligonucleotides.

The term "5' ", when used directionally, generally refers to a region or position in a polynucleotide or oligonucleotide 5' (toward the 5' end of the nucleotide) from another region or position in the same polynucleotide or oligonucleotide. As used herein, the term "5' end" generally refers to the 5' terminal nucleotide of the component polynucleotide.

All sequences disclosed herein are shown as DNA sequences, unless indicated otherwise. A person skilled in the art is well aware of the fact that in respective RNA sequences T/t residues of the DNA sequence are to be replaced with U/u residues. For example, even though the targeting regions and polynucleotides of SEQ ID NOs: 2 to 9, 11 to 31, 33 to 39 and 41 to 72 are depicted as DNA sequences in the below Table of Sequences, the present application also encompasses the respective RNA sequences, and variants of these RNA sequences.

A polynucleotide of the invention comprises a nucleotide sequence, which sequence comprises a targeting region comprising or consisting of a nucleotide sequence complementary to (i) a U1 snRNA or to a continuous or discontinuous portion of said U1 snRNA or (ii) a 5' untranslated region (5'-UTR) of a mRNA or to a continuous or discontinuous portion of said 5' untranslated region.

The targeting region has a nucleotide sequence that is capable of hybridizing or hybridizes to a corresponding portion of a target nucleic acid, *e*.*g*., the U1 snRNA or the 5'-UTR. The targeting region is preferably a single-stranded nucleic acid.

A nucleic acid is "capable of hybridizing" or "hybridizes" to another nucleic acid if the two sequences are complementary with one another. A nucleic acid is "complementary" to another nucleic acid if the two sequences are capable of forming a stable duplex with one another under the appropriate *in vitro* and/or *in vivo* conditions of temperature and solution ionic strength. As is known in the art, standard Watson-Crick base-pairing includes: adenine (A) pairing with thymine (T), adenine (A) pairing with uracil (U), and guanine (G) pairing with cytosine (C).

Hybridization and washing conditions are also well known. The conditions of temperature and ionic strength determine the "stringency" of the hybridization. Hybridization requires that the two nucleic acids contain complementary sequences, although mismatches between bases are possible. According to the invention, hybridization is preferably carried out under conditions which allow specific hybridization between polynucleotides (stringent conditions). Stringent conditions are described, for example, in Molecular Cloning: A Laboratory Manual, J. Sambrook et al., Editors, 2nd Edition, Cold Spring Harbor Laboratory press, Cold Spring Harbor, New York, 1989 or Current Protocols in Molecular Biology, F.M. Ausubel et al., Editors, John Wiley & Sons, Inc., New York.

A person skilled in the art is well aware of the fact that nucleotide sequences need not be 100% complementary to that of its target nucleic acid to be specifically hybridizable or hybridizable. Moreover, a polynucleotide may hybridize over one or more segments such that intervening or adjacent segments are not involved in the hybridization event (*e.g*., a loop structure or hairpin structure). A nucleotide sequence of a targeting region can comprise at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence complementarity to the nucleotide sequence of a target region, *e*.*g*., the U1 snRNA or the 5'-UTR or a respective portion thereof. For example, 5, 6, 7, 8, 9, 10 nucleotides out of 10 nucleotides being 50%, 60%, 70%, 80%, 90%, and 100% complementary. If the complementary is not 100%, the remaining non-complementary nucleotides may be clustered or interspersed with complementary nucleotides and need not be contiguous to each other or to complementary nucleotides. "Fully complementary" or "100% complementary" means that all the contiguous residues of a nucleotide sequence will hydrogen bond with the same number of contiguous residues in a second nucleotide acid sequence. Preferably, the degree of complementarity according to the invention is at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%. Most preferably, the degree of complementarity according to the invention is 100%.

Methods of alignment of sequences for comparison are well-known in the art. Thus, the determination of percent identity between any two sequences can be accomplished using a mathematical algorithm. Sequence comparisons between two nucleotide sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Needleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

"Targeting" or "targeted" means the process of design and selection of a nucleotide sequence that will hybridize, preferably specifically hybridize, to a target sequence, for example a target RNA, such as a U1 snRNA portion or a 5'-UTR portion. A "target sequence", "target nucleic acid" or "target RNA" or "target DNA" all refer to a nucleotide sequence to which a targeting region is targeted.

The polynucleotides of the invention are for targeting either (i) a U1 snRNA or a continuous or discontinuous portion of said U1 snRNA or (ii) a 5'-UTR or a continuous or discontinuous portion of said 5'-UTR. Thus, the U1 snRNA or the 5'-UTR is, *e*.*g*., a target sequence.

The term "portion", which is used herein interchangeably with the terms "part" and "fragment", refers to a continuous or discontinuous fraction of a structure. With respect to a particular structure such as an amino acid sequence or protein or a nucleotide sequence the terms "part", "fragment" and "portion" thereof may designate a continuous or a discontinuous fraction of said structure. Preferably, a "part", "fragment" and "portion" of a structure such as an amino acid sequence or a nucleotide sequence preferably comprises, preferably consists of at least 2%, at least 4%, at least 10%, at least 20%, at least 30%, at least 40%, at least 50%, at least 60%, at least 70%, at least 80%, at least 85%, at least 90%, at least 92%, at least 94%, at least 96%, at least 98%, at least 99% of the entire structure or amino acid sequence or nucleotide sequence. A portion, a part or a fragment of a structure preferably comprises one or more functional or structural properties of said structure. For example, a portion, a part or a fragment may be capable of forming a stem-loop in secondary structure. If the portion, part or fragment is a discontinuous fraction said discontinuous fraction is preferably composed of 2, 3, 4, 5, 6, 7, 8, or more parts or segments of a structure, each of these parts or segments being a continuous element of the structure. For example, a discontinuous fraction of a nucleotide sequence may be composed of 2, 3, 4, 5, 6, 7, 8, or more, preferably not more than 4 parts of said nucleotide sequence, wherein each part or segment preferably comprises at least 5 continuous nucleotides, preferably at least 10 continuous nucleotides, more preferably at least 12 continuous nucleotides, more preferably at least 15 continuous nucleotides of the nucleotide sequence.

The terms "continuous" and "contiguous" may be used interchangeably herein and refer preferably to an uninterrupted extension on the nucleotide sequence, *e*.*g*., of the U1 snRNA or the 5'-UTR. Two segments are interrupted, if one or more nucleotides are inserted between two segments, *e*.*g*., a first segment and a second segment, thereby connecting these segments, which inserted nucleotides may be any nucleotides and are preferably nucleotides different in number and/or in chemical structure from the respective nucleotides present in the uninterrupted starting sequence. Furthermore, the terms first, second, third and the like in the description and in the claims, are used for distinguishing between similar elements and not necessarily for describing a sequential or chronological order, unless the context indicates otherwise.

A portion of a U1 snRNA or a 5'-UTR target sequence may, for example, be a nucleotide sequence of a stem-loop or a subregion of such a stem-loop. A portion of a target sequence may also be sequence which is located 5' or 3' to such a stem-loop or a subregion of such a stem-loop.

A "stem-loop " as used herein, preferably refers to a nucleotide sequence capable of forming a secondary structure that includes a region of nucleotides which are known or predicted to form a double strand (stem portion) that is linked on one side by a region of predominantly single-stranded nucleotides (loop portion). Such structures are well known in the art and these terms are used consistently with their known meanings in the art. As is known in the art, a stem-loop structure does not require exact base-pairing. Thus, the stem may include one or more base mismatches. Alternatively, the base- pairing may be exact, *i.e.,* not include any mismatches.

As used herein, any reference to a particular nucleotide position in a target sequence should not be construed to that extent that in a corresponding target sequence of another species or variant the corresponding nucleotide must be present at the same nucleotide position, as insertions or deletions of nucleotides may occur. That is, the corresponding nucleotide positions may vary and can, for example, be determined after performing a sequence alignment of the sequences in questions. Thus, when reference is made herein to a "nucleotide position", *e*.*g*., "a nucleotide position of the sequence depicted in SEQ ID NO: X", or to "a portion comprising nucleotides x to y", *e*.*g*., "a portion comprises nucleotides x to y of the sequence depicted in SEQ ID NO: X", these phrases are intended to comprise also the corresponding nucleotide position or the corresponding portion in another sequence, *e*.*g*., in another target sequence.

In one embodiment, the target sequence is a U1 snRNA or a continuous or discontinuous portion of said U1 snRNA. The U1 snRNA can be a human U1 snRNA. The U1 snRNA is a general component of the spliceosome that governs 5' splice site recognition and selection. Therefore, by targeting the U1 snRNA with antisense RNA molecules pre-mRNA splicing can be inhibited and/or alternative splicing patterns can be modulated.

The target sequence can be the complete U1 snRNA, for example a U1 snRNA having or comprising a sequence depicted in SEQ ID NO: 1 or can be a variant of this depicted nucleotide sequence. Alternatively, the U1 snRNA can be divided into portions and the target sequence comprises one or more of these portions or one or more segments of these portions.

According to the invention, the U1 snRNA target sequence can be selected from the following portions:
- a portion comprising nucleotides 1 to 100 of the sequence depicted in SEQ ID NO: 1 or a variant thereof;
- a portion comprising nucleotides 1 to 70 of the sequence depicted in SEQ ID NO: 1 or a variant thereof;
- a portion comprising nucleotides 1 to 50 of the sequence depicted in SEQ ID NO: 1 or a variant thereof;
- a portion comprising nucleotides 1 to 42 of the sequence depicted in SEQ ID NO: 1 or a variant thereof;
- a portion comprising nucleotides 1 to 40 of the sequence depicted in SEQ ID NO: 1 or a variant thereof;
- a portion comprising nucleotides 1 to 30 of the sequence depicted in SEQ ID NO: 1 or a variant thereof;
- a portion comprising nucleotides 1 to 26 of the sequence depicted in SEQ ID NO: 1 or a variant thereof;
- a portion comprising nucleotides 1 to 25 of the sequence depicted in SEQ ID NO: 1 or a variant thereof;
- a portion comprising nucleotides 1 to 22 of the sequence depicted in SEQ ID NO: 1 or a variant thereof;
- a portion comprising nucleotides 1 to 20 of the sequence depicted in SEQ ID NO: 1 or a variant thereof;
- a portion comprising nucleotides 5 to 100 of the sequence depicted in SEQ ID NO: 1 or a variant thereof;
- a portion comprising nucleotides 5 to 70 of the sequence depicted in SEQ ID NO: 1 or a variant thereof;
- a portion comprising nucleotides 5 to 50 of the sequence depicted in SEQ ID NO: 1 or a variant thereof;
- a portion comprising nucleotides 5 to 42 of the sequence depicted in SEQ ID NO: 1 or a variant thereof;
- a portion comprising nucleotides 5 to 40 of the sequence depicted in SEQ ID NO: 1 or a variant thereof;
- a portion comprising nucleotides 5 to 30 of the sequence depicted in SEQ ID NO: 1 or a variant thereof;
- a portion comprising nucleotides 5 to 26 of the sequence depicted in SEQ ID NO: 1 or a variant thereof;
- a portion comprising nucleotides 5 to 25 of the sequence depicted in SEQ ID NO: 1 or a variant thereof;
- a portion comprising nucleotides 5 to 22 of the sequence depicted in SEQ ID NO: 1 or a variant thereof;
- a portion comprising nucleotides 5 to 20 of the sequence depicted in SEQ ID NO: 1 or a variant thereof;
- a portion comprising nucleotides 10 to 100 of the sequence depicted in SEQ ID NO: 1 or a variant thereof;
- a portion comprising nucleotides 10 to 70 of the sequence depicted in SEQ ID NO: 1 or a variant thereof;
- a portion comprising nucleotides 10 to 50 of the sequence depicted in SEQ ID NO: 1 or a variant thereof;
- a portion comprising nucleotides 10 to 42 of the sequence depicted in SEQ ID NO: 1 or a variant thereof;
- a portion comprising nucleotides 10 to 40 of the sequence depicted in SEQ ID NO: 1 or a variant thereof;
- a portion comprising nucleotides 10 to 30 of the sequence depicted in SEQ ID NO: 1 or a variant thereof;
- a portion comprising nucleotides 10 to 26 of the sequence depicted in SEQ ID NO: 1 or a variant thereof;
- a portion comprising nucleotides 10 to 25 of the sequence depicted in SEQ ID NO: 1 or a variant thereof;
- a portion comprising nucleotides 10 to 22 of the sequence depicted in SEQ ID NO: 1 or a variant thereof;
- a portion comprising nucleotides 10 to 20 of the sequence depicted in SEQ ID NO: 1 or a variant thereof;
- a portion comprising nucleotides 12 to 27 of the sequence depicted in SEQ ID NO: 1 or a variant thereof;
- a portion comprising nucleotides 28 to 37 of the sequence depicted in SEQ ID NO: 1 or a variant thereof.

Preferred targeting regions comprise or consist of a nucleotide sequence complementary to at least one continuous portion selected form the group consisting of nucleotides 1 to 42, nucleotides 1 to 30, nucleotides 1 to 26, nucleotides 1 to 25, nucleotides 1 to 22, nucleotides 12 to 27, nucleotides 28 to 37, each of the sequence depicted in SEQ ID NO: 1, and any variants of these portions.

According to one embodiment of the invention, the nucleotide sequence of the targeting region may comprise at least two segments, each segment complementary to a nucleotide sequence of the U1 snRNA, wherein the complementary nucleotide sequences of the U1 snRNA are not continuous. Preferred combinations of such segments are disclosed in the summary of the invention and the attached claims. Within the nucleotide sequence of these targeting regions complementary to a discontinuous portion of the U1 snRNA one or more nucleotides, between two consecutive segments can be comprised.

A polynucleotide of the invention can comprise a targeting region comprising or consisting of a nucleotide sequence depicted in SEQ ID NO: 3, which sequence is complementary to nucleotides 1 to 20 of the U1 snRNA sequence as depicted in SEQ ID NO: 1. Alternatively, a polynucleotide of the invention can comprise a targeting region comprising or consisting of a nucleotide sequence having at least 70%, at least 75%, at least 80%, at least 85%, at least 90%, at least 95%, at least 97%, at least 99%, or 100% identity to the nucleotide sequence depicted in SEQ ID NO: 3.

**Table 1: Nucleotide sequence for targeting nucleotides 1 to 20 of a human U1 snRNA:**

| **SEQ ID NO:** | **5'elongation** | **AS-U1-20 (SEQ ID NO: 3)** | **3'elongation** |
|---|---|---|---|
| 3 | | CTCCCCTGCCAGGTAAGTAT | |

For increasing specificity and/or efficiency the nucleotide sequence of the targeting region of said polynucleotide can be further elongated by one or more additional nucleotides. The nucleotide sequence depicted in SEQ ID NO: 3 can be elongated in 5' direction, *i.e.,* 5' relative to the said sequence.

Thus, the nucleotide sequence as set forth in SEQ ID NO: 3 can be additionally flanked 5' in a contiguous manner by one or more nucleotides for targeting nucleotide 21 up to at most nucleotide 100, preferably up to at most nucleotide 50, 42 or 30 of the sequence depicted in SEQ ID NO: 1, respectively, or the respective nucleotides in variant sequences. The first nucleotide added 5' to the sequence depicted in SEQ ID NO: 3 is for targeting the nucleotide at position 21 of the sequence depicted in SEQ ID NO: 1. The second nucleotide added to the elongated sequence (*i.e.,* SEQ ID NO: 3 plus 1 nucleotide) is for targeting the nucleotide at position 22 of the sequence depicted in SEQ ID NO: 1, the third nucleotide added to the elongated sequence (*i.e.,* SEQ ID NO: 3 plus 2 nucleotides) is for targeting the nucleotide at position 23 of the sequence depicted in SEQ ID NO: 1, the forth nucleotide added to the elongated sequence (*i.e.,* SEQ ID NO: 3 plus 3 nucleotide) is for targeting the nucleotide at position 24 of the sequence depicted in SEQ ID NO: 1, and so forth. Thus, the nucleotide sequence as set forth in SEQ ID NO: 3 can be 5' elongated in a contiguous manner by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 nucleotides. Each nucleotide added 5' can be complementary or not to its respective nucleotide in the sequence depicted in SEQ ID NO: 1. For example, the first nucleotide added at the 5' end of the sequence (5'-CTCCCCTGCCAGGTAAGTAT-3') depicted in SEQ ID NO: 3 is a nucleotide having the nucleobase thymine/uracil for targeting the nucleotide having the nucleobase adenine at position 21 of the target strand (shown from 5' to 3' in the sequence below), the second nucleotide added at the 5' end of the elongated sequence is a nucleotide having the nucleobase adenine for targeting the nucleotide having the nucleobase thymine/uracil at position 22 of the target strand, the third nucleotide added at the 5' end of the elongated sequence is a nucleotide having the nucleobase thymine/uracil for targeting the nucleotide having the nucleobase adenine at position 23 of the target strand, the fourth nucleotide added at the 5' end of the elongated sequence is a nucleotide having the nucleobase guanine for targeting the nucleotide having the nucleobase cytosine at position 24 of the target strand, and so forth.

3'-TATGAATGGACCGTCCCCTC-5'      SEQ ID NO: 3)
   ||||||||||||||||||||
5'-ATACTTACCTGGCAGGGGAGATACCATGATCACGAAGGTGGTTTTCCCAGGGCGAGGCTTATCCATT
GCACTCCGGATGTGCTGACCCCTGCGATTTCCCCAAATGTGGGAAACTCGACTGCATAATTTGTGGTAGT
GGGGGACTGCGTTCGCGCTTTCCCCTG-3'
(SEQ ID NO: 1)

Preferred nucleotide sequences of targeting regions which are elongated in 5' direction comprise any of the sequences as shown in Table 2, or any variants thereof:

**Table 2:**

| **SEQ ID NO:** | **5'elongation** | **AS-U1-20 (SEQ ID NO: 3)** | **3'elongation** |
|---|---|---|---|
| 4 | AT | CTCCCCTGCCAGGTAAGTAT | |
| 5 | ATCATGGTAT | CTCCCCTGCCAGGTAAGTAT | |
| 6 | ACCACCTTCGTGATCATGGTAT | CTCCCCTGCCAGGTAAGTAT | |

These preferred 5' elongations are for targeting the portion comprising nucleotides 1 to 22 (SEQ ID NO: 4) or nucleotides 1 to 30 (SEQ ID NO: 5) or nucleotides 1 to 42 (SEQ ID NO: 6) of the human U1 snRNA. Also encompassed by the present invention are variants of the sequences depicted in SEQ ID NO: 4 to SEQ ID NO: 6.

The polynucleotides of the present invention can also comprise a targeting region comprising or consisting of a nucleotide sequence which is the result of a 5' elongation of the sequence depicted in SEQ ID NO: 3 for targeting a portion selected from the group consisting of nucleotides 1 to 25, nucleotides 1 to 26, nucleotides 1 to 27, nucleotides 1 to 28, nucleotides 1 to 29, nucleotides 1 to 31, nucleotides 1 to 32, nucleotides 1 to 33, nucleotides 1 to 34, nucleotides 1 to 35, nucleotides 1 to 36, nucleotides 1 to 37, nucleotides 1 to 38, nucleotides 1 to 39, nucleotides 1 to 40, nucleotides 1 to 41, nucleotides 1 to 43, nucleotides 1 to 44, nucleotides 1 to 45, nucleotides 1 to 46, nucleotides 1 to 47, nucleotides 1 to 48, nucleotides 1 to 49, nucleotides 1 to 50, nucleotides 1 to 60, nucleotides 1 to 70, each of the sequence depicted in SEQ ID NO: 1, and any variants of these portions.

The polynucleotides of the present invention can also comprise a targeting region comprising or consisting of a nucleotide sequence for targeting a portion selected from the group consisting of nucleotides 2 to 20, nucleotides 2 to 21, nucleotides 2 to 22, nucleotides 2 to 23, nucleotides 2 to 24, nucleotides 2 to 25, nucleotides 2 to 26, nucleotides 2 to 27, nucleotides 2 to 28, nucleotides 2 to 29, nucleotides 2 to 30, nucleotides 2 to 31, nucleotides 2 to 32, nucleotides 2 to 33, nucleotides 2 to 34, nucleotides 2 to 35, nucleotides 2 to 36, nucleotides 2 to 37, nucleotides 2 to 38, nucleotides 2 to 39, nucleotides 2 to 40, nucleotides 2 to 41, nucleotides 2 to 42, nucleotides 2 to 43, nucleotides 2 to 44, nucleotides 2 to 45, nucleotides 2 to 46, nucleotides 2 to 47, nucleotides 2 to 48, nucleotides 2 to 49, nucleotides 2 to 50, nucleotides 2 to 60, nucleotides 2 to 70, each of the sequence depicted in SEQ ID NO: 1, and any variants of these portions.

The polynucleotides of the present invention can also comprise a targeting region comprising or consisting of a nucleotide sequence for targeting a portion selected from the group consisting of nucleotides 3 to 20, nucleotides 3 to 21, nucleotides 3 to 22, nucleotides 3 to 23, nucleotides 3 to 24, nucleotides 3 to 25, nucleotides 3 to 26, nucleotides 3 to 27, nucleotides 3 to 28, nucleotides 3 to 29, nucleotides 3 to 30, nucleotides 3 to 31, nucleotides 3 to 32, nucleotides 3 to 33, nucleotides 3 to 34, nucleotides 3 to 35, nucleotides 3 to 36, nucleotides 3 to 37, nucleotides 3 to 38, nucleotides 3 to 39, nucleotides 3 to 40, nucleotides 3 to 41, nucleotides 3 to 42, nucleotides 3 to 43, nucleotides 3 to 44, nucleotides 3 to 45, nucleotides 3 to 46, nucleotides 3 to 47, nucleotides 3 to 48, nucleotides 3 to 49, nucleotides 3 to 50, nucleotides 3 to 60, nucleotides 3 to 70, each of the sequence depicted in SEQ ID NO: 1, and any variants of these portions.

The polynucleotides of the present invention can also comprise a targeting region comprising or consisting of a nucleotide sequence for targeting a portion selected from the group consisting of nucleotides 4 to 20, nucleotides 4 to 21, nucleotides 4 to 22, nucleotides 4 to 23, nucleotides 4 to 24, nucleotides 4 to 25, nucleotides 4 to 26, nucleotides 4 to 27, nucleotides 4 to 28, nucleotides 4 to 29, nucleotides 4 to 30, nucleotides 4 to 31, nucleotides 4 to 32, nucleotides 4 to 33, nucleotides 4 to 34, nucleotides 4 to 35, nucleotides 4 to 36, nucleotides 4 to 37, nucleotides 4 to 38, nucleotides 4 to 39, nucleotides 4 to 40, nucleotides 4 to 41, nucleotides 4 to 42, nucleotides 4 to 43, nucleotides 4 to 44, nucleotides 4 to 45, nucleotides 4 to 46, nucleotides 4 to 47, nucleotides 4 to 48, nucleotides 4 to 49, nucleotides 4 to 50, nucleotides 4 to 60, nucleotides 4 to 70, each of the sequence depicted in SEQ ID NO: 1, and any variants of these portions.

The polynucleotides of the present invention can also comprise a targeting region comprising or consisting of a nucleotide sequence for targeting a portion selected from the group consisting of nucleotides 5 to 20, nucleotides 5 to 21, nucleotides 5 to 22, nucleotides 5 to 23, nucleotides 5 to 24, nucleotides 5 to 25, nucleotides 5 to 26, nucleotides 5 to 27, nucleotides 5 to 28, nucleotides 5 to 29, nucleotides 5 to 30, nucleotides 5 to 31, nucleotides 5 to 32, nucleotides 5 to 33, nucleotides 5 to 34, nucleotides 5 to 35, nucleotides 5 to 36, nucleotides 5 to 37, nucleotides 5 to 38, nucleotides 5 to 39, nucleotides 5 to 40, nucleotides 5 to 41, nucleotides 5 to 42, nucleotides 5 to 43, nucleotides 5 to 44, nucleotides 5 to 45, nucleotides 5 to 46, nucleotides 5 to 47, nucleotides 5 to 48, nucleotides 5 to 49, nucleotides 5 to 50, nucleotides 5 to 60, nucleotides 5 to 70, each of the sequence depicted in SEQ ID NO: 1, and any variants of these portions.

The polynucleotides of the present invention can also comprise a targeting region comprising or consisting of a nucleotide sequence for targeting a portion selected from the group consisting of nucleotides 10 to 20, nucleotides 10 to 21, nucleotides 10 to 22, nucleotides 10 to 23, nucleotides 10 to 24, nucleotides 10 to 25, nucleotides 10 to 26, nucleotides 10 to 27, nucleotides 10 to 28, nucleotides 10 to 29, nucleotides 10 to 30, nucleotides 10 to 31, nucleotides 10 to 32, nucleotides 10 to 33, nucleotides 10 to 34, nucleotides 10 to 35, nucleotides 10 to 36, nucleotides 10 to 37, nucleotides 10 to 38, nucleotides 10 to 39, nucleotides 10 to 40, nucleotides 10 to 41, nucleotides 10 to 42, nucleotides 10 to 43, nucleotides 10 to 44, nucleotides 10 to 45, nucleotides 10 to 46, nucleotides 10 to 47, nucleotides 10 to 48, nucleotides 10 to 49, nucleotides 10 to 50, nucleotides 10 to 60, nucleotides 10 to 70, each of the sequence depicted in SEQ ID NO: 1, and any variants of these portions.

The polynucleotides of the present invention can also comprise a targeting region comprising or consisting of a nucleotide sequence for targeting a contiguous portion of a U1 snRNA, wherein the targeting nucleotide sequence is 20 to 50 nucleotides in length and is complementary to a contiguous portion of the U1 snRNA. In one embodiment, the U1 snRNA comprises a nucleotide sequence as depicted in SEQ ID NO: 1 or is a variant thereof. In one embodiment, the target region is located within nucleotides 1 to 100, 1 to 90, 1 to 80, 1 to 70, 1 to 60, 1 to 50, each of the sequence depicted in SEQ ID NO: 1, and any variants of these portions.

In one embodiment, the targeting region of the polynucleotide comprises two or more copies of the nucleotide sequence complementary to a U1 snRNA or to a continuous or discontinuous portion of said U1 snRNA, *e*.*g*., the aforementioned portions of the U1 snRNA or the aforementioned embodiments of the U1 snRNA targeting sequences. In one embodiment of the polynucleotide, the targeting region of the polynucleotide comprises two or three copies of the nucleotide sequence complementary to a U1 snRNA or to a continuous or discontinuous portion of said U1 snRNA.

The two or more copies of the nucleotide sequence complementary to a U1 snRNA or to a continuous or discontinuous portion of said U1 snRNA can be linked to each other by spacer sequence, each spacer sequence may comprise 1 to 10, preferably 2 to 8, 2 to 6, or 2 to 4 nucleotides. In one embodiment, the spacer sequence is not complementary to the U1 snRNA sequence. In one embodiment, the nucleotides of the spacer sequence are nucleotides having the nucleobase adenine and/or the nucleobase thymine/uracil.

Accordingly, other preferred targeting regions comprised in a polynucleotide of the invention may comprise or consist of a nucleotide sequence depicted in any of SEQ ID NO: 7 to 9, as shown in Table 3, or any variants thereof.

**Table 3:**

| **SEQ ID NO:** | **Name** | **Sequence** |
|---|---|---|
| 7 | AS-U1-3x25 | GGTATCTCCCCTGCCAGGTAAGTATATATGGTATCTCCCCTGCCAGG TAAGTATATATGGTATCTCCCCTGCCAGGTAAGTAT |
| 8 | AS-U1-2x30 | ATCATGGTATCTCCCCTGCCAGGTAAGTATAATTAAATCATGGTATC TCCCCTGCCAGGTAAGTAT |
| 9 | AS-U1-3x30 | ATCATGGTATCTCCCCTGCCAGGTAAGTATAATTAAATCATGGTATC TCCCCTGCCAGGTAAGTATAATTAAATCATGGTATCTCCCCTGCCAG GTAAGTAT |

The sequence depicted in SEQ ID NO: 7 is for targeting nucleotide positions 1 to 25 of the sequence depicted in SEQ ID NO: 1, wherein the sequence depicted in SEQ ID NO: 7 comprises three copies of the nucleotide sequence complementary to nucleotides 1 to 25 of the U1 snRNA. The sequence depicted in SEQ ID NO: 8 is for targeting nucleotide positions 1 to 30 of the sequence depicted in SEQ ID NO: 1, wherein the sequence depicted in SEQ ID NO: 8 comprises two copies of the nucleotide sequence complementary to nucleotides 1 to 30 of the U1 snRNA. The sequence depicted in SEQ ID NO: 9 is for targeting nucleotide positions 1 to 30 of the sequence depicted in SEQ ID NO: 1, wherein the sequence depicted in SEQ ID NO: 9 comprises three copies of the nucleotide sequence complementary to nucleotides 1 to 30 of the U1 snRNA. In each of these sequences as depicted in SEQ ID NOs: 7 to 9 the spacer sequences are shown in bold and underlined in Table 3. Also encompassed by the present invention are variants of the sequences depicted in SEQ ID NO: 7 to SEQ ID NO: 9.

In case the targeting region of the polynucleotide comprises two or more copies of the nucleotide sequence complementary to a U1 snRNA or to a continuous or discontinuous portion of said U1 snRNA, the entire length of the nucleotide sequence of the targeting region is preferably at least 50, at least 55, or at least 60 nucleotides in length. In one embodiment, the nucleotide sequence of the entire targeting region is at most 120 nucleotides, at most 110 nucleotides, or at most 100 nucleotides in length. For example, the nucleotide sequence of the entire targeting region may be 50 to 120 or may be 55 to 100 nucleotides in length. As will be easily understood by a person skilled in the art any of the lower limits disclosed can be combined with any of the upper limits mentioned.

In another aspect, the target structure is involved in mRNA translation and the polynucleotide is capable of inhibiting mRNA translation. Therefore, the target region can be a 5' untranslated region (5'-UTR) of a mRNA or a continuous or discontinuous portion of said 5' untranslated region. For example, the targeting sequence can be directed against a 5'-UTR of a human β-globin (HBB) mRNA or a 5'-UTR of a MDM2 (mouse double minute 2 homolog) gene, *e*.*g*., of a human MDM2 gene. By targeting a 5'-UTR translation of the gene of interest can be inhibited or reduced.

As established in the art, mRNA generally contains a 5' untranslated region (5'-UTR), a peptide coding region and a 3' untranslated region (3'-UTR). The term "untranslated region" or "UTR" relates to a region in a DNA molecule which is transcribed but is not translated into an amino acid sequence, or to the corresponding region in an RNA molecule, such as an mRNA molecule. An untranslated region (UTR) can be present 5' (upstream) of an open reading frame (5'-UTR) and/or 3' (downstream) of an open reading frame (3'-UTR). A 5'-UTR, if present, is located at the 5' end, upstream of the start codon of a protein-encoding region. A 5'-UTR is downstream of the 5'-cap (if present), *e*.*g*., directly adjacent to the 5'-cap.

The term "open reading frame" (ORF) refers to a nucleotide sequence beginning with a start codon (*e.g*., methionine (ATG or AUG, respectively)) and ending with a stop codon (*e.g*., TAA, TAG or TGA), and encoding a protein or peptide.

The target sequence can be the complete 5' untranslated region (5'-UTR). Alternatively, the 5'-UTR can be divided into portions and the target sequence comprises one or more of these portions or one or more segments of these portions.

In one embodiment, the 5'-UTR is a eukaryotic 5'-UTR, for example a 5'-UTR having or comprising a sequence depicted in SEQ ID NO: 10, SEQ ID NO: 32 or SEQ ID NO: 40, or can be a variant of these depicted nucleotide sequences.

In one embodiment, the targeting region is complementary to at least one region of the 5' UTR which located in the 5' terminal of the 5'-UTR. As used herein, a region of the 5' terminal of the 5'-UTR can comprise or consist of a nucleotide sequence comprised in the first 60% of the complete 5'-UTR nucleotide sequence, relative to the 5' end of the 5'-UTR sequence. For example, the 5' terminal of the 5'-UTR can comprise or consist of a nucleotide sequence comprised in the first 55%, 50%, 45% of 40% of the complete 5'-UTR nucleotide sequence.

In one embodiment, the targeting region is complementary to at least one region of the 5' UTR which is located in the AUG-proximal region of the 5'-UTR. In one embodiment, the AUG-proximal region of the 5'-UTR does not comprise the start codon AUG of the ORF or the main ORF. In one embodiment, the AUG-proximal region of the 5'-UTR comprises the start codon AUG of the ORF or the main ORF, wherein the A nucleotide of the AUG start codon is referred to as the nucleotide at position +1 herein.

The AUG-proximal region of the 5'-UTR comprises the start codon AUG comprises at least nucleotides -3 to -1 of the 5'-UTR nucleotide sequence, and at least nucleotides +4 to +9 of the ORF or the main ORF nucleotide sequence. In one embodiment, the AUG-proximal region of the 5'-UTR complementary to at least the nucleotides -3 to +9 is flanked on one or both ends by a flanking region, wherein each flanking region comprises one or more additional nucleotides, and each additional nucleotide is complementary or non-complementary to the corresponding nucleotide of the nucleotides flanking the portion of the AUG-proximal region of the 5'-UTR to which the nucleotide sequence of the targeting region is complementary. In one embodiment, the nucleotide sequence of the targeting region complementary to at least the nucleotides -3 to +9 is flanked 5' by one or more additional nucleotides for targeting the nucleotide at position -4 up to at most to the nucleotide at position -40 of the 5'-UTR sequence. In one embodiment, the nucleotide sequence of the targeting region complementary to at least the nucleotides -3 to +9 is flanked 5' by one or more additional nucleotides for targeting the nucleotide at position -4 up to at most to the nucleotide at position -30 of the 5'-UTR sequence. In one embodiment, the nucleotide sequence of the targeting region complementary to at least the nucleotides -3 to +9 is flanked 5' by one or more additional nucleotides for targeting the nucleotide at position -4 up to at most to the nucleotide at position -20 of the 5'-UTR sequence. In one embodiment, the nucleotide sequence of the targeting region complementary to at least the nucleotides -3 to +9 is flanked 5' by one or more additional nucleotides for targeting the nucleotide at position -4 up to at most to the nucleotide at position -10 of the 5'-UTR sequence. In one embodiment, the nucleotide sequence of the targeting region complementary to at least the nucleotides -3 to +9 is flanked 3' by one or more additional nucleotides for targeting the nucleotide at position +10 up to at most to the nucleotide at position +50 of the ORF or the main ORF nucleotide sequence. In one embodiment, the nucleotide sequence of the targeting region complementary to at least the nucleotides -3 to +9 is flanked 3' by one or more additional nucleotides for targeting the nucleotide at position +10 up to at most to the nucleotide at position +50 of the ORF or the main ORF nucleotide sequence. In one embodiment, the nucleotide sequence of the targeting region complementary to at least the nucleotides -3 to +9 is flanked 3' by one or more additional nucleotides for targeting the nucleotide at position +10 up to at most to the nucleotide at position +40 of the ORF or the main ORF nucleotide sequence. In one embodiment, the nucleotide sequence of the targeting region complementary to at least the nucleotides -3 to +9 is flanked 3' by one or more additional nucleotides for targeting the nucleotide at position +10 up to at most to the nucleotide at position +30 or +20 of the ORF or the main ORF nucleotide sequence.

The 5'-UTR can comprise at least one upstream open reading frame (uORF). In one embodiment, the nucleotide sequence of the targeting region does not comprise a nucleotide sequence complementary to nucleotide sequence comprising an AUG start codon of the at least one uORF.

According to the invention, the 5'-UTR target sequence can be selected from the following portions:
- a portion comprising nucleotides 1 to 60 of the 5'-UTR sequence;
- a portion comprising nucleotides 1 to 50 of the 5'-UTR sequence;
- a portion comprising nucleotides 1 to 40 of the 5'-UTR sequence;
- a portion comprising nucleotides 10 to 60 of the 5'-UTR sequence;
- a portion comprising nucleotides 10 to 50 of the 5'-UTR sequence;
- a portion comprising nucleotides 10 to 40 of the 5'-UTR sequence;
- a portion comprising nucleotides 22 to 60 of the 5'-UTR sequence;
- a portion comprising nucleotides 22 to 50 of the 5'-UTR sequence;
- a portion comprising nucleotides 22 to 40 of the 5'-UTR sequence.

In one embodiment, the targeting region is complementary to at least the nucleotides 22 to 40 of the 5'-UTR nucleotide sequence.

For increasing specificity and/or efficiency the nucleotide sequence of the targeting region can be further elongated by one or more additional nucleotides in one or both directions, *i.e.,* 3' and/or 5' relative to the said sequence. In one embodiment, the 5' terminal targeting region complementary to at least the nucleotides 22 to 40 of the 5'-UTR is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary. In one embodiment, the nucleotide sequence of the targeting region complementary to at least nucleotides 22 to 40 of the 5'-UTR is flanked 3' by one or more additional nucleotides for targeting the nucleotide at position 21 up to at most to the nucleotide at position 1 of the 5'-UTR sequence. In one embodiment, the nucleotide sequence of the targeting region complementary to at least nucleotides 22 to 40 of the 5'-UTR is flanked 3' by one or more additional nucleotides for targeting the nucleotide at position 21 up to at least to the nucleotide at position 10 of the 5'-UTR sequence. In one embodiment, the nucleotide sequence of the targeting region complementary to at least nucleotides 22 to 40 of the 5'-UTR is flanked 5' by one or more additional nucleotides for targeting the nucleotide at position 41 up to at most to the nucleotide at position 60 of the 5'-UTR sequence. In one embodiment, the nucleotide sequence of the targeting region complementary to at least nucleotides 22 to 40 of the 5'-UTR is flanked 5' by one or more additional nucleotides for targeting the nucleotide at position 41 up to at least to the nucleotide at position 50 of the 5'-UTR sequence. The polynucleotides of the present invention can also comprise a targeting region comprising or consisting of a nucleotide sequence which is the result of any combination between above mentioned 5' elongations and 3' elongations, including variants of these combined nucleotide sequences.

In one embodiment, the 5'-UTR is from a MDM2 (mouse double minute 2 homolog) gene, *e*.*g*., from a human MDM2 proto-oncogene. The MDM2 gene shows a complex expression and processing pattern, with a relatively long, complex 5'-UTR that is processed in several variants and contains two upstream open reading frames (uORFs). MDM2 encodes a cellular phosphoprotein that plays a key role in the regulation of the tumor suppressor protein p53, negatively modulating its activity and stability through ubiquitylation and proteasome-dependent degradation. Functionally, MDM2 and p53 act in a self-regulatory feedback loop. MDM2 can bind p53 with high affinity, inducing its nuclear export and inhibiting its ability to act as a transcription factor. In many cancers, the MDM2-p53 feedback loop is deregulated, which makes it an attractive target for therapy, in particular for the therapy of proliferative diseases, such as the treatment of cancer. Furthermore, overexpression of MDM2 can promote the formation, progression, and drug resistance of malignant tumors.

MDM2 comprises six main isoforms. Isoform 1 comprises the sequence set forth in NCBI Reference Sequence: NM_002392, isoform 2 comprises the sequence set forth in NCBI Reference Sequence: NM_001145339, isoform 3 comprises the sequence set forth in NCBI Reference Sequence: NM_001145337, isoform 4 comprises the sequence set forth in NCBI Reference Sequence: NM_001145340, isoform 5 comprises the sequence set forth in NCBI Reference Sequence: NM_001278462, isoform 6 comprises the sequence set forth in NCBI Reference Sequence: NM_001367990. Isoforms 1 and 2 contain a long 5'-UTR sequence (nucleotides 1 to 324 as depicted in SEQ ID NO: 10) with two additional uORFs. Isoforms 3 to 6 have a short 5'-UTR sequence (nucleotides 1 to 67 without uORFs as depicted in SEQ ID NO: 32).

In one embodiment, the target sequence can be the complete 5'-UTR having or comprising a sequence depicted in SEQ ID NO: 10 or can be a variant of this depicted nucleotide sequence. Alternatively, the 5'-UTR having or comprising a sequence depicted in SEQ ID NO: 10 can be divided into portions and the target sequence comprises one or more of these portions or one or more segments of these portions.

According to the invention, the 5'-UTR target sequence can be selected from the following portions:
- a portion comprising nucleotides 1 to 60 of the sequence depicted in SEQ ID NO: 10 or a variant thereof;
- a portion comprising nucleotides 1 to 50 of the sequence depicted in SEQ ID NO: 10 or a variant thereof;
- a portion comprising nucleotides 1 to 40 of the sequence depicted in SEQ ID NO: 10 or a variant thereof;
- a portion comprising nucleotides 10 to 60 of the sequence depicted in SEQ ID NO: 10 or a variant thereof;
- a portion comprising nucleotides 10 to 50 of the sequence depicted in SEQ ID NO: 10 or a variant thereof;
- a portion comprising nucleotides 10 to 40 of the sequence depicted in SEQ ID NO: 10 or a variant thereof;
- a portion comprising nucleotides 22 to 60 of the sequence depicted in SEQ ID NO: 10 or a variant thereof;
- a portion comprising nucleotides 22 to 50 of the sequence depicted in SEQ ID NO: 10 or a variant thereof;
- a portion comprising nucleotides 22 to 40 of the sequence depicted in SEQ ID NO: 10 or a variant thereof;
- a portion comprising nucleotides 47 to 106 of the sequence depicted in SEQ ID NO: 10 or a variant thereof;
- a portion comprising nucleotides 57 to 106 of the sequence depicted in SEQ ID NO: 10 or a variant thereof;
- a portion comprising nucleotides 67 to 106 of the sequence depicted in SEQ ID NO: 10 or a variant thereof;
- a portion comprising nucleotides 47 to 96 of the sequence depicted in SEQ ID NO: 10 or a variant thereof;
- a portion comprising nucleotides 57 to 96 of the sequence depicted in SEQ ID NO: 10 or a variant thereof;
- a portion comprising nucleotides 67 to 96 of the sequence depicted in SEQ ID NO: 10 or a variant thereof;
- a portion comprising nucleotides 47 to 86 of the sequence depicted in SEQ ID NO: 10 or a variant thereof;
- a portion comprising nucleotides 57 to 86 of the sequence depicted in SEQ ID NO: 10 or a variant thereof;
- a portion comprising nucleotides 67 to 86 of the sequence depicted in SEQ ID NO: 10 or a variant thereof;
- a portion comprising nucleotides 118 to 177 of the sequence depicted in SEQ ID NO: 10 or a variant thereof;
- a portion comprising nucleotides 128 to 177 of the sequence depicted in SEQ ID NO: 10 or a variant thereof;
- a portion comprising nucleotides 138 to 177 of the sequence depicted in SEQ ID NO: 10 or a variant thereof;
- a portion comprising nucleotides 118 to 167 of the sequence depicted in SEQ ID NO: 10 or a variant thereof;
- a portion comprising nucleotides 128 to 167 of the sequence depicted in SEQ ID NO: 10 or a variant thereof;
- a portion comprising nucleotides 138 to 167 of the sequence depicted in SEQ ID NO: 10 or a variant thereof;
- a portion comprising nucleotides 118 to 157 of the sequence depicted in SEQ ID NO: 10 or a variant thereof;
- a portion comprising nucleotides 128 to 157 of the sequence depicted in SEQ ID NO: 10 or a variant thereof;
- a portion comprising nucleotides 138 to 157 of the sequence depicted in SEQ ID NO: 10 or a variant thereof;
- a portion comprising nucleotides 259 to 324 of the sequence depicted in SEQ ID NO: 10 or a variant thereof;
- a portion comprising nucleotides 269 to 324 of the sequence depicted in SEQ ID NO: 10 or a variant thereof;
- a portion comprising nucleotides 279 to 324 of the sequence depicted in SEQ ID NO: 10 or a variant thereof;
- a portion comprising nucleotides 259 to 318 of the sequence depicted in SEQ ID NO: 10 or a variant thereof;
- a portion comprising nucleotides 269 to 318 of the sequence depicted in SEQ ID NO: 10 or a variant thereof;
- a portion comprising nucleotides 279 to 318 of the sequence depicted in SEQ ID NO: 10 or a variant thereof.

According to the invention the targeting region of the polynucleotide may comprise or consist of a nucleotide sequence complementary to at least one of these portions or to at least one segment within these portions.

Preferred targeting regions comprise or consist of a nucleotide sequence complementary to at least one continuous portion selected form the group consisting of nucleotides 1 to 40, nucleotides 22 to 40, nucleotides 57 to 96, nucleotides 67 to 86, nucleotides 128 to 167, nucleotides 138 to 157, nucleotides 279 to 318, nucleotides 279 to 324, nucleotides 301 to 318, nucleotides 301 to 324, each of the sequence depicted in SEQ ID NO: 10, and any variants of these portions.

According to one embodiment of the invention, the nucleotide sequence of the targeting region may comprise at least two segments, each segment complementary to a nucleotide sequence of the 5'-UTR, wherein the complementary nucleotide sequences of the 5'-UTR are not continuous. Within the nucleotide sequence of these targeting regions complementary to a discontinuous portion of the 5'-UTR one or more nucleotides, between two consecutive segments can be comprised.

A polynucleotide of the invention can comprise a targeting region comprising or consisting of a nucleotide sequence depicted in SEQ ID NO: 11, which sequence is complementary to nucleotides 22 to 40 of the 5'-UTR sequence as depicted in SEQ ID NO: 10. Alternatively, a polynucleotide of the invention can comprise a targeting region comprising or consisting of a nucleotide sequence having at least 70%, at least 75%, at least 80%, at least 85%, at least 90%, at least 95%, at least 97%, at least 99%, or 100% identity to the nucleotide sequence depicted in SEQ ID NO: 11.

**Table 4: Nucleotide sequence for targeting nucleotides 22 to 40 of the 5'-UTR:**

| **SEQ ID NO:** | **5'elongation** | **AS-MDM2 22-40 (iso1-2) (SEQ ID NO: 11)** | **3'elongation** |
|---|---|---|---|
| 11 | | CAGCCAAGCTCGCCGCGGT | |

For increasing specificity and/or efficiency the nucleotide sequence of the targeting region of said polynucleotide can be further elongated by one or more additional nucleotides. The nucleotide sequence depicted in SEQ ID NO: 11 can be elongated in one or both directions, *i.e.,* 3' and/or 5' relative to the said sequence.

Thus, the nucleotide sequence as set forth in SEQ ID NO: 11 can be additionally flanked 3' in a contiguous manner by one or more nucleotides for targeting nucleotide 21 up to at most nucleotide 1 or up to at most nucleotide 10 of the sequence depicted in SEQ ID NO: 10, respectively, or the respective nucleotides in variant sequences. The first nucleotide added 3' to the sequence depicted in SEQ ID NO: 11 is for targeting the nucleotide at position 21 of the sequence depicted in SEQ ID NO: 10. The second nucleotide added to the elongated sequence (*i.e.,* SEQ ID NO: 11 plus 1 nucleotide) is for targeting the nucleotide at position 20 of the sequence depicted in SEQ ID NO: 10, the third nucleotide added to the elongated sequence (*i.e.,* SEQ ID NO: 11 plus 2 nucleotides) is for targeting the nucleotide at position 19 of the sequence depicted in SEQ ID NO: 10, the forth nucleotide added to the elongated sequence (*i.e.,* SEQ ID NO: 11 plus 3 nucleotide) is for targeting the nucleotide at position 18 of the sequence depicted in SEQ ID NO: 10, and so forth. Thus, the nucleotide sequence as set forth in SEQ ID NO: 11 can be 3' elongated in a contiguous manner by 1, 2, 3, 4, 5, 6, 7, 8, 9, 10, 11, 12, 13, 14, 15, 16, 17, 18, 19, 20 or 21 nucleotides. Each nucleotide added 3' can be complementary or not to its respective nucleotide in the sequence depicted in SEQ ID NO: 10. For example, the first nucleotide added at the 3' end of the sequence (5'-CAGCCAAGCTCGCCGCGGT-3') depicted in SEQ ID NO: 11 is a nucleotide having the nucleobase guanin for targeting the nucleotide having the nucleobase cytosine at position 21 of the target strand (shown from 5' to 3' in the sequence below), the second nucleotide added at the 3' end of the elongated sequence is a nucleotide having the nucleobase cytosine for targeting the nucleotide having the nucleobase guanin at position 20 of the target strand, the third nucleotide added at the 3' end of the elongated sequence is a nucleotide having the nucleobase cytosine for targeting the nucleotide having the nucleobase guanin at position 19 of the target strand, the fourth nucleotide added at the 3' end of the elongated sequence is a nucleotide having the nucleobase thymine/uracil for targeting the nucleotide having the nucleobase adenine at position 18 of the target strand, and so forth.

(SEQ ID NO: 11)      3′-TGGCGCCGCTCGAACCGAC-5′
                          |||||||||||||||||||
5′-GTAGGGGGCGCGCACCGAGGCACCGCGGCGAGCTTGGCTGCTTCTGGGGCCTGTGTGGCCCTGTGTG
TCGGAAAGATGGAGCAAGAAGCCGAGCCCGAGGGGCGGCCGCGACCCCTCTGACCGAGATCCTGCTGCTT
TCGCAGCCAGGAGCACCGTCCCTCCCCGGATTAGTGCGTACGAGCGCCCAGTGCCCTGGCCCGGAGAGTG
GAATGATCCCCGAGGCCCAGGGCGTCGTGCTTCCGCGCGCCCCGTGAAGGAAACTGGGGAGTCTTGAGGG
ACCCCCGACTCCAAGCGCGAAAACCCCGGATGGTGAGGAGCAGGCAA-3′
(SEQ ID NO: 10)

Preferred nucleotide sequences of targeting regions which are elongated in 3' direction comprise any of the sequences as shown in Table 5, or any variants thereof:

**Table 5:**

| **SEQ ID NO:** | **5'elongation** | **AS-MDM2 22-40 (iso1-2) (SEQ ID NO: 11)** | **3'elongation** |
|---|---|---|---|
| 12 | | CAGCCAAGCTCGCCGCGGT | GCCTCGGTGCGC |
| 13 | | CAGCCAAGCTCGCCGCGGT | GCCTCGGTGCGCGCCCCCTAC |

These preferred 3' elongations are for targeting the portion comprising nucleotides 10 to 40 (SEQ ID NO: 12) or nucleotides 1 to 40 (SEQ ID NO: 13) of the 5'-UTR of isoforms 1 and 2 of MDM2. Also encompassed by the present invention are variants of the sequences depicted in SEQ ID NO: 12 or 13.

Other nucleotide sequences of the targeting region which are elongated in 3' direction comprise the sequences for targeting nucleotides 2 to 40, nucleotides 3 to 40, nucleotides 4 to 40, nucleotides 5 to 40, nucleotides 6 to 40, nucleotides 7 to 40, nucleotides 8 to 40, nucleotides 9 to 40, nucleotides 11 to 40, nucleotides 12 to 40, nucleotides 13 to 40, nucleotides 14 to 40, nucleotides 15 to 40, nucleotides 16 to 40, nucleotides 17 to 40, nucleotides 18 to 40, nucleotides 19 to 40, each of the sequence depicted in SEQ ID NO: 10, and any variants of these portions.

Alternatively, or in addition to each possible elongation at 3', the nucleotide sequence depicted in SEQ ID NO: 11 can be flanked 5' in a contiguous manner by one or more nucleotides for targeting nucleotide 41 up to at most nucleotide 60, preferably up to at most nucleotide 50 of the sequence depicted in SEQ ID NO: 10, respectively. The first nucleotide added 5' to the sequence as set forth in SEQ ID NO: 11 is for targeting the nucleotide at position 41 of the sequence depicted in SEQ ID NO: 10. The second nucleotide added to the elongated sequence (*i.e.,* SEQ ID NO: 11 plus 1 nucleotide) is for targeting the nucleotide at position 42 of the sequence depicted in SEQ ID NO: 10, the third nucleotide added to the elongated sequence (*i.e.,* SEQ ID NO: 11 plus 2 nucleotides) is for targeting the nucleotide at position 43 of the sequence depicted in SEQ ID NO: 10, the forth nucleotide added to the elongated sequence (*i.e.,* SEQ ID NO: 11 plus 3 nucleotide) is for targeting the nucleotide at position 44 of the sequence depicted in SEQ ID NO: 10, and so forth. Thus, the nucleotide sequence as set forth in SEQ ID NO: 11 can be 5' elongated in a contiguous manner by 1, 2, 3, 4, 5, 6, 7, 8, 9, 10, 11, 12, 13, 14, 15, 16, 17, 18, 19, or 20 nucleotides. Each nucleotide added 5' can be complementary or not to its respective nucleotide in the sequence depicted in SEQ ID NO: 10. For example, the first nucleotide added at the 5' end of the sequence (5'-CAGCCAAGCTCGCCGCGGT-3') depicted in SEQ ID NO: 11 is a nucleotide having the nucleobase guanin for targeting the nucleotide having the nucleobase cytosine at position 41 of the target strand (shown from 5' to 3' in the sequence below), the second nucleotide added at the 5' end of the elongated sequence is a nucleotide having the nucleobase adenine for targeting the nucleotide having the nucleobase thymine/uracil at position 42 of the target strand, the third nucleotide added at the 5' end of the elongated sequence is a nucleotide having the nucleobase adenine for targeting the nucleotide having the nucleobase thymine/uracil at position 43 of the target strand, the fourth nucleotide added at the 5' end of the elongated sequence is a nucleotide having the nucleobase guanin for targeting the nucleotide having the nucleobase cytosine at position 44 of the target strand, and so forth.

(SEQ ID NO: 11)      3′-TGGCGCCGCTCGAACCGAC-5′
                           |||||||||||||||||||
5′-GTAGGGGGCGCGCACCGAGGCACCGCGGCGAGCTTGGCTGCTTCTGGGGCCTGTGTGGCCCTGTGTG
TCGGAAAGATGGAGCAAGAAGCCGAGCCCGAGGGGCGGCCGCGACCCCTCTGACCGAGATCCTGCTGCTT
TCGCAGCCAGGAGCACCGTCCCTCCCCGGATTAGTGCGTACGAGCGCCCAGTGCCCTGGCCCGGAGAGTG
GAATGATCCCCGAGGCCCAGGGCGTCGTGCTTCCGCGCGCCCCGTGAAGGAAACTGGGGAGTCTTGAGGG
ACCCCCGACTCCAAGCGCGAAAACCCCGGATGGTGAGGAGCAGGCAA-3′
(SEQ ID NO: 10)

Preferred nucleotide sequences of targeting regions which are elongated in 5' direction comprise any of the sequences as shown in Table 6, or any variants thereof:

**Table 6:**

| **SEQ ID NO:** | **5'elongation** | **AS-MDM2 22-40 (iso1-2) (SEQ ID NO: 11)** | **3'elongation** |
|---|---|---|---|
| 14 | GCCCCAGAAG | CAGCCAAGCTCGCCGCGGT | |
| 15 | GGCCACACAGGCCCCAGAAG | CAGCCAAGCTCGCCGCGGT | |

These preferred 3' elongations are for targeting the portion comprising nucleotides 22 to 50 (SEQ ID NO: 14) or nucleotides 22 to 60 (SEQ ID NO: 15) of the 5'-UTR of isoforms 1 and 2 of MDM2. Also encompassed by the present invention are variants of the sequences depicted in SEQ ID NO: 14 or 15.

Other preferred nucleotide sequences of the targeting region which are elongated in 5' direction comprise the sequences for targeting nucleotides 22 to 59, nucleotides 22 to 58, nucleotides 22 to 57, nucleotides 22 to 56, nucleotides 22 to 55, nucleotides 22 to 54, nucleotides 22 to 53, nucleotides 22 to 52, nucleotides 22 to 51, nucleotides 22 to 49, nucleotides 22 to 48, nucleotides 22 to 47, nucleotides 22 to 46, nucleotides 22 to 45, nucleotides 22 to 44, nucleotides 22 to 43, nucleotides 22 to 42, nucleotides 22 to 41, each of the sequence depicted in SEQ ID NO: 10, and any variants of these portions.

The polynucleotides of the present invention can also comprise a targeting region comprising or consisting of a nucleotide sequence which is the result of any combination between above mentioned 5' elongations and 3' elongations, including variants of these combined nucleotide sequences.

A preferred targeting region comprises or consists of a nucleotide sequence depicted in SEQ ID NO: 13.

A polynucleotide of the invention can comprise a targeting region comprising or consisting of a nucleotide sequence depicted in SEQ ID NO: 16, which sequence is complementary to nucleotides 67 to 86 of the 5'-UTR sequence as depicted in SEQ ID NO. 10. Alternatively, a polynucleotide of the invention can comprise a targeting region comprising or consisting of a nucleotide sequence having at least 70%, at least 75%, at least 80%, at least 85%, at least 90%, at least 95%, at least 97%, at least 99%, or 100% identity to the nucleotide sequence depicted in SEQ ID NO: 16.

**Table 7: Nucleotide sequence for targeting nucleotides 67 to 86 of the 5'-UTR:**

| **SEQ ID NO:** | **5'elongation** | **AS-MDM2 67-86 (iso1-2) (SEQ ID NO: 16)** | **3'elongation** |
|---|---|---|---|
| 16 | | TCXTGCTCCATCTTTCCGAC | |

In the nucleotide sequence depicted in SEQ ID NO: 16 the nucleotide "X" has the meaning C, G, A or T. In one embodiment the nucleotide "X" is A or T.

For increasing specificity and/or efficiency the nucleotide sequence of the targeting region of said polynucleotide can be further elongated by one or more additional nucleotides. The nucleotide sequence depicted in SEQ ID NO: 16 can be elongated in one or both directions, *i.e.,* 3' and/or 5' relative to the said sequence.

Thus, the nucleotide sequence as set forth in SEQ ID NO: 16 can be additionally flanked 3' in a contiguous manner by one or more nucleotides for targeting nucleotide 66 up to at most nucleotide 47, preferably up to at most nucleotide 57 of the sequence depicted in SEQ ID NO: 10, respectively, or the respective nucleotides in variant sequences. The first nucleotide added 3' to the sequence depicted in SEQ ID NO: 16 is for targeting the nucleotide at position 66 of the sequence depicted in SEQ ID NO: 10. The second nucleotide added to the elongated sequence (*i.e.,* SEQ ID NO: 16 plus 1 nucleotide) is for targeting the nucleotide at position 65 of the sequence depicted in SEQ ID NO: 10, the third nucleotide added to the elongated sequence (*i.e.,* SEQ ID NO: 16 plus 2 nucleotides) is for targeting the nucleotide at position 64 of the sequence depicted in SEQ ID NO: 10, the forth nucleotide added to the elongated sequence (*i.e.,* SEQ ID NO: 16 plus 3 nucleotide) is for targeting the nucleotide at position 63 of the sequence depicted in SEQ ID NO: 10, and so forth. Thus, the nucleotide sequence as set forth in SEQ ID NO: 16 can be 3' elongated in a contiguous manner by 1, 2, 3, 4, 5, 6, 7, 8, 9, 10, 11, 12, 13, 14, 15, 16, 17, 18, 19, or 20 nucleotides. Each nucleotide added 3' can be complementary or not to its respective nucleotide in the sequence depicted in SEQ ID NO: 10.

Preferred nucleotide sequences of targeting regions which are elongated in 3' direction comprise any of the sequences as shown in Table 8, or any variants thereof:

**Table 8:**

| **SEQ ID NO:** | **5'elongation** | **AS-MDM2 67-86 (iso1-2) (SEQ ID NO: 16)** | **3'elongation** |
|---|---|---|---|
| 17 | | TCNTGCTCCATCTTTCCGAC | ACACAGGGCC |
| 18 | | TCNTGCTCCATCTTTCCGAC | ACACAGGGCCACACAGGCCC |

In the nucleotide sequences depicted in SEQ ID NO: 17 or 18 the nucleotide "N" has the meaning A or C or G or T/U. In one embodiment the nucleotide "N" is A or T/U. In one embodiment the nucleotide "N" is A.

These preferred 3' elongations are for targeting the portion comprising nucleotides 57 to 86 (SEQ ID NO: 17) or nucleotides 47 to 86 (SEQ ID NO: 18) of the 5'-UTR of isoforms 1 and 2 of MDM2. Also encompassed by the present invention are variants of the sequences depicted in SEQ ID NO: 17 or 18.

Other preferred nucleotide sequences of the targeting region which are elongated in 3' direction comprise the sequences for targeting nucleotides 48 to 86, nucleotides 49 to 86, nucleotides 50 to 86, nucleotides 51 to 86, nucleotides 52 to 86, nucleotides 53 to 86, nucleotides 54 to 86, nucleotides 55 to 86, nucleotides 56 to 86, nucleotides 58 to 86, nucleotides 59 to 86, nucleotides 60 to 86, nucleotides 61 to 86, nucleotides 62 to 86, nucleotides 63 to 86, nucleotides 64 to 86, nucleotides 65 to 86, nucleotides 66 to 86 each of the sequence depicted in SEQ ID NO: 10, and any variants of these portions.

Alternatively, or in addition to each possible elongation at 3', the nucleotide sequence depicted in SEQ ID NO: 16 can be flanked 5' in a contiguous manner by one or more nucleotides for targeting nucleotide 87 up to at most nucleotide 106, preferably up to at most nucleotide 96 of the sequence depicted in SEQ ID NO: 10, respectively. The first nucleotide added 5' to the sequence as set forth in SEQ ID NO: 16 is for targeting the nucleotide at position 87 of the sequence depicted in SEQ ID NO: 10. The second nucleotide added to the elongated sequence (*i.e.,* SEQ ID NO: 16 plus 1 nucleotide) is for targeting the nucleotide at position 88 of the sequence depicted in SEQ ID NO: 10, the third nucleotide added to the elongated sequence (*i.e.,* SEQ ID NO: 16 plus 2 nucleotides) is for targeting the nucleotide at position 89 of the sequence depicted in SEQ ID NO: 10, the forth nucleotide added to the elongated sequence (*i.e.,* SEQ ID NO: 16 plus 3 nucleotide) is for targeting the nucleotide at position 90 of the sequence depicted in SEQ ID NO: 10, and so forth. Thus, the nucleotide sequence as set forth in SEQ ID NO: 16 can be 5' elongated in a contiguous manner by 1, 2, 3, 4, 5, 6, 7, 8, 9, 10, 11, 12, 13, 14, 15, 16, 17, 18, 19, or 20 nucleotides. Each nucleotide added 5' can be complementary or not to its respective nucleotide in the sequence depicted in SEQ ID NO: 10.

Preferred nucleotide sequences of the targeting region which are elongated in 5' direction comprise any of the sequences as shown in Table 9, or any variants thereof:

**Table 9:**

| **SEQ ID NO:** | **5'elongation** | **AS-MDM2 67-86 (iso1-2) (SEQ ID NO: 16)** | **3'elongation** |
|---|---|---|---|
| 19 | GGGCTCGGCT | TCNTGCTCCATCTTTCCGAC | |
| 20 | GCCGCCCCTCGGGCTCGGCT | TCNTGCTCCATCTTTCCGAC | |

In the nucleotide sequences depicted in SEQ ID NO: 19 or 20 the nucleotide "N" has the meaning A or C or G or T/U. In one embodiment the nucleotide "N" is A or T/U. In one embodiment the nucleotide "N" is A.

These preferred 5' elongations are for targeting the portion comprising nucleotides 67 to 96 (SEQ ID NO: 19) or nucleotides 67 to 106 (SEQ ID NO: 20) the 5'-UTR of isoforms 1 and 2 of MDM2. Also encompassed by the present invention are variants of the sequences depicted in SEQ ID NO: 19 or 20.

Other preferred nucleotide sequences of the targeting region which are elongated in 5' direction comprise the sequences for targeting nucleotides 67 to 105, nucleotides 67 to 104, nucleotides 67 to 103, nucleotides 67 to 102, nucleotides 67 to 101, nucleotides 67 to 100, nucleotides 67 to 99, nucleotides 67 to 98, nucleotides 67 to 97, nucleotides 67 to 95, nucleotides 67 to 94, nucleotides 67 to 93, nucleotides 67 to 92, nucleotides 67 to 91, nucleotides 67 to 90, nucleotides 67 to 89, nucleotides 67 to 88, nucleotides 67 to 87, each of the sequence depicted in SEQ ID NO: 10, and any variants of these portions.

The polynucleotides of the present invention can also comprise a targeting region comprising or consisting of a nucleotide sequence which is the result of any combination between above mentioned 5' elongations and 3' elongations, including variants of these combined nucleotide sequences.

A preferred (combined) targeting region comprises or consists of a nucleotide sequence depicted in SEQ ID NO: 21 and/or Table 10, or any variants thereof. The nucleotide sequence is for targeting the portion comprising nucleotides 57 to 96 the 5'-UTR of isoforms 1 and 2 of MDM2.

**Table 10:**

| **SEQ ID NO:** | **5'elongation** | **AS-MDM2 67-86 (iso1-2) (SEQ ID NO: 16)** | **3'elongation** |
|---|---|---|---|
| 21 | GGGCTCGGCT | TCNTGCTCCATCTTTCCGAC | ACACAGGGCC |

In the nucleotide sequence depicted in SEQ ID NO: 21 the nucleotide "N" has the meaning A or C or G or T/U. In one embodiment the nucleotide "N" is A or T/U. In one embodiment the nucleotide "N" is A.

A polynucleotide of the invention can comprise a targeting region comprising or consisting of a nucleotide sequence depicted in SEQ ID NO: 22, which sequence is complementary to nucleotides 138 to 157 of the 5'-UTR sequence as depicted in SEQ ID NO: 10. Alternatively, a polynucleotide of the invention can comprise a targeting region comprising or consisting of a nucleotide sequence having at least 70%, at least 75%, at least 80%, at least 85%, at least 90%, at least 95%, at least 97%, at least 99%, or 100% identity to the nucleotide sequence depicted in SEQ ID NO: 22.

**Table 11: Nucleotide sequence for targeting nucleotides 138 to 157 of the 5'-UTR:**

| **SEQ ID NO:** | **5'elongation** | **AS-MDM2 138-157 (iso1-2) (SEQ ID NO: 22)** | **3'elongation** |
|---|---|---|---|
| 22 | | GACGGTGCTCCTGGCTGCGA | |

For increasing specificity and/or efficiency the nucleotide sequence of the targeting region of said polynucleotide can be further elongated by one or more additional nucleotides. The nucleotide sequence depicted in SEQ ID NO: 22 can be elongated in one or both directions, *i.e.,* 3' and/or 5' relative to the said sequence.

Thus, the nucleotide sequence as set forth in SEQ ID NO: 22 can be additionally flanked 3' in a contiguous manner by one or more nucleotides for targeting nucleotide 137 up to at most nucleotide 118, preferably up to at most nucleotide 128 of the sequence depicted in SEQ ID NO: 10, respectively, or the respective nucleotides in variant sequences. The first nucleotide added 3' to the sequence depicted in SEQ ID NO: 22 is for targeting the nucleotide at position 137 of the sequence depicted in SEQ ID NO: 10. The second nucleotide added to the elongated sequence (*i.e.,* SEQ ID NO: 22 plus 1 nucleotide) is for targeting the nucleotide at position 136 of the sequence depicted in SEQ ID NO: 10, the third nucleotide added to the elongated sequence (*i.e.,* SEQ ID NO: 22 plus 2 nucleotides) is for targeting the nucleotide at position 135 of the sequence depicted in SEQ ID NO: 10, the forth nucleotide added to the elongated sequence (*i.e.,* SEQ ID NO: 22 plus 3 nucleotide) is for targeting the nucleotide at position 134 of the sequence depicted in SEQ ID NO: 10, and so forth. Thus, the nucleotide sequence as set forth in SEQ ID NO: 22 can be 3' elongated in a contiguous manner by 1, 2, 3, 4, 5, 6, 7, 8, 9, 10, 11, 12, 13, 14, 15, 16, 17, 18, 19, or 20 nucleotides. Each nucleotide added 3' can be complementary or not to its respective nucleotide in the sequence depicted in SEQ ID NO: 10.

Preferred nucleotide sequences of targeting regions which are elongated in 3' direction comprise any of the sequences as shown in Table 12, or any variants thereof:

**Table 12:**

| **SEQ ID NO:** | **5'elongation** | **AS-MDM2 138-157 (iso1-2) (SEQ ID NO: 22)** | **3'elongation** |
|---|---|---|---|
| 23 | | GACGGTGCTCCTGGCTGCGA | AAGCAGCAGG |
| 24 | | GACGGTGCTCCTGGCTGCGA | AAGCAGCAGGATCTCGGTCA |

These preferred 3' elongations are for targeting the portion comprising nucleotides 128 to 157 (SEQ ID NO: 23) or nucleotides 118 to 157 (SEQ ID NO: 24) of the 5'-UTR of isoforms 1 and 2 of MDM2. Also encompassed by the present invention are variants of the sequences depicted in SEQ ID NO: 23 or 24.

Other preferred nucleotide sequences of the targeting region which are elongated in 3' direction comprise the sequences for targeting nucleotides 119 to 157, nucleotides 120 to 157, nucleotides 121 to 157, nucleotides 122 to 157, nucleotides 123 to 157, nucleotides 124 to 157, nucleotides 125 to 157, nucleotides 126 to 157, nucleotides 127 to 157, nucleotides 129 to 157, nucleotides 130 to 157, nucleotides 131 to 157, nucleotides 132 to 157, nucleotides 133 to 157, nucleotides 134 to 157, nucleotides 135 to 157, nucleotides 136 to 157, nucleotides 137 to 157, each of the sequence depicted in SEQ ID NO: 10, and any variants of these portions.

Alternatively, or in addition to each possible elongation at 3', the nucleotide sequence depicted in SEQ ID NO: 22 can be flanked 5' in a contiguous manner by one or more nucleotides for targeting nucleotide 158 up to at most nucleotide 177, preferably up to at most nucleotide 176 of the sequence depicted in SEQ ID NO: 10, respectively. The first nucleotide added 5' to the sequence as set forth in SEQ ID NO: 22 is for targeting the nucleotide at position 158 of the sequence depicted in SEQ ID NO: 10. The second nucleotide added to the elongated sequence (*i.e.,* SEQ ID NO: 22 plus 1 nucleotide) is for targeting the nucleotide at position 159 of the sequence depicted in SEQ ID NO: 10, the third nucleotide added to the elongated sequence (*i.e.,* SEQ ID NO: 22 plus 2 nucleotides) is for targeting the nucleotide at position 160 of the sequence depicted in SEQ ID NO: 10, the forth nucleotide added to the elongated sequence (*i.e.,* SEQ ID NO: 22 plus 3 nucleotide) is for targeting the nucleotide at position 161 of the sequence depicted in SEQ ID NO: 10, and so forth. Thus, the nucleotide sequence as set forth in SEQ ID NO: 22 can be 5' elongated in a contiguous manner by 1, 2, 3, 4, 5, 6, 7, 8, 9, 10, 11, 12, 13, 14, 15, 16, 17, 18, 19, or 20 nucleotides. Each nucleotide added 5' can be complementary or not to its respective nucleotide in the sequence depicted in SEQ ID NO: 10.

Preferred nucleotide sequences of targeting regions which are elongated in 5' direction comprise any of the sequences as shown in Table 13, or any variants thereof:

**Table 13:**

| **SEQ ID NO:** | **5'elongation** | **AS-MDM2 138-157 (iso1-2) (SEQ ID NO: 22)** | **3'elongation** |
|---|---|---|---|
| **25** | TCCGGGGAGG | GACGGTGCTCCTGGCTGCGA | |
| **26** | TACGCACTAATCCGGGGAGG | GACGGTGCTCCTGGCTGCGA | |

These preferred 5' elongations are for targeting the portion comprising nucleotides 138 to 167 (SEQ ID NO: 25) or nucleotides 138 to 177 (SEQ ID NO: 26) of the 5'-UTR of isoforms 1 and 2 of MDM2. Also encompassed by the present invention are variants of the sequences depicted in SEQ ID NO: 25 or 26.

Other preferred nucleotide sequences of the targeting region which are elongated in 5' direction comprise the sequences for targeting nucleotides 138 to 176, nucleotides 138 to 175, nucleotides 138 to 174, nucleotides 138 to 173, nucleotides 138 to 172, nucleotides 138 to 171, nucleotides 138 to 170, nucleotides 138 to 169, nucleotides 138 to 168, nucleotides 138 to 166, nucleotides 138 to 165, nucleotides 138 to 164, nucleotides 138 to 163, nucleotides 138 to 162, nucleotides 138 to 161, nucleotides 138 to 160, nucleotides 138 to 159, nucleotides 138 to 158, each of the sequence depicted in SEQ ID NO: 10, and any variants of these portions.

The polynucleotides of the present invention can also comprise a targeting region comprising or consisting of a nucleotide sequence which is the result of any combination between above mentioned 5' elongations and 3' elongations, including variants of these combined nucleotide sequences.

A preferred (combined) targeting regions comprises or consists of a nucleotide sequence depicted in any of SEQ ID NO: 27 and/or Table 14, or any variants thereof. The nucleotide sequence is for targeting the portion comprising nucleotides 128 to 167 the 5'-UTR of isoforms 1 and 2 of MDM2.

**Table 14:**

| **SEQ ID NO:** | **5'elongation** | **AS-MDM2 138-157 (iso1-2) (SEQ ID NO: 22)** | **3'elongation** |
|---|---|---|---|
| 27 | TCCGGGGAGG | GACGGTGCTCCTGGCTGCGA | AAGCAGCAGG |

A polynucleotide of the invention can comprise a targeting region comprising or consisting of a nucleotide sequence depicted in SEQ ID NO: 28, which sequence is complementary to nucleotides 301 to 318 of the 5'-UTR sequence as depicted in SEQ ID NO: 10. Alternatively, a polynucleotide of the invention can comprise a targeting region comprising or consisting of a nucleotide sequence having at least 70%, at least 75%, at least 80%, at least 85%, at least 90%, at least 95%, at least 97%, at least 99%, or 100% identity to the nucleotide sequence depicted in SEQ ID NO: 28.

**Table 15: Nucleotide sequence for targeting nucleotides 301 to 318 of the 5'-UTR:**

| **SEQ ID NO:** | **5'elongation** | **AS-MDM2 301-318 (iso1-2) (SEQ ID NO: 28)** | **3'elongation** |
|---|---|---|---|
| 28 | | GCTCCTCACCATCCGGGG | |

For increasing specificity and/or efficiency the nucleotide sequence of the targeting region of said polynucleotide can be further elongated by one or more additional nucleotides. The nucleotide sequence depicted in SEQ ID NO: 28 can be elongated in one or both directions, *i.e.,* 3' and/or 5' relative to the said sequence.

Thus, the nucleotide sequence as set forth in SEQ ID NO: 28 can be additionally flanked 3' in a contiguous manner by one or more nucleotides for targeting nucleotide 301 up to at most nucleotide 269, preferably up to at most nucleotide 279 of the sequence depicted in SEQ ID NO: 10, respectively, or the respective nucleotides in variant sequences. The first nucleotide added 3' to the sequence depicted in SEQ ID NO: 28 is for targeting the nucleotide at position 300 of the sequence depicted in SEQ ID NO: 10. The second nucleotide added to the elongated sequence (*i.e.,* SEQ ID NO: 28 plus 1 nucleotide) is for targeting the nucleotide at position 299 of the sequence depicted in SEQ ID NO: 10, the third nucleotide added to the elongated sequence (*i.e.,* SEQ ID NO: 28 plus 2 nucleotides) is for targeting the nucleotide at position 298 of the sequence depicted in SEQ ID NO: 10, the forth nucleotide added to the elongated sequence (*i.e.,* SEQ ID NO: 28 plus 3 nucleotide) is for targeting the nucleotide at position 297 of the sequence depicted in SEQ ID NO: 10, and so forth. Thus, the nucleotide sequence as set forth in SEQ ID NO: 28 can be 3' elongated in a contiguous manner by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, or 32 nucleotides. Each nucleotide added 3' can be complementary or not to its respective nucleotide in the sequence depicted in SEQ ID NO: 10.

Preferred nucleotide sequences of targeting regions which are elongated in 3' direction comprise any of the sequences as shown in Table 16, or any variants thereof:

**Table 16:**

| **SEQ ID NO:** | **5'elongation** | **AS-MDM2 301-318 (iso1-2) (SEQ ID NO: 28)** | **3'elongation** |
|---|---|---|---|
| 29 | | GCTCCTCACCATCCGGGG | TTNTCGCGCTTGGAGTCGGGGG |
| 30 | | GCTCCTCACCATCCGGGG | TTNTCGCGCTTGGAGTCGGGGGT CCCTCAAGA |

In the nucleotide sequences depicted in SEQ ID NO: 29 or 30 the nucleotide "N" has the meaning A or C or G or T/U. In one embodiment the nucleotide "N" is A or T/U. In one embodiment the nucleotide "N" is A.

These preferred 3' elongations are for targeting the portion comprising nucleotides 279 to 318 (SEQ ID NO: 29), or nucleotides 269 to 318 (SEQ ID NO: 30) of the 5'-UTR of isoforms 1 and 2 of MDM2 5'-UTR. Also encompassed by the present invention are variants of the sequences depicted in SEQ ID NO: 29 or 30.

Other preferred nucleotide sequences of the targeting region which are elongated in 3' direction comprise the sequences for targeting nucleotides 270 to 318, nucleotides 271 to 318, nucleotides 272 to 318, nucleotides 273 to 318, nucleotides 274 to 318, nucleotides 275 to 318, nucleotides 276 to 318, nucleotides 277 to 318, nucleotides 278 to 318, nucleotides 280 to 318, nucleotides 281 to 318, nucleotides 282 to 318, nucleotides 283 to 318, nucleotides 284 to 318, nucleotides 285 to 318, nucleotides 286 to 318, nucleotides 287 to 318, nucleotides 288 to 318, nucleotides 289 to 318, nucleotides 290 to 318, nucleotides 291 to 318, nucleotides 292 to 318, nucleotides 293 to 318, nucleotides 294 to 318, nucleotides 295 to 318, nucleotides 296 to 318, nucleotides 297 to 318, nucleotides 298 to 318, nucleotides 299 to 318, nucleotides 300 to 318, each of the sequence depicted in SEQ ID NO: 10, and any variants of these portions.

Alternatively, or in addition to each possible elongation at 3', the nucleotide sequence depicted in SEQ ID NO: 28 can be flanked 5' in a contiguous manner by one or more nucleotides for targeting nucleotide 301 up to at most nucleotide 324 of the sequence depicted in SEQ ID NO: 10, respectively. The first nucleotide added 5' to the sequence as set forth in SEQ ID NO: 28 is for targeting the nucleotide at position 319 of the sequence depicted in SEQ ID NO: 10. The second nucleotide added to the elongated sequence (*i.e*., SEQ ID NO: 28 plus 1 nucleotide) is for targeting the nucleotide at position 320 of the sequence depicted in SEQ ID NO: 10, the third nucleotide added to the elongated sequence (*i.e*., SEQ ID NO: 28 plus 2 nucleotides) is for targeting the nucleotide at position 321 of the sequence depicted in SEQ ID NO: 10, the forth nucleotide added to the elongated sequence (*i.e*., SEQ ID NO: 28 plus 3 nucleotide) is for targeting the nucleotide at position 322 of the sequence depicted in SEQ ID NO: 10, and so forth. Thus, the nucleotide sequence as set forth in SEQ ID NO: 28 can be 5' elongated in a contiguous manner by 1, 2, 3, 4, 5, or 6 nucleotides. Each nucleotide added 5' can be complementary or not to its respective nucleotide in the sequence depicted in SEQ ID NO: 10.

A preferred nucleotide sequence of the targeting region which is elongated in 5' direction comprise the sequence as shown in Table 17, or any variants thereof:

**Table 17:**

| **SEQ ID NO:** | **5'elongation** | **AS-MDM2 301-318 (iso1-2) (SEQ ID NO: 28)** | **3'elongation** |
|---|---|---|---|
| 31 | TTGCCT | GCTCCTCACCATCCGGGG | |

This preferred 5' elongation is for targeting the portion comprising nucleotides 301 to 324 (SEQ ID NO: 31) of the 5'-UTR of isoforms 1 and 2 of MDM2. Also encompassed by the present invention are variants of the sequences depicted in SEQ ID NO: 31.

Other preferred nucleotide sequences of the targeting region which are elongated in 5' direction comprise the sequences for targeting nucleotides 301 to 319, nucleotides 301 to 320, nucleotides 301 to 321, nucleotides 301 to 322, nucleotides 301 to 323, each of the sequence depicted in SEQ ID NO: 10, and any variants of these portions.

The polynucleotides of the present invention can also comprise a targeting region comprising or consisting of a nucleotide sequence which is the result of any combination between above mentioned 5' elongations and 3' elongations, including variants of these combined nucleotide sequences.

A preferred targeting region comprises or consists of a nucleotide sequence depicted in SEQ ID NO: 29 or any variants thereof.

In one embodiment, the AUG-proximal region of the 5'-UTR is complementary to at least the nucleotides covering the start codon AUG of the main ORF, wherein the A nucleotide of the AUG start codon is referred to as the nucleotide at position +1, at least nucleotides -3 to -1 of the 5'-UTR nucleotide sequence as depicted in SEQ ID NO: 10, and at least nucleotides +4 to +9 of the main ORF nucleotide sequence. The nucleotides -3 to -1 of the 5'-UTR nucleotide sequence are nucleotides 322 to 324 of the 5'-UTR nucleotide sequence as depicted in SEQ ID NO: 10. In one embodiment, the AUG-proximal region of the 5'-UTR complementary to at least the nucleotides - 3 to +9 is flanked on one or both ends by a flanking region, wherein each flanking region comprises one or more additional nucleotides, and each additional nucleotide is complementary or non-complementary to the corresponding nucleotide of the nucleotides flanking the portion of the AUG-proximal region of the 5'-UTR to which the nucleotide sequence of the targeting region is complementary.

The AUG-proximal region of the 5'-UTR complementary to at least the nucleotides -3 to +9 can be additionally flanked 3' in a contiguous manner by one or more nucleotides for targeting nucleotide 321 up to at most nucleotide 291, preferably up to at most nucleotide 301 of the sequence depicted in SEQ ID NO: 10, respectively, or the respective nucleotides in variant sequences.

Alternatively, or in addition the AUG-proximal region of the 5'-UTR complementary to at least the nucleotides -3 to +9 can be additionally flanked 5' in a contiguous manner by one or more nucleotides for targeting nucleotide +10 up to at most nucleotide +30, preferably up to at most nucleotide +20 of the main ORF, or the respective nucleotides in variant sequences.

The polynucleotides of the present invention can also comprise a targeting region comprising or consisting of a nucleotide sequence which is the result of any combination between above mentioned 5' elongations and 3' elongations, including variants of these combined nucleotide sequences.

In one embodiment, the targeting region comprises the sequence as depicted in SEQ ID NO: 31, wherein the sequence is additionally flanked 5' in a contiguous manner by one or more nucleotides for targeting nucleotide +1 up to at most nucleotide +26, preferably up to at most nucleotide +16 of the main ORF.

In one embodiment, the target region is a 5'-UTR of the mRNAs encoding isoforms 3 to 6 of MDM2, or is a continuous or discontinuous portion of said 5'-UTR.

In one embodiment, the target sequence can be the complete 5'-UTR having or comprising a sequence depicted in SEQ ID NO: 32 or can be a variant of this depicted nucleotide sequence. Alternatively, the 5'-UTR having or comprising a sequence depicted in SEQ ID NO: 32 can be divided into portions and the target sequence comprises one or more of these portions or one or more segments of these portions.

According to the invention, the 5'-UTR target sequence can be selected from the following portions:
- a portion comprising nucleotides 1 to 61 of the sequence depicted in SEQ ID NO: 32 or a variant thereof;
- a portion comprising nucleotides 1 to 50 of the sequence depicted in SEQ ID NO: 32 or a variant thereof;
- a portion comprising nucleotides 1 to 40 of the sequence depicted in SEQ ID NO: 32 or a variant thereof;
- a portion comprising nucleotides 10 to 61 of the sequence depicted in SEQ ID NO: 32 or a variant thereof;
- a portion comprising nucleotides 10 to 50 of the sequence depicted in SEQ ID NO: 32 or a variant thereof;
- a portion comprising nucleotides 10 to 40 of the sequence depicted in SEQ ID NO: 32 or a variant thereof;
- a portion comprising nucleotides 22 to 61 of the sequence depicted in SEQ ID NO: 32 or a variant thereof;
- a portion comprising nucleotides 22 to 50 of the sequence depicted in SEQ ID NO: 32 or a variant thereof;
- a portion comprising nucleotides 22 to 40 of the sequence depicted in SEQ ID NO: 32 or a variant thereof.

According to the invention the targeting region of the polynucleotide may comprise or consist of a nucleotide sequence complementary to at least one of these portions or to at least one segment within these portions.

Preferred targeting region comprise or consist of a nucleotide sequence complementary to at least one continuous portion selected form the group consisting of nucleotides 1 to 40, nucleotides 22 to 40, nucleotides 22 to 61, nucleotides 32 to 67, nucleotides 44 to 67, each of the sequence depicted in SEQ ID NO: 32, and any variants of these portions.

According to one embodiment of the invention, the nucleotide sequence of the targeting region may comprise at least two segments, each segment complementary to a nucleotide sequence of the 5'-UTR, wherein the complementary nucleotide sequences of the 5'-UTR are not continuous. Within the nucleotide sequence of these targeting regions complementary to a discontinuous portion of the 5'-UTR one or more nucleotides, between two consecutive segments can be comprised.

A polynucleotide of the invention can comprise a targeting region comprising or consisting of a nucleotide sequence depicted in SEQ ID NO: 33, which sequence is complementary to nucleotides 22 to 40 of the 5'-UTR sequence as depicted in SEQ ID NO: 32. Alternatively, a polynucleotide of the invention can comprise a targeting region comprising or consisting of a nucleotide sequence having at least 70%, at least 75%, at least 80%, at least 85%, at least 90%, at least 95%, at least 97%, at least 99%, or 100% identity to the nucleotide sequence depicted in SEQ ID NO: 33.

**Table 18: Nucleotide sequence for targeting nucleotides 22 to 40 of the 5'-UTR:**

| **SEQ ID NO:** | **5'elongation** | **AS-MDM2 22-40 (iso3-6) (SEQ ID NO: 33)** | **3'elongation** |
|---|---|---|---|
| 33 | | CGTGCGTCCGTGCCCACAG | |

For increasing specificity and/or efficiency the nucleotide sequence of the targeting region of said polynucleotide can be further elongated by one or more additional nucleotides. The nucleotide sequence depicted in SEQ ID NO: 33 can be elongated in one or both directions, *i.e.,* 3' and/or 5' relative to the said sequence.

Thus, the nucleotide sequence as set forth in SEQ ID NO: 33 can be additionally flanked 3' in a contiguous manner by one or more nucleotides for targeting nucleotide 21 up to at most nucleotide 1 or nucleotide 10 of the sequence depicted in SEQ ID NO: 32, respectively, or the respective nucleotides in variant sequences. The first nucleotide added 3' to the sequence depicted in SEQ ID NO: 33 is for targeting the nucleotide at position 21 of the sequence depicted in SEQ ID NO: 32. The second nucleotide added to the elongated sequence (*i.e.,* SEQ ID NO: 33 plus 1 nucleotide) is for targeting the nucleotide at position 20 of the sequence depicted in SEQ ID NO: 32, the third nucleotide added to the elongated sequence (*i.e.,* SEQ ID NO: 33 plus 2 nucleotides) is for targeting the nucleotide at position 19 of the sequence depicted in SEQ ID NO: 32, the forth nucleotide added to the elongated sequence (*i.e.,* SEQ ID NO: 33 plus 3 nucleotide) is for targeting the nucleotide at position 18 of the sequence depicted in SEQ ID NO: 32, and so forth. Thus, the nucleotide sequence as set forth in SEQ ID NO: 33 can be 3' elongated in a contiguous manner by 1, 2, 3, 4, 5, 6, 7, 8, 9, 10, 11, 12, 13, 14, 15, 16, 17, 18, 19, 20 or 21 nucleotides. Each nucleotide added 3' can be complementary or not to its respective nucleotide in the sequence depicted in SEQ ID NO: 32.

(SEQ ID NO: 33)      3′-GACACCCGTGCCTGCGTGC-5′
                          |||||||||||||||||||
5′-AGTGGCGATTGGAGGGTAGACCTGTGGGCACGGACGCACGCCACTTTTTCTCTGCTGATCCAGG
CAA-3′ (SEQ ID NO: 32)

Preferred nucleotide sequences of targeting regions which are elongated in 3' direction comprise any of the sequences as shown in Table 19, or any variants thereof:

**Table 19:**

| **SEQ ID NO:** | **5'elongation** | **AS-MDM2 22-40 (iso3-6) (SEQ ID NO: 33)** | **3'elongation** |
|---|---|---|---|
| 34 | | CGTGCGTCCGTGCCCACAG | GTCTACCCTCCA |
| 35 | | CGTGCGTCCGTGCCCACAG | GTCTACCCTCCAATCGCCACT |

These preferred 3' elongations are for targeting the portion comprising nucleotides 10 to 40 (SEQ ID NO: 34) or nucleotides 1 to 40 (SEQ ID NO: 35) of the 5'-UTR of isoforms 3 to 6 of MDM2. Also encompassed by the present invention are variants of the sequences depicted in SEQ ID NO: 34 or 35.

Other nucleotide sequences of the targeting region which are elongated in 3' direction comprise the sequences for targeting nucleotides 2 to 40, nucleotides 3 to 40, nucleotides 4 to 40, nucleotides 5 to 40, nucleotides 6 to 40, nucleotides 7 to 40, nucleotides 8 to 40, nucleotides 9 to 40, nucleotides 11 to 40, nucleotides 12 to 40, nucleotides 13 to 40, nucleotides 14 to 40, nucleotides 15 to 40, nucleotides 16 to 40, nucleotides 17 to 40, nucleotides 18 to 40, nucleotides 19 to 40, each of the sequence depicted in SEQ ID NO: 32, and any variants of these portions.

Alternatively, or in addition to each possible elongation at 3', the nucleotide sequence depicted in SEQ ID NO: 33 can be flanked 5' in a contiguous manner by one or more nucleotides for targeting nucleotide 41 up to at most nucleotide 61, preferably up to at most nucleotide 51 of the sequence depicted in SEQ ID NO: 32, respectively. The first nucleotide added 5' to the sequence as set forth in SEQ ID NO: 33 is for targeting the nucleotide at position 41 of the sequence depicted in SEQ ID NO: 32. The second nucleotide added to the elongated sequence *(i.e.,* SEQ ID NO: 33 plus 1 nucleotide) is for targeting the nucleotide at position 42 of the sequence depicted in SEQ ID NO: 32, the third nucleotide added to the elongated sequence (*i.e.,* SEQ ID NO: 33 plus 2 nucleotides) is for targeting the nucleotide at position 43 of the sequence depicted in SEQ ID NO: 32, the forth nucleotide added to the elongated sequence *(i.e.,* SEQ ID NO: 33 plus 3 nucleotide) is for targeting the nucleotide at position 44 of the sequence depicted in SEQ ID NO: 32, and so forth. Thus, the nucleotide sequence as set forth in SEQ ID NO: 33 can be 5' elongated in a contiguous manner by 1, 2, 3, 4, 5, 6, 7, 8, 9, 10, 11, 12, 13, 14, 15, 16, 17, 18, 19, 20, or 21 nucleotides. Each nucleotide added 5' can be complementary or not to its respective nucleotide in the sequence depicted in SEQ ID NO: 32.

Preferred nucleotide sequences of targeting regions which are elongated in 5' direction comprise any of the sequences as shown in Table 20, or any variants thereof:

**Table 20:**

| **SEQ ID NO:** | **5'elongation** | **AS-MDM2 22-40 (iso3-6) (SEQ ID NO: 33)** | **3'elongation** |
|---|---|---|---|
| 36 | GAAAAAGTGG | CGTGCGTCCGTGCCCACAG | |
| 37 | GGATCAGCAGAGAAAAAGTGG | CGTGCGTCCGTGCCCACAG | |

These preferred 5' elongations are for targeting the portion comprising nucleotides 22 to 50 (SEQ ID NO: 36) or nucleotides 22 to 61 (SEQ ID NO: 37) of the 5'-UTR of isoforms 3 to 6 of MDM2. Also encompassed by the present invention are variants of the sequences depicted in SEQ ID NO: 36 or 37.

Other preferred nucleotide sequences of the targeting region which are elongated in 5' direction comprise the sequences for targeting nucleotides 22 to 60, nucleotides 22 to 59, nucleotides 22 to 58, nucleotides 22 to 57, nucleotides 22 to 56, nucleotides 22 to 55, nucleotides 22 to 54, nucleotides 22 to 53, nucleotides 22 to 52, nucleotides 22 to 51, nucleotides 22 to 49, nucleotides 22 to 48, nucleotides 22 to 47, nucleotides 22 to 46, nucleotides 22 to 45, nucleotides 22 to 44, nucleotides 22 to 43, nucleotides 22 to 42, nucleotides 22 to 41, each of the sequence depicted in SEQ ID NO: 32, and any variants of these portions.

The polynucleotides of the present invention can also comprise a targeting region comprising or consisting of a nucleotide sequence which is the result of any combination between above mentioned 5' elongations and 3' elongations, including variants of these combined nucleotide sequences.

Preferred targeting regions comprise or consist of a nucleotide sequence depicted in SEQ ID NO: 35 or SEQ ID NO: 37, or any variants thereof.

A polynucleotide of the invention can comprise a targeting region comprising or consisting of a nucleotide sequence depicted in SEQ ID NO: 38, which sequence is complementary to nucleotides 44 to 67 of the 5'-UTR sequence as depicted in SEQ ID NO: 32. Alternatively, a polynucleotide of the invention can comprise a targeting region comprising or consisting of a nucleotide sequence having at least 70%, at least 75%, at least 80%, at least 85%, at least 90%, at least 95%, at least 97%, at least 99%, or 100% identity to the nucleotide sequence depicted in SEQ ID NO: 38.

**Table 21: Nucleotide sequence for targeting nucleotides 44 to 67 of the 5'-UTR:**

| **SEQ ID NO:** | **5'elongation** | **AS-MDM2 44-67 (iso3-6) (SEQ ID NO: 38)** | **3'elongation** |
|---|---|---|---|
| 38 | | TTGCCTGGATCAGCAGAGAAAAAG | |

For increasing specificity and/or efficiency the nucleotide sequence of the targeting region of said polynucleotide can be further elongated by one or more additional nucleotides. The nucleotide sequence depicted in SEQ ID NO: 38 can be elongated in one or both directions, *i.e.,* 3' and/or 5' relative to the said sequence.

Thus, the nucleotide sequence as set forth in SEQ ID NO: 38 can be additionally flanked 3' in a contiguous manner by one or more nucleotides for targeting nucleotide 43 up to at most nucleotide 32 of the sequence depicted in SEQ ID NO: 32, or the respective nucleotides in variant sequences. The first nucleotide added 3' to the sequence depicted in SEQ ID NO: 38 is for targeting the nucleotide at position 43 of the sequence depicted in SEQ ID NO: 32. The second nucleotide added to the elongated sequence (*i.e.,* SEQ ID NO: 38 plus 1 nucleotide) is for targeting the nucleotide at position 42 of the sequence depicted in SEQ ID NO: 32, the third nucleotide added to the elongated sequence (*i.e.,* SEQ ID NO: 38 plus 2 nucleotides) is for targeting the nucleotide at position 41 of the sequence depicted in SEQ ID NO: 32, the forth nucleotide added to the elongated sequence (*i.e.,* SEQ ID NO: 38 plus 3 nucleotide) is for targeting the nucleotide at position 40 of the sequence depicted in SEQ ID NO: 32, and so forth. Thus, the nucleotide sequence as set forth in SEQ ID NO: 38 can be 3' elongated in a contiguous manner by 1, 2, 3, 4, 5, 6, 7, 8, 9, 10, 11, 12, 13, 14, 15, 16, 17, 18, 19, 20, 21 or 22 nucleotides.

Each nucleotide added 3' can be complementary or not to its respective nucleotide in the sequence depicted in SEQ ID NO: 32.

A preferred nucleotide sequence of the targeting region which is elongated in 3' direction comprise the sequence as shown in Table 22, or any variants thereof:

**Table 22:**

| **SEQ ID NO:** | **5'elongation** | **AS-MDM2 44-67 (iso3-6) (SEQ ID NO: 38)** | **3'elongation** |
|---|---|---|---|
| 39 | | TTGCCTGGATCAGCAGAGAAAAAG | ACCGCACGCA |

This preferred 3' elongation is for targeting the portion comprising nucleotides 32 to 67 (SEQ ID NO: 39) of the 5'-UTR of isoforms 3 to 6 of MDM2. Also encompassed by the present invention are variants of the sequences depicted in SEQ ID NO: 39.

Other preferred nucleotide sequences of the targeting region which are elongated in 3' direction comprise the sequences for targeting nucleotides 33 to 67, nucleotides 34 to 67, nucleotides 35 to 67, nucleotides 36 to 67, nucleotides 37 to 67, nucleotides 38 to 67, nucleotides 39 to 67, nucleotides 40 to 67, nucleotides 41 to 67, nucleotides 42 to 67, nucleotides 43 to 67, each of the sequence depicted in SEQ ID NO: 32, and any variants of these portions.

Alternatively, or in addition to each possible elongation at 3', the nucleotide sequence depicted in SEQ ID NO: 38 can be flanked 5' in a contiguous manner by one or more nucleotides. The 5' flanking region is complementary to at least the nucleotides covering the start codon AUG of the main ORF, wherein the A nucleotide of the AUG start codon is referred to as the nucleotide at position +1. In one embodiment the 5' flanking region is complementary to nucleotide +1 up to at most nucleotide +26, preferably up to at most nucleotide +16 of the ORF.

The polynucleotides of the present invention can also comprise a targeting region comprising or consisting of a nucleotide sequence which is the result of any combination between above mentioned 5' elongations and 3' elongations, including variants of these combined nucleotide sequences.

In one embodiment, an AUG-proximal region of the 5'-UTR is complementary to at least the nucleotides covering the start codon AUG of the main ORF, wherein the A nucleotide of the AUG start codon is referred to as the nucleotide at position +1, at least nucleotides -3 to -1 of the 5'-UTR nucleotide sequence as depicted in SEQ ID NO: 32, and at least nucleotides +4 to +9 of the ORF nucleotide sequence. The nucleotides -3 to -1 of the 5'-UTR nucleotide sequence are nucleotides 65 to 67 of the 5'-UTR nucleotide sequence as depicted in SEQ ID NO: 32. In one embodiment, the AUG-proximal region of the 5'-UTR complementary to at least the nucleotides - 3 to +9 is flanked on one or both ends by a flanking region, wherein each flanking region comprises one or more additional nucleotides, and each additional nucleotide is complementary or non-complementary to the corresponding nucleotide of the nucleotides flanking the portion of the AUG-proximal region of the 5'-UTR to which the nucleotide sequence of the targeting region is complementary.

The AUG-proximal region of the 5'-UTR complementary to at least the nucleotides -3 to +9 can be additionally flanked 3' in a contiguous manner by one or more nucleotides for targeting nucleotide 64 up to at most nucleotide 32, preferably up to at most nucleotide 44 of the sequence depicted in SEQ ID NO: 32, respectively, or the respective nucleotides in variant sequences.

Alternatively, or in addition the AUG-proximal region of the 5'-UTR complementary to at least the nucleotides -3 to +9 can be additionally flanked 5' in a contiguous manner by one or more nucleotides for targeting nucleotide +10 up to at most nucleotide +30, preferably up to at most nucleotide +20 of the ORF, or the respective nucleotides in variant sequences.

The polynucleotides of the present invention can also comprise a targeting region comprising or consisting of a nucleotide sequence which is the result of any combination between above mentioned 5' elongations and 3' elongations, including variants of these combined nucleotide sequences.

In one embodiment, the targeting region comprises the sequences as depicted in SEQ ID NO: 38, wherein the sequence is additionally flanked 5' in a contiguous manner by one or more nucleotides for targeting nucleotide +1 up to at most nucleotide +26, preferably up to at most nucleotide +16 of the ORF.

In another embodiment, the target region is a 5'-UTR of the mRNAs encoding human β-globin (HBB), or is a continuous or discontinuous portion of said 5'-UTR. The HBB gene has a short 5'-UTR of only 50 nucleotides and encodes the β-globin subunit of the α₂β₂ heterotetrameric hemoglobin.

In one embodiment, the target sequence can be the complete 5'-UTR having or comprising a sequence depicted in SEQ ID NO: 40 or can be a variant of this depicted nucleotide sequence. Alternatively, the 5'-UTR having or comprising a sequence depicted in SEQ ID NO: 40 can be divided into portions and the target sequence comprises one or more of these portions or one or more segments of these portions.

According to the invention, the 5'-UTR target sequence can be selected from the following portions:
- a portion comprising nucleotides 1 to 50 of the sequence depicted in SEQ ID NO: 40 or a variant thereof;
- a portion comprising nucleotides 1 to 44 of the sequence depicted in SEQ ID NO: 40 or a variant thereof;
- a portion comprising nucleotides 10 to 50 of the sequence depicted in SEQ ID NO: 40 or a variant thereof;
- a portion comprising nucleotides 10 to 44 of the sequence depicted in SEQ ID NO: 40 or a variant thereof;
- a portion comprising nucleotides 45 to 50 of the sequence depicted in SEQ ID NO: 40 or a variant thereof.

According to the invention the targeting region of the polynucleotide may comprise or consist of a nucleotide sequence complementary to at least one of these portions or to at least one segment within these portions.

Preferred targeting region comprise or consist of a nucleotide sequence complementary to at least one continuous portion selected form the group consisting of nucleotides 1 to 44, nucleotides 10 to 44, nucleotides 17 to 44, nucleotides 17 to 50, nucleotides 45 to 85, each of the sequence depicted in SEQ ID NO: 40, and any variants of these portions.

According to one embodiment of the invention, the nucleotide sequence of the targeting region may comprise at least two segments, each segment complementary to a nucleotide sequence of the 5'-UTR, wherein the complementary nucleotide sequences of the 5'-UTR are not continuous. Within the nucleotide sequence of these targeting regions complementary to a discontinuous portion of the 5'-UTR one or more nucleotides, between two consecutive segments can be comprised.

A polynucleotide of the invention can comprise a targeting region comprising or consisting of a nucleotide sequence depicted in SEQ ID NO: 41, which sequence is complementary to nucleotides 17 to 44 of the 5'-UTR sequence as depicted in SEQ ID NO. 40. Alternatively, a polynucleotide of the invention can comprise a targeting region comprising or consisting of a nucleotide sequence having at least 70%, at least 75%, at least 80%, at least 85%, at least 90%, at least 95%, at least 97%, at least 99%, or 100% identity to the nucleotide sequence depicted in SEQ ID NO: 41.

**Table 23: Nucleotide sequence for targeting nucleotides 17 to 44 of the 5'-UTR:**

| **SEQ ID NO:** | **5'elongation** | **AS-HBB 17-44 (SEQ ID NO: 41)** | **3'elongation** |
|---|---|---|---|
| 41 | | TGTTTGAGGTTGCTAGTGAACACAGTTG | |

For increasing specificity and/or efficiency the nucleotide sequence of the targeting region of said polynucleotide can be further elongated by one or more additional nucleotides. The nucleotide sequence depicted in SEQ ID NO: 41 can be elongated in one or both directions, *i.e.,* 3' and/or 5' relative to the said sequence.

Thus, the nucleotide sequence as set forth in SEQ ID NO: 41 can be additionally flanked 3' in a contiguous manner by one or more nucleotides for targeting nucleotide 16 up to at most nucleotide 1, preferably up to at most nucleotide 10 of the sequence depicted in SEQ ID NO: 40, respectively, or the respective nucleotides in variant sequences. The first nucleotide added 3' to the sequence depicted in SEQ ID NO: 41 is for targeting the nucleotide at position 16 of the sequence depicted in SEQ ID NO: 40. The second nucleotide added to the elongated sequence (*i.e.,* SEQ ID NO: 41 plus 1 nucleotide) is for targeting the nucleotide at position 15 of the sequence depicted in SEQ ID NO: 40, the third nucleotide added to the elongated sequence (*i.e.,* SEQ ID NO: 41 plus 2 nucleotides) is for targeting the nucleotide at position 14 of the sequence depicted in SEQ ID NO: 40, the forth nucleotide added to the elongated sequence (*i.e.,* SEQ ID NO: 41 plus 3 nucleotide) is for targeting the nucleotide at position 13 of the sequence depicted in SEQ ID NO: 40, and so forth. Thus, the nucleotide sequence as set forth in SEQ ID NO: 41 can be 3' elongated in a contiguous manner by 1, 2, 3, 4, 5, 6, 7, 8, 9, 10, 11, 12, 13, 14, 15, or 16 nucleotides. Each nucleotide added 3' can be complementary or not to its respective nucleotide in the sequence depicted in SEQ ID NO: 40.

Preferred nucleotide sequences of targeting regions which are elongated in 3' direction comprise any of the sequences as shown in Table 24, or any variants thereof:

**Table 24:**

| **SEQ ID NO:** | **5'elongation** | **AS-HBB 17-44 (SEQ ID NO: 41)** | **3'elongation** |
|---|---|---|---|
| 42 | | TGTTTGAGGTTGCTAGTGAACACAGTTG | TGTCAGA |
| 43 | | TGTTTGAGGTTGCTAGTGAACACAGTTG | TGTCAGAAGCAAATGT |

These preferred 3' elongations are for targeting the portion comprising nucleotides 10 to 44 (SEQ ID NO: 42) or nucleotides 1 to 44 (SEQ ID NO: 43) of the 5'-UTR of HBB. Also encompassed by the present invention are variants of the sequences depicted in SEQ ID NO: 42 or 43.

Other preferred nucleotide sequences of the targeting region which are elongated in 3' direction comprise the sequences for targeting nucleotides 2 to 44, nucleotides 3 to 44, nucleotides 4 to 44, nucleotides 5 to 44, nucleotides 6 to 44, nucleotides 7 to 44, nucleotides 8 to 44, nucleotides 9 to 44, nucleotides 11 to 44, nucleotides 12 to 44, nucleotides 13 to 44, nucleotides 14 to 44, nucleotides 15 to 44, nucleotides 16 to 44, nucleotides 17 to 44, nucleotides 18 to 44, nucleotides 19 to 44, each of the sequence depicted in SEQ ID NO: 40, and any variants of these portions.

Alternatively, or in addition to each possible elongation at 3', the nucleotide sequence depicted in SEQ ID NO: 41 can be flanked 5' in a contiguous manner by one or more nucleotides for targeting nucleotide 45 up to at most nucleotide 50, or the respective nucleotides in variant sequences. The first nucleotide added 5' to the sequence as set forth in SEQ ID NO: 41 is for targeting the nucleotide at position 45 of the sequence depicted in SEQ ID NO: 40. The second nucleotide added to the elongated sequence (*i.e.,* SEQ ID NO: 41 plus 1 nucleotide) is for targeting the nucleotide at position 46 of the sequence depicted in SEQ ID NO: 40, the third nucleotide added to the elongated sequence (*i.e.,* SEQ ID NO: 41 plus 2 nucleotides) is for targeting the nucleotide at position 47 of the sequence depicted in SEQ ID NO: 40, the forth nucleotide added to the elongated sequence (*i.e.,* SEQ ID NO: 41 plus 3 nucleotide) is for targeting the nucleotide at position 48 of the sequence depicted in SEQ ID NO: 40, and so forth. Thus, the nucleotide sequence as set forth in SEQ ID NO: 41 can be 5' elongated in a contiguous manner by 1, 2, 3, 4, 5, or 6 nucleotides. Each nucleotide added 5' can be complementary or not to its respective nucleotide in the sequence depicted in SEQ ID NO: 40.

A preferred nucleotide sequence of the targeting region which is elongated in 5' direction comprise the sequence as shown in Table 25, or any variants thereof:

**Table 25:**

| **SEQ ID NO:** | **5'elongation** | **AS-HBB 17-44 (SEQ ID NO: 41)** | **3'elongation** |
|---|---|---|---|
| 44 | GGTGTC | TGTTTGAGGTTGCTAGTGAACACAGTTG | |

The preferred 5' elongation is for targeting the portion comprising nucleotides 17 to 50 (SEQ ID NO: 44) of the 5' UTR of HBB. Also encompassed by the present invention are variants of the sequences depicted in SEQ ID NO: 44.

Other preferred nucleotide sequences of the targeting region which are elongated in 5' direction comprise the sequences for targeting nucleotides 17 to 45, nucleotides 17 to 46, nucleotides 17 to 47, nucleotides 17 to 48, nucleotides 17 to 49, each of the sequence depicted in SEQ ID NO: 40, and any variants of these portions.

The polynucleotides of the present invention can also comprise a targeting region comprising or consisting of a nucleotide sequence which is the result of any combination between above mentioned 5' elongations and 3' elongations, including variants of these combined nucleotide sequences.

Preferred targeting regions comprise or consist of a nucleotide sequence depicted in any of SEQ ID NOs: 45 to 47 and/or Table 26, or any variants thereof.

**Table 26:**

| **SEQ ID NO:** | **5'elongation** | **AS-HBB 17-44 (SEQ ID NO: 41)** | **3'elongation** |
|---|---|---|---|
| 45 | | TGTTTGAGGTTGCTAGTGAACACAGTTG | TGTCAGAAGCAAAT |
| 46 | | TGTTTGAGGTTGCTAGTGAACACAGTTG | TGT |
| 47 | GTC | TGTTTGAGGTTGCTAGTGAACACAGTTG | |

In one embodiment, the targeting region is complementary to an AUG-proximal region of the 5'-UTR of HBB, *e.g.,* a 5'-UTR having a sequence as depicted in SEQ ID NO: 40 of the sequence listing.

The AUG-proximal region of the 5'-UTR can be complementary to at least the nucleotides covering the start codon AUG of the main ORF, wherein the A nucleotide of the AUG start codon is referred to as the nucleotide at position +1, at least nucleotides -3 to -1 of the 5'-UTR nucleotide sequence as depicted in SEQ ID NO: 40, and at least nucleotides +4 to +9 of the main ORF nucleotide sequence. The nucleotides -3 to -1 of the 5'-UTR nucleotide sequence are nucleotides 48 to 50 of the 5'-UTR nucleotide sequence as depicted in SEQ ID NO: 40. In one embodiment, the AUG-proximal region of the 5'-UTR complementary to at least the nucleotides -3 to +9 is flanked on one or both ends by a flanking region, wherein each flanking region comprises one or more additional nucleotides, and each additional nucleotide is complementary or non-complementary to the corresponding nucleotide of the nucleotides flanking the portion of the AUG-proximal region of the 5'-UTR to which the nucleotide sequence of the targeting region is complementary.

The AUG-proximal region of the 5'-UTR complementary to at least the nucleotides -3 to +9 can be additionally flanked 3' in a contiguous manner by one or more nucleotides for targeting nucleotide 47 up to at most nucleotide 35, preferably up to at most nucleotide 45 of the sequence depicted in SEQ ID NO: 40, respectively, or the respective nucleotides in variant sequences.

Alternatively, or in addition the AUG-proximal region of the 5'-UTR complementary to at least the nucleotides -3 to +9 can be additionally flanked 5' in a contiguous manner by one or more nucleotides for targeting nucleotide +10 up to at most nucleotide +50, preferably up to at most nucleotide +40 of the ORF, or the respective nucleotides in variant sequences.

The polynucleotides of the present invention can also comprise a targeting region comprising or consisting of a nucleotide sequence which is the result of any combination between above mentioned 5' elongations and 3' elongations, including variants of these combined nucleotide sequences.

As it relates to the polynucleotides of the present invention for targeting a 5'-UTR or a 5'-UTR portion, in one embodiment the nucleotide sequence of the targeting region, preferably the targeting region as specified above, is preferably at least 20, at least 25, or at least 30 nucleotides in length. In one embodiment, the nucleotide sequence of the targeting region, preferably the targeting region as specified above, is at most 60 nucleotides, at most 55 nucleotides, or at most 50 nucleotides in length. For example, the nucleotide sequence of the targeting region may be 20 to 60 or may be 25 to 50 nucleotides in length. As will be easily understood by a person skilled in the art any of the lower limits disclosed can be combined with any of the upper limits mentioned.

In one embodiment, the polynucleotide can in addition to (i) the targeting region, comprise (ii) a region comprising or consisting of a nucleotide sequence capable of forming a stem-loop, which region is linked to the targeting region of (i), and (iii) two linker nucleotide sequences flanking the nucleotide sequence of the targeting region at both sides, *i.e.,* 5' and 3'. That is, when the polynucleotide is in a circularized form, the targeting region is on both sides separated from region capable of forming a stem-loop of (ii) by the one linker sequence. The linkers allow a more flexible presentation of the antisense sequence.

The nucleotide sequence of the targeting region may be at least 15 nucleotides, at least 20 nucleotides, or at least 25 nucleotides in length, and/or the nucleotide sequence of the targeting region may be at most 300 nucleotides, at most 265 nucleotides, at most 250 nucleotides, or at most 150 nucleotides in length. For example, the nucleotide sequence of the targeting region may be 15 to 300 nucleotides or 20 to 250 nucleotides or 15 to 265 nucleotides in length. As will be easily understood by a person skilled in the art any of the lower limits disclosed can be combined with any of the upper limits mentioned.

The region capable of forming a stem-loop may comprise at least 2, 3, 4, 5 or 6, and/or at most 15, 14, 13, 12, 11, 10, 9 or 8 perfect base-pairs with two overhanging ends. For example, the region capable of forming a stem-loop may comprise 4 to 10 or 5 to 8 perfect base-pairs. As will be easily understood by a person skilled in the art any of the lower limits disclosed can be combined with any of the upper limits mentioned. The overhanging ends may be each at least 2, 3, 4, 5, 6, 7, 8, 9 or 10 nucleotides in length, and/or the overhanging ends may be each at most 40, 35, 30, 20 or 15 nucleotides in length. For example, the overhanging ends may be each 2 to 30 or 3 to 20 nucleotides in length. As will be easily understood by a person skilled in the art any of the lower limits disclosed can be combined with any of the upper limits mentioned.

Each linker is preferably at least 1, 2 or 3 nucleotides in length, and/or is preferably at most 20, 15, 12, 10 or 8 nucleotides in length. For example, each linker may be 1 to 20 or 2 to 10 nucleotides in length. As will be easily understood by a person skilled in the art any of the lower limits disclosed can be combined with any of the upper limits mentioned.

Preferred embodiments of polynucleotides, *e.g.,* RNAs, comprising (i) a targeting region, (ii) a backbone region or a region comprising or consisting of a nucleotide sequence capable of forming a stem-loop, and (iii) two linker nucleotide sequences are represented by the sequences depicted in SEQ ID NO: 48 to 53, 55 to 58, 61 to 70, or any variants thereof. These polynucleotides, *e.g.,* RNAs, are circularizable. In one embodiment, the polynucleotides, *e.g.,* RNAs, comprise or consist of any one of the nucleotide sequences as depicted in SEQ ID NO: 48 to 53, 55 to 58, 61 to 70, or any variants thereof are in circular form.

A polynucleotide of the invention can in one embodiment comprise at least one ribonucleotide and/or at least one deoxyribonucleotide. In one embodiment, a polynucleotide of the invention consists of ribonucleotides, deoxyribonucleotides or a combination thereof, with the 5' end of one nucleotide and the 3' end of another nucleotide being linked to one another by a phosphodiester bond. These polynucleotides may be synthesized in the conventional manner or produced recombinantly. According to the present invention, the polynucleotide may be or preferably is circular, which means that the polynucleotide, *e.g.,* the RNA has been circularized.

In the context of the present invention, the term "RNA" relates to a molecule which comprises ribonucleotide residues and preferably being entirely or substantially composed of ribonucleotide residues. "Ribonucleotide" relates to a nucleotide with a hydroxyl group at the 2'-position of a β-D-ribofuranosyl group. The term includes double stranded RNA, single stranded RNA, isolated RNA such as partially or completely purified RNA, essentially pure RNA, synthetic RNA, recombinantly produced RNA, as well as modified RNA that differs from naturally occurring RNA by the addition, deletion, substitution and/or alteration of one or more nucleotides. Such alterations can include addition of non-nucleotide material, such as internally, for example, at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs or analogs of naturallyoccurring RNA.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring. The term "found in nature" means "present in nature" and includes known objects as well as objects that have not yet been discovered and/or isolated from nature, but that may be discovered and/or isolated in the future from a natural source.

The term "recombinant" in the context of the present invention means "made through genetic engineering". Preferably, a "recombinant object" in the context of the present invention is not occurring naturally.

According to the present invention, the term "RNA" includes and preferably relates to "circular RNA" or "circRNA" which means a RNA molecule that has been circularized, for example such that it does not have a 5' and 3' end, *e.g.,* one that results from the ligation of the 5' end to the 3' end or one that results from the action of one or more ribozymes. Preferably, the RNA is produced by *in vitro* transcription using a DNA template and then the ends are ligated together. In one embodiment of the invention, the RNA is obtained by *in vitro* transcription or chemical synthesis. The *in vitro* transcription and ligation methodologies are known to the skilled person. For example, there is a variety of commercially available *in vitro* transcription kits as well as ligation kits.

The RNA according to the invention may be unmodified and/or comprise naturally occurring nucleobases only, *i.e.,* the naturally occurring heterocyclic nucleobases of RNA or DNA: the purine bases adenine (A) and guanine (G), and the pyrimidine bases thymine (T), cytosine (C) (including 5-methyl C), and uracil (U). Alternatively, the RNA according to the invention may contain a modification. As used herein, " modification" means a chemical difference in a compound when compared to a naturally occurring counterpart. In reference to a polynucleotide, chemical modification does not include differences only in nucleobase sequence, *i.e.,* a base modification, but for example, also comprise a backbone modification or a sugar modification. That is, the polynucleotide may be modified in very different ways, without impairing its ability to bind its target, in order to increase, for example, its stability or therapeutic efficacy.

In one embodiment, the RNA has modified ribonucleotides, which can increase its stability and/or decrease cytotoxicity or immunogenicity. For example, in one embodiment, in the RNA used according to the invention, 5-methylcytidine is substituted partially or completely, preferably completely, for cytidine. Alternatively, or additionally, in one embodiment, in the RNA used according to the invention pseudouridine is substituted partially or completely, preferably completely, for uridine.

Other modified ribonucleotides include 1-methyl-adenine, 2-methyladenine, 2-methylthio-N6-isopentenyladenine, N6-methyladenine, N6-isopentenyladenine, 2-thio-cytosine, 3-methylcytosine, 4-acetylcytosine, 5-methylcytosine, 2,6-diaminopurine, 1-methylguanine, 2-methylguanine, 2,2-dimethylguanine, 7-methylguanine, 7-deaza-guanosine, inosine, 1-methylinosine, pseudouracil (5-uracil), dihydrouracil, 2-thiouracil, 4-thiouracil, 5-carboxymethylaminomethyl-2-thiouracil, 5-(carboxyhydroxymethyl)-uracil, 5-fluorouracil, 5-bromouracil, 5-carboxymethylaminomethyluracil, 5- methyl-2-thiouracil, 5-methyluracil, N-uracil-5-oxyacetic acid methyl ester, 5-methylamino-methyluracil, 5-methoxyaminomethyl-2-thiouracil, 5'-methoxycarbonylmethyluracil, 5-methoxyuracil, uracil-5-oxyacetic acid methyl ester, uracil-5-oxyacetic acid, 1-methylpseudouracil, queosine, and β-D-mannosylqueosine.

Backbone modifications typically include, without being limited thereto, modifications from the group consisting of methylphosphonates, methylphosphoramidates, phosphoramidates, phosphorothioates, and boranophosphates.

A sugar modification in connection with the circular RNA described herein is a chemical modification of the sugar of the nucleotides it contains and typically includes, without being limited thereto, sugar modifications chosen from the group consisting of 2'-deoxy-2'-fluorooligoribonucleotide (2'-fluoro-2'-deoxycytidine 5'-triphosphate, 2'-fluoro-2'-deoxyuridine 5'-triphosphate), 2'-deoxy-2'-deamine-oligoribonucleotide (2'-amino-2'-deoxycytidine 5'-triphosphate, 2'-amino-2'-deoxyuridine 5'-triphosphate), 2'-O-alkyl-oligoribonucleotide, 2'-deoxy-2'-C-alkyloligoribonucleotide (2'-O-methylcytidine 5'-triphosphate, 2'-methyluridine 5'-triphosphate), 2'-C-alkyloligoribonucleotide, and isomers thereof (2'-aracytidine 5'-triphosphate, 2'-arauridine 5'-triphosphate), or azidotriphosphates (2'-azido-2'-deoxycytidine 5'-triphosphate, 2'-azido-2'-deoxyuridine 5'-triphosphate).

The term "stability" of an agent, *e.g.,* circular RNA, to the "half-life" of the agent. "Half-life" relates to the period of time which is needed to eliminate half of the maximum (pharmacologic) activity, amount, or number of molecules of an agent, *e.g.,* from the body or blood of a patient to which the agent was administered or as measured in an *in vitro* assay. The maximum pharmacologic activity is defined by the steady state value, where intake equals elimination. Since the kinetics of certain agents, such as pharmaceutical drugs, can be complex, the "half-life" of an agent does not necessarily follow or is limited to first order kinetics.

The nucleic acid, *e.g.,* the RNA, to be administered according to the invention is preferably non-immunogenic. The term "non-immunogenic nucleic acid" or "non-immunogenic RNA" as used herein refers to a nucleic acid or RNA, respectively, that does not induce a response by the immune system upon administration, *e.g.,* to a mammal, or induces a weaker response than would have been induced by the same nucleic acid, *e.g.,* the same RNA, that differs only in that it has not been subjected to the modifications and treatments that render the immunogenic nucleic acid, *e.g.,* the RNA, non-immunogenic. In one preferred embodiment a non-immunogenic nucleic acid or a non-immunogenic RNA is rendered non-immunogenic by incorporating modified nucleotides suppressing nucleic acid or RNA-mediated activation of innate immune receptors into the nucleic acid or the RNA or by having a sequence that does not form double-stranded structures or by removing any double-stranded nucleic acids or double-stranded RNA (dsRNA) present with the circular RNA.

For rendering the non-immunogenic nucleic acid, *e.g.,* the RNA, non-immunogenic by the incorporation of modified nucleotides, any modified nucleotide may be used as long as it lowers or suppresses immunogenicity of the nucleic acid, *e.g.,* the RNA. Particularly preferred are modified nucleotides that suppress a nucleic acid-mediated activation of innate immune receptors. In one embodiment, the modified nucleotides comprise a replacement of one or more uridines with a nucleoside comprising a modified nucleobase. In one embodiment, the modified nucleobase is a modified uracil. In one embodiment, the nucleoside comprising a modified nucleobase is selected from the group consisting of 3-methyl-uridine (m3U), 5-methoxy-uridine (mo5U), 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho5U), 5-aminoallyluridine, 5-halo-uridine (*e.g.*, 5-iodo-uridineor 5-bromo-uridine), uridine 5-oxyacetic acid (cmo5U), uridine 5-oxyacetic acid methyl ester (mcmo5U), 5-carboxymethyl-uridine (cm5U), 1-carboxymethyl-pseudouridine, 5-carboxyhydroxymethyl-uridine (chm5U), 5-carboxyhydroxy-methyluridine methyl ester (mchm5U), 5-methoxycarbonylmethyl-uridine (mcm5U), 5-methoxycarbonylmethyl-2-thio-uridine (mcm5s2U), 5-aminomethyl-2-thio-uridine (nm5s2U), 5-methylaminomethyl-uridine (mnm5U), 1-ethyl-pseudouridine, 5-methyl-aminomethyl-2-thio-uridine (mnm5s2U), 5-methylaminomethyl-2-seleno-uridine (mnm5se2U), 5-carbamoylmethyluridine (ncm5U), 5-carboxymethylaminomethyl-uridine (cmnm5U), 5-carboxymethylaminomethyl-2-thio-uridine (cmnm5s2U), 5-propynyluridine, 1-propynylpseudouridine, 5-taurinomethyl-uridine (τm5U), 1-taurino-methyl-pseudouridine, 5-taurinomethyl-2-thio-uridine (τm5s2U), 1-taurinomethyl-4-thio-pseudouridine, 5-methyl-2-thio-uridine (m5s2U), 1-methyl-4-thio-pseudouridine (m1s4ψ), 4-thio-1-methyl-pseudouridine, 3-methyl-pseudouridine (m3ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m5D), 2-thio-dihydrouridine, 2-thiodihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp3U), 1-methyl-3-(3-amino-3-carboxypropyl)pseudouridine (acp3 ψ), 5-(isopentenylaminomethyl)uridine (inm5U), 5-(isopentenylaminomethyl)-2-thio-uridine (inm5s2U), α-thio-uridine, 2'-O-methyl-uridine (Um), 5,2'-O-dimethyl-uridine (m5Um), 2'-O-methyl-pseudouridine (ψm), 2-thio-2'-O-methyl-uridine (s2Um), 5-methoxycarbonylmethyl-2'-O-methyl-uridine (mcm5Um), 5-carbamoylmethyl-2'-O-methyl-uridine (ncm5Um), 5-carboxymethylaminomethyl-2'-O-methyluridine (cmnm5Um), 3,2'-O-dimethyl-uridine (m3Um), 5-(isopentenylaminomethyl)-2'-O-methyluridine (inm5Um), 1-thio-uridine, deoxythymidine, 2'-F-ara-uridine, 2'-F-uridine, 2'-OH-arauridine, 5-(2-carbomethoxy-vinyl) uridine, and 5-[3-(1-E-propenylamino)uridine. In one embodiment, the nucleoside comprising a modified nucleobase is pseudouridine (ψ), N1-methyl-pseudouridine (m1ψ), 5-methyl-uridine (m5U), or 1-methyl-pseudouridine.

The structure of an exemplary nucleoside comprising a modified nucleobase is 1-methylpseudouridine m1ψ:

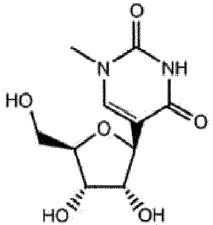

In one embodiment, the replacement of one or more uridines with a nucleoside comprising a modified nucleobase comprises a replacement of at least 1%, at least 2%, at least 3%, at least 4%, at least 5%, at least 10%, at least 25%, at least 50%, at least 75%, at least 90%, at least 95%, at least 96%, at least 97%, at least 98%, at least 99% or 100% of the uridines.

During synthesis of mRNA by *in vitro* transcription (IVT) using T7 RNA polymerase aberrant products, including double-stranded RNA (dsRNA) are produced due to unconventional activity of the enzyme. dsRNA induces inflammatory cytokines and activates effector enzymes leading to protein synthesis inhibition. dsRNA can be removed from RNA such as IVT RNA, by methods known to the skilled person, for example, by ion-pair reversed phase HPLC using a non-porous or porous C-18 polystyrene-divinylbenzene (PS-DVB) matrix.

According to the invention, "double-stranded RNA" or "dsRNA" means RNA with two partially or completely complementary strands. Although the circRNA described herein preferably is a circularized "single-stranded" RNA molecule, it may nonetheless contain self-complementary sequences that allow parts of the RNA to fold back and to form secondary structure motifs including without limitation base pairs, stems, stem loops and bulges.

As the term is used herein, "remove" or "removal" refers to the characteristic of a population of first substances, such as non-immunogenic RNA, being separated from the proximity of a population of second substances, such as dsRNA, wherein the population of first substances is not necessarily devoid of the second substance, and the population of second substances is not necessarily devoid of the first substance. However, a population of first substances characterized by the removal of a population of second substances has a measurably lower content of second substances as compared to the non-separated mixture of first and second substances. The polynucleotides of the invention, *e.g.,* the circular RNAs, and compositions described herein are useful in methods of treating or preventing a disease, for example for treating or preventing proliferative disease, such as cancer.

The polynucleotides and compositions described herein are also useful in methods for shaping the cellular proteome. The polynucleotides and compositions described herein can be involved in pre-mRNA splicing and the polynucleotides or compositions can be capable of at least one of inhibiting pre-mRNA splicing, resulting in intron retention, resulting in alternative 5' splice site use, and/or resulting in single-exon skipping. Alternatively, the polynucleotides and compositions described herein can be capable of inhibiting mRNA translation. According to the invention preferably circular DNA or circular RNA, more preferably non-immunogenic circular DNA or circular RNA, is administered to the patient/subject.

The expression "for use in treating or preventing a disease, for example for treating or preventing a proliferative disorder" is used herein also replaceable with "for use as a medicament, especially in a method of treating or preventing a proliferative disorder"; or the use of said products in the preparation of a pharmaceutical formulation for use in said method of treatment in humans (or more generically a subject in need thereof).

The term "disease" refers to an abnormal condition that affects the body of an individual. A disease is often construed as a medical condition associated with specific symptoms and signs. A disease may be caused by factors originally from an external source, such as infectious disease, or it may be caused by internal dysfunctions, such as cancer. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behaviors, and atypical variations of structure and function, while in other contexts and for other purposes these may be considered distinguishable categories.

Diseases usually affect individuals not only physically, but also emotionally, as contracting and living with many diseases can alter one's perspective on life, and one's personality. According to the invention, the term "disease" preferably includes a proliferative disease, such as cancer. According to the invention, a "proliferative disease" includes a disease characterized by aberrantly regulated cellular growth, proliferation, differentiation, adhesion, and/or migration. Particular examples of proliferative diseases are cancer and psoriasis.

The term "treatment" or "therapeutic treatment" relates to any treatment which improves the health status and/or prolongs (increases) the lifespan of an individual. Said treatment may eliminate the disease in an individual, arrest or slow the development of a disease in an individual, inhibit or slow the development of a disease in an individual, decrease the frequency or severity of symptoms in an individual, and/or decrease the recurrence in an individual who currently has or who previously has had a disease.

The terms "prophylactic treatment" or "preventive treatment" relate to any treatment that is intended to prevent a disease from occurring in an individual, in particular an individual being at risk for the disease. The terms "prophylactic treatment" or "preventive treatment" are used herein interchangeably.

The terms "protect", "prevent", "prophylactic", "preventive", or "protective" relate to the prevention and/or treatment of the occurrence and/or the propagation of a disease, *e.g.,* a proliferative disease, in an individual. For example, a prophylactic administration of a circular RNA, *e.g.,* by administering a composition of the present invention, can protect the receiving individual from the development of serve symptoms. For example, by administering a composition of the present invention, the development of a disease can be stopped, *e.g.,* leading to inhibition of the progress of the disease. This comprises the deceleration of the progress of the disease, in particular a disruption of the progression of the disease.

By "being at risk" is meant a subject, *i.e.,* a patient, that is identified as having a higher than normal chance of developing a disease compared to the general population.

The term *"in vivo"* relates to the situation in a subject.

The term "individual" or "subject" or "patient" relates to vertebrates, particularly mammals. For example, mammals in the context of the present invention are humans, non-human primates, domesticated mammals such as dogs, cats, sheep, cattle, goats, pigs, horses *etc.,* laboratory animals such as mice, rats, rabbits, guinea pigs, *etc.* as well as animals in captivity such as animals of zoos. The term "subject" also relates to non-mammalian vertebrates such as birds (particularly domesticated birds such as chicken, ducks, geese, turkeys) and to fish (particularly farmed fish, *e.g.,* salmon or catfish). The term "animal" as used herein also includes humans.

The agents, *e.g.,* the circular RNA molecules described herein, may be administered in the form of any suitable pharmaceutical composition. The term "pharmaceutical composition" relates to a formulation comprising a therapeutically effective agent or a salt thereof, preferably together with pharmaceutical excipients such as buffers, preservatives and tonicity modifiers. Said pharmaceutical composition is useful for treating or preventing a disease or disorder by administration of said pharmaceutical composition to an individual. A pharmaceutical composition is also known in the art as a pharmaceutical formulation. The pharmaceutical composition can be administered locally or systemically, alone or in combination with any other agent.

The term "systemic administration" refers to the administration of a therapeutically effective agent such that the agent becomes widely distributed in the body of an individual in significant amounts and develops a biological effect. According to the present invention, it is preferred that administration is by parenteral administration, wherein the polynucleotide or the pharmaceutical composition of the invention can be administered alone or in combination with any other agent.

In one embodiment, the subject being administered the polynucleotide of the invention or the pharmaceutical composition of the invention is additionally treated with at least one of a chemotherapeutic agent, an immunotherapeutic agent, a cytokine, a hormone, radiation or surgery. The chemotherapeutic agent can be selected from anthracyclines, taxanes, nucleotide analogs, platinum-based agents, and combinations thereof. The immunotherapeutic agent can be an antibody or an antigen-binding fragment thereof targeting a checkpoint inhibitor. The cytokine can be selected from interferon, interleukin, growth factors, and combinations thereof.

The term "parenteral administration" refers to administration of a therapeutically effective agent such that the agent does not pass the intestine. The term "parenteral administration" includes intravenous administration, subcutaneous administration, intradermal administration, inhalation or intraarterial administration, but is not limited thereto.

The pharmaceutical composition according to the present invention is generally applied in a "pharmaceutically effective amount" and in "a pharmaceutically acceptable preparation".

The term "pharmaceutically effective amount" refers to the amount which achieves a desired reaction or a desired effect alone or together with further doses. In the case of the treatment of a particular disease, the desired reaction preferably relates to inhibition of the course of the disease. This comprises slowing down the progress of the disease and, in particular, interrupting or reversing the progress of the disease. The desired reaction in a treatment of a disease may also be delay of the onset or a prevention of the onset of said disease or said condition. An effective amount of the compositions described herein will depend on the condition to be treated, the severity of the disease, the individual parameters of the patient, including age, physiological condition, size and weight, the duration of treatment, the type of an accompanying therapy (if present), the specific route of administration and similar factors. Accordingly, the doses administered of the compositions described herein may depend on several of such parameters. In the case that a reaction in a patient is insufficient with an initial dose, higher doses (or effectively higher doses achieved by a different, more localized route of administration) may be used.

The term "pharmaceutically acceptable" refers to the non-toxicity of a material which does not interact with the action of the active component of the pharmaceutical composition.

The pharmaceutical compositions of the present invention may contain salts, buffers, preserving agents, carriers and optionally other therapeutic agents. Preferably, the pharmaceutical compositions of the present invention comprise one or more pharmaceutically acceptable carriers, diluents and/or excipients.

The term "carrier" refers to an organic or inorganic component, of a natural or synthetic nature, in which the active component is combined in order to facilitate, enhance or enable application. According to the invention, the term "carrier" also includes one or more compatible solid or liquid fillers, diluents or encapsulating substances, which are suitable for administration to a patient. Possible carrier substances include, *e.g.,* sterile water, Ringer, Ringer lactate, sterile sodium chloride solution, polyalkylene glycols, hydrogenated naphthalenes and, in particular, biocompatible lactide polymers, lactide/glycolide copolymers or polyoxyethylene/polyoxypropylene copolymers, oils, including those which are derived from mineral oil, animals, or plants, such as peanut oil, soy bean oil, sesame oil, sunflower oil, *etc.* Salt solutions and aqueous dextrose and glycerin solutions may also be used as aqueous carrier compounds.

The term "excipient" when used herein is intended to indicate all substances which may be present in a pharmaceutical composition and which are not active ingredients such as, *e.g.,* carriers, binders, lubricants, thickeners, surface active agents, preservatives, emulsifiers, buffers, flavoring agents, or colorants.

The term "diluent" relates a diluting and/or thinning agent. Moreover, the term "diluent" includes any one or more of fluid, liquid or solid suspension and/or mixing media.

Pharmaceutically acceptable carriers or diluents for therapeutic use are well known in the pharmaceutical art, and are described, for example, in Remington's Pharmaceutical Sciences, Mack Publishing Co. (A. R Gennaro edit. 1985). Examples of suitable carriers include, for example, magnesium carbonate, magnesium stearate, talc, sugar, lactose, pectin, dextrin, starch, gelatin, tragacanth, methylcellulose, sodium carboxymethyl-cellulose, a low melting wax, cocoa butter, and the like. Examples of suitable diluents include ethanol, glycerol and water.

Pharmaceutical carriers, excipients or diluents can be selected with regard to the intended route of administration and standard pharmaceutical practice. The pharmaceutical compositions of the present invention may comprise as, or in addition to, the carrier(s), excipient(s) or diluent(s) any suitable binder(s), lubricant(s), suspending agent(s), coating agent(s), and/or solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavoring agents may be provided in the pharmaceutical composition. Examples of preservatives include sodium benzoate, benzalkonium chloride, chlorobutanol, paraben, thimersol, sorbic acid and esters of p-hydroxybenzoic acid. Antioxidants and suspending agents may be also used.

In one embodiment, the composition is an aqueous composition. The aqueous composition may optionally comprise solutes, *e.g.,* salts. In one embodiment, the composition is in the form of a freeze-dried composition. A freeze-dried composition is obtainable by freeze-drying a respective aqueous composition.

In one embodiment, the composition is an injectable composition, optionally comprising sodium, potassium and calcium salts, such as Ringer's solution or Ringer's Lactate.

Terms such as "transferring", "introducing", "transfecting" or "transducing" are used interchangeably herein and relate to the introduction of nucleic acids, in particular exogenous or heterologous nucleic acids, such as circular RNA into a cell. According to the present invention, the cell can be present *in vitro* or *in vivo, e.g.,* the cell can form part of an organ, a tissue and/or an organism. According to the invention, transfection can be transient or stable. For some applications of transfection, it is sufficient if the transfected genetic material is only transiently present. Since the nucleic acid introduced in the transfection process is usually not integrated into the nuclear genome, the foreign nucleic acid will be diluted through mitosis or degraded.

In one embodiment, the polynucleotide, *e.g*., the circular RNA molecule, to be administered is naked, *i.e.,* not complexed with any kind of delivery material or proteins. In one embodiment, the polynucleotide, *e.g*., the RNA molecule, to be administered is formulated in a delivery vehicle. The delivery vehicle may be used to transport the polynucleotide, *e.g*., the RNA molecule, into the target organ or tissue by preventing its degradation. In one embodiment, the delivery vehicle comprises particles. In one embodiment, the delivery vehicle comprises a lipid. In one embodiment, the lipid comprises a cationic lipid. In one embodiment, the lipid forms a complex with and/or encapsulates the polynucleotide molecule. In one embodiment, the polynucleotide molecule is formulated in liposomes, preferably liposomes comprising a cationic lipid and optionally another lipid. Liposomes may be formed using standard methods known to the skilled person. Respective methods include the reverse evaporation method, the ethanol injection method, the dehydration-rehydration method, sonication or other suitable methods. Following liposome formation, the liposomes can be sized to obtain a population of liposomes having a substantially homogeneous size range.

The pharmaceutical compositions of the invention are preferably sterile and contain an effective amount of the agents described herein and optionally of further agents as discussed herein to generate the desired reaction or the desired effect.

Pharmaceutical compositions are usually provided in a uniform dosage form and may be prepared in a manner known *per se.* A pharmaceutical composition may, *e.g.,* be in the form of a solution or suspension.

The pharmaceutical composition according to the invention can be buffered, *e.g.,* with an acetate buffer, a citrate buffer, a succinate buffer, a Tris buffer, or a phosphate buffer.

In some embodiments, the polynucleotides, *e.g.,* the circular RNA molecules, of the present invention are provided in complexed or encapsulated form. Respective pharmaceutical compositions are provided in the present invention. In particular, in some embodiments, the pharmaceutical composition of the present invention comprises nucleic acid-containing particles, preferably RNA-containing particles. Respective pharmaceutical compositions are referred to as particulate formulations. In particulate formulations according to the present invention, a particle comprises a polynucleotide according to the invention and a pharmaceutically acceptable carrier or a pharmaceutically acceptable vehicle that is suitable for delivery of the nucleic acid. The nucleic acid-containing particles may be, for example, in the form of proteinaceous particles or in the form of lipid-containing particles. Suitable proteins or lipids are referred to as particle forming agents. Proteinaceous particles and lipid-containing particles have been described previously to be suitable for delivery of RNA in particulate form (*e.g*., Strauss & Strauss, 1994, Microbiol. Rev. 58:491-562).

In one embodiment, the particulate formulation of the present invention is a nanoparticulate formulation. In that embodiment, the composition according to the present invention comprises a polynucleotide according to the invention in the form of nanoparticles. Nanoparticulate formulations can be obtained by various protocols and with various complexing compounds. Lipids, polymers, oligomers, or amphiphiles are typical constituents of nanoparticulate formulations.

As used herein, the term "nanoparticle" refers to any particle having a diameter making the particle suitable for systemic, in particular parenteral, administration, of, in particular, nucleic acids, typically a diameter of 1000 nanometers (nm) or less. In one embodiment, the nanoparticles have an average diameter in the range of from about 50 nm to about 1000 nm, preferably from about 50 nm to about 400 nm, preferably about 100 nm to about 300 nm such as about 150 nm to about 200 nm. In one embodiment, the nanoparticles have a diameter in the range of about 200 to about 700 nm, about 200 to about 600 nm, preferably about 250 to about 550 nm, in particular about 300 to about 500 nm or about 200 to about 400 nm.

In other embodiments, the polynucleotide, *e.g.,* the circular RNA, according to the present invention is present in the form of an emulsion. Emulsions have been previously described to be used for delivery of nucleic acid molecules, such as RNA molecules, to cells. Preferred herein are oil-in-water emulsions. The respective emulsion particles comprise an oil core and a cationic lipid. More preferred are cationic oil-in-water emulsions in which the polynucleotide, *e.g.,* the circular RNA, according to the present invention is complexed to the emulsion particles. The emulsion particles comprise an oil core and a cationic lipid. The cationic lipid can interact with the negatively charged RNA, thereby anchoring the circular RNA to the emulsion particles. In an oil-in-water emulsion, emulsion particles are dispersed in an aqueous continuous phase. For example, the average diameter of the emulsion particles may typically be from about 80 nm to 180 nm. In one embodiment, the pharmaceutical composition of the present invention is a cationic oil-in-water emulsion, wherein the emulsion particles comprise an oil core and a cationic lipid, as described in WO 2012/006380 A2. The polynucleotide, *e.g.,* the RNA, according to the present invention may be present in the form of an emulsion comprising a cationic lipid wherein the N:P ratio of the emulsion is at least 4:1, as described in WO 2013/006834 A1. The polynucleotide, *e.g.,* the RNA according to the present invention may be present in the form of a cationic lipid emulsion, as described in WO 2013/006837 A1. In particular, the composition may comprise RNA complexed with a particle of a cationic oil-in-water emulsion, wherein the ratio of oil/lipid is at least about 8:1 (mole:mole).

As used herein, with respect to polynucleotides or nucleic acid molecules, the term "variant" includes degenerate nucleotide sequences, wherein a degenerate nucleotide sequence according to the invention is a nucleotide sequence that differs from a reference nucleotide sequence in codon sequence due to the degeneracy of the genetic code.

Furthermore, a "variant" of a given nucleotide sequence according to the invention includes nucleotide sequences comprising single or multiple such as at least 2, at least 4, or at least 6 and preferably up to 3, up to 4, up to 5, up to 6, up to 10, up to 15, or up to 20 nucleotide substitutions, deletions and/or additions.

Preferably the degree of identity between a given nucleotide sequence and a nucleotide sequence which is a variant of said given nucleotide sequence will be at least 70%, preferably at least 75%, preferably at least 80%, more preferably at least 85%, even more preferably at least 90% or most preferably at least 95%, 96%, 97%, 98% or 99%. The degree of identity is preferably given for a region of at least about 30, at least about 50, at least about 70, at least about 90, at least about 100, at least about 150, at least about 200, at least about 250, at least about 300, or at least about 400 nucleotides. In preferred embodiments, the degree of identity is given for the entire length of the reference nucleotide sequence.

"Sequence identity" between two nucleotide sequences indicates the percentage of nucleotides that are identical between the sequences.

The term "percentage identity" is intended to denote a percentage of nucleotides which are identical between the two sequences to be compared, obtained after the best alignment, this percentage being purely statistical and the differences between the two sequences being distributed randomly and over their entire length. Sequence comparisons between two nucleotide sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Needleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

The present invention is further illustrated by the following figures and examples which are not be construed as limiting the scope of the invention.

### FIGURES

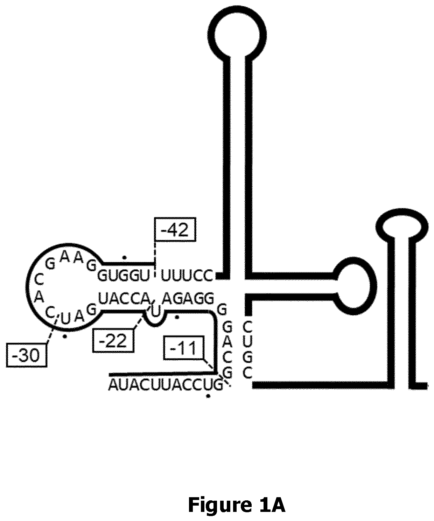

Figure 1A is a secondary structure model of a human U1 snRNA. In the context of the schematic U1 snRNA secondary structure, the target regions in the 5'-terminal region of the U1 snRNA are shown, all covering sequence from the 5'-end of the U1 snRNA to various positions (-11, -22, - 30, and -42). The RNA nucleotide sequence shows in particular the 5'-terminal region of the human U1 snRNA sequence to be targeted.

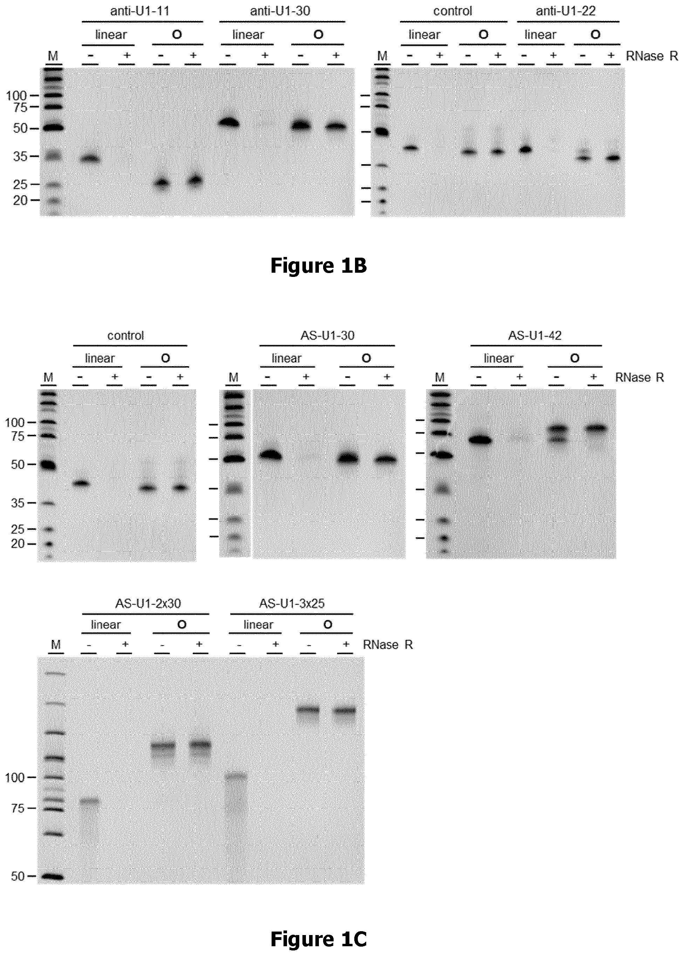

Figure 1B shows the results of antisense RNAs synthesis targeting U1 snRNA. AS-U1-11 (anti-U1-11), AS-U1-22 (anti-U1-22), AS-U1-30 (anti-U1-30), and the control RNA were analyzed by denaturing PAGE and ethidium bromide staining, each in linear and circular configuration, and after mock or RNase R treatment (-/+). *M* indicates markers (sizes in nucleotides).
Figure 1C shows the results of the synthesis of AS-U1-30, AS-U1-42, AS-U1-2x30, AS-U1-3x25 RNAs, or the negative control RNA. All AS-RNAs are based on a common 20-nucleotide stem-loop as backbone and were analyzed after gel purification by denaturing PAGE and ethidium bromide staining, each in linear and circular configuration, and after mock or RNase R treatment (-/+). *M* indicates markers (sizes in nucleotides).

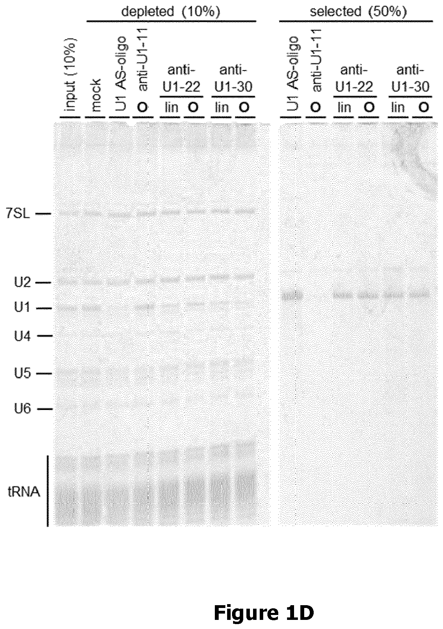

Figure 1D shows the results of U1 snRNA affinity selection from HeLa nuclear extract by biotinylated AS-U1 RNAs. Biotinylated AS-U1 RNAs -11, -22, and -30 (linear and circular (indicated as "∘"), as well as a linear, 3'-biotinylated 13-nucleotide 2'-OMe RNA antisense oligonucleotide (U1 AS-oligo) as a control, were incubated with HeLa nuclear extract, followed by neutravidin agarose selection. Depleted and affinity-selected material, including mock-treated samples and the input material were analyzed by denaturing PAGE and silver staining. Mobilities of the snRNAs, 7SL, and tRNAs are marked on the left.

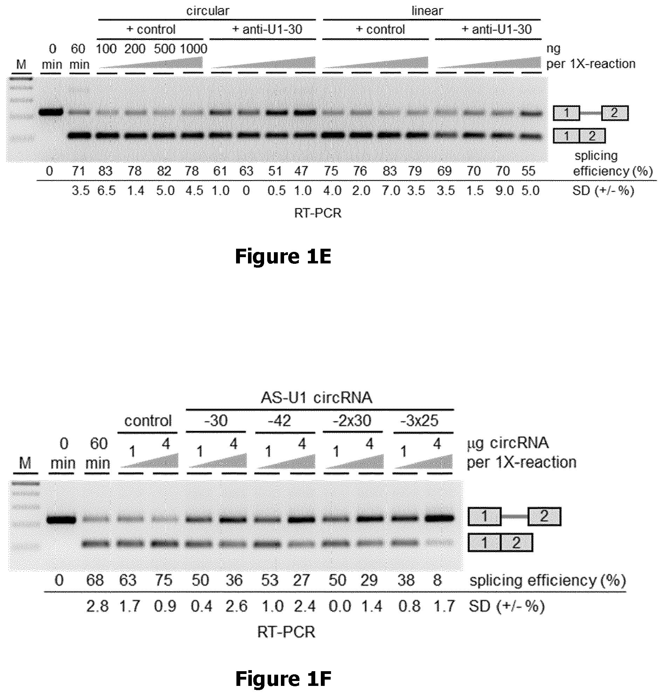

Figure 1E shows the results of *in vitro* splicing inhibition by AS-U1-30 RNA. AS-U1-30 and control RNAs, both in circular and linear configuration, and in concentrations between 100 and 1000 ng per 1X-reaction (25 µl), were pre-incubated in HeLa nuclear extract, followed by the addition of MINX pre-mRNAs and *in vitro* splicing for 60 minutes. Splicing efficiencies were monitored by RT-PCR, with quantitations indicated on the bottom of the figure (in %; with standard deviations, n=3). Vindicates markers.
Figure 1F shows the results of *in vitro* splicing inhibition by extended AS-U1 circRNAs. AS-U1 circRNAs -30, -42, -2x30, -3x25, or the negative control circRNA, were pre-incubated in HeLa nuclear extract, followed by the addition of MINX pre-mRNA and *in vitro* splicing for 60 minutes. One and four µg of each circRNA per 1X-reaction (25 µl) were tested in each case, and untreated mock samples (0 and 60 minutes) were included. *In vitro* splicing activities were assessed by RT-PCR (splicing efficiencies quantitated on the bottom of the figure, in %, with standard deviations, n=2 and technical duplicates). *M* indicates markers.

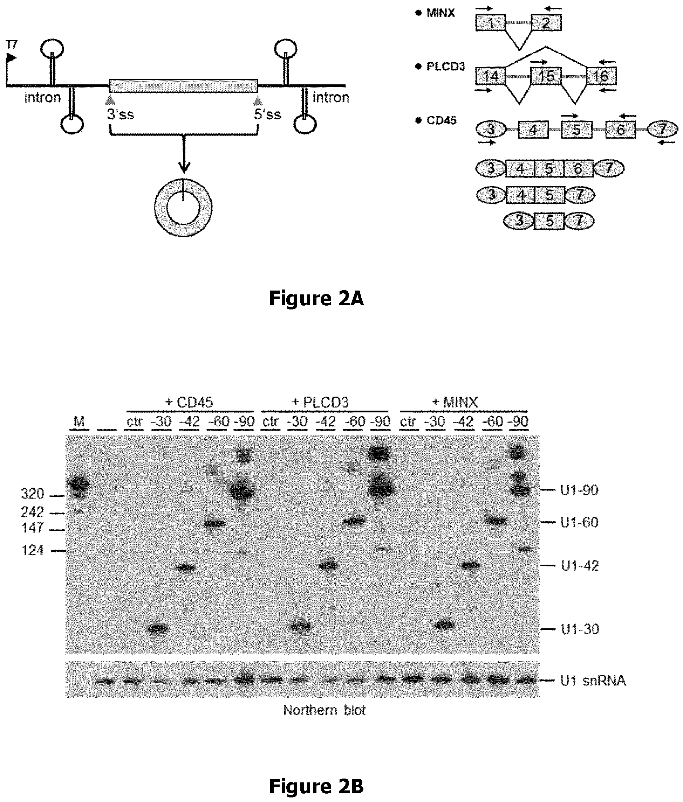

Figure 2A (left side) is a schematic representation of Tornado-based constructs for overexpression of AS-U1 circRNAs (AS-U1-30, AS-U1-42, AS-U1-60, AS-U1-90, control (ctr)). 3'ss indicates the 3' splice site, 5'ss indicates the 5' splice site. The circular junction of the antisense RNA constructs is indicated by a vertical line. To the right, the three splice reporter minigenes (MINX, PLCD3, and CD45) that were cotransfected with the circRNA overexpression constructs are schematically represented, including splicing patterns and positions of primers (indicated as arrows) used for RT-PCR assays.
Figure 2B shows the results of the overexpression of AS-U1 circRNAs by Northern analysis. HeLa cells were cotransfected with Tornado-based circRNA constructs (-30, -42, -60, -90; or a control circRNA) and splice reporter minigenes (MINX, PLCD3, CD45). The AS-U1 RNAs were detected by Northern blot, using U1 snRNA (bottom) as an input control. *M* indicates markers (sizes in nucleotides).

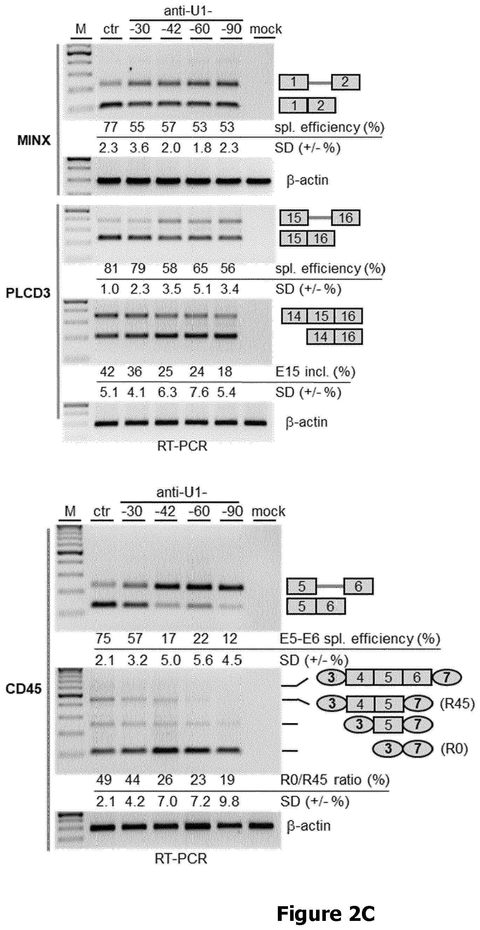

Figure 2C shows the results of splicing inhibition and modulation *in vivo* by AS-U1 circRNAs. HeLa cells cotransfected with circRNA overexpression constructs (-30, -42, -60, -90; or a control circRNA) and splice reporter minigenes (MINX, PLCD3, CD45) were analyzed by RT-PCR for splicing, with β-actin as input control. A mock transfection served as an additional control. Splicing efficiencies were quantitated, as indicated in the figure (in %; with standard deviations, n=3). *M* indicates markers.

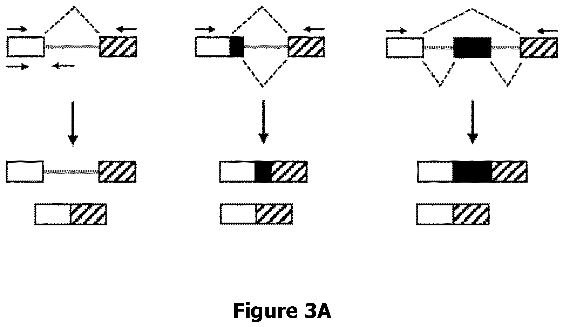

Figure 3A is a schematic representation of alternative splicing effects, namely intron retention (left side), alternative 5' splice site (5'ss) use (in the middle) and exon skipping (right side). In the schematic representation, introns are indicated as lines and exons as rectangles. The arrows indicate positions of sense and antisense primers used for RT-PCR assays.

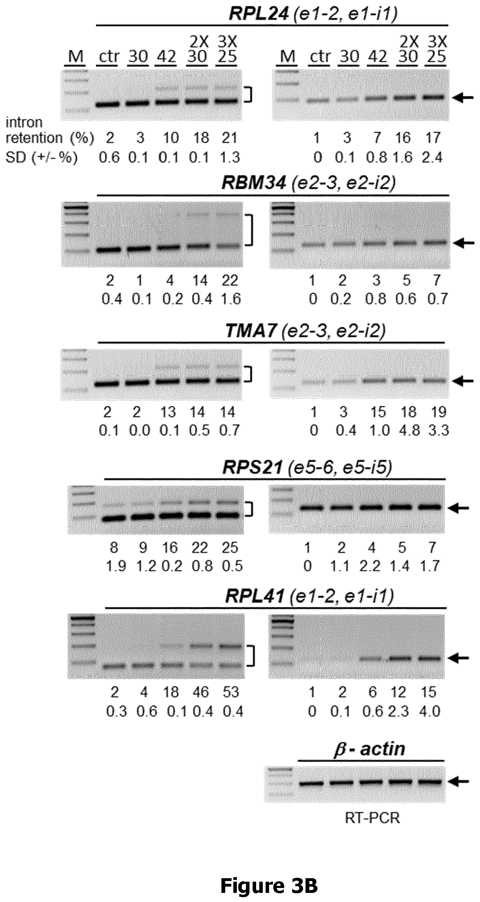

Figure 3B shows the results of RT-PCR validation of intron retention induced by AS-U1 circRNAs. HeLa cells were transfected with AS-U1 circRNAs (AS-U1-30, AS-U1-42, AS-U1-2x30, AS-U1-3x25) or the negative-control circRNA (ctr). After 24 hours, intron retention of endogenous genes was analyzed by RT-PCR and gene-specific primers, for five representative targets (RPL24, RBM34, TMA7, RPS21, RPL41) with both exon-exon and exon-intron primer combinations. Band intensities are quantitated below; for intron retention levels, quantitation is based on qPCR and exon-intron primer combinations and expressed as % intron retention relative to control levels, after normalization to β-actin. *M* indicates markers.

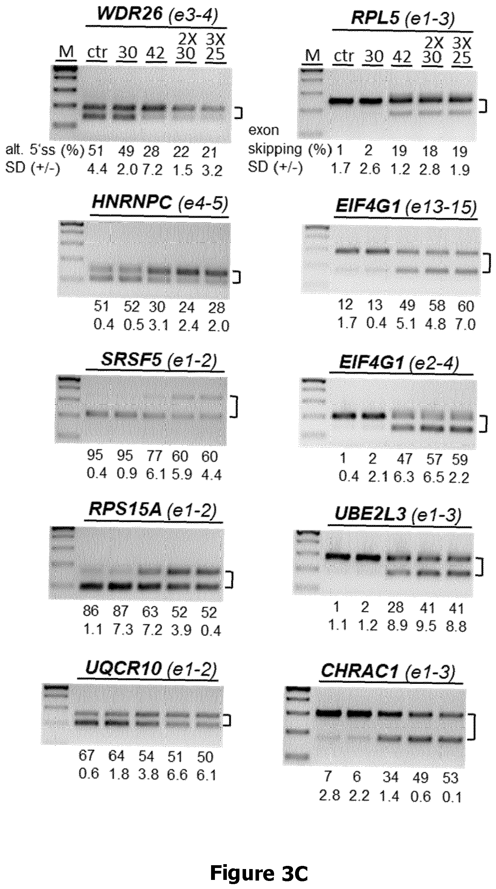

Figure 3C shows the results of RT-PCR validation of alternative 5'ss use (left panel) and exon skipping (right panel) induced by AS-U1 circRNAs. HeLa cells were transfected with AS-U1 circRNAs (AS-U1-30, AS-U1-42, AS-U1-2x30, AS-U1-3x25) or the negative-control circRNA (ctr). After 24 hours, alternative splicing of the endogenous genes as indicated was analyzed by RT-PCR and gene-specific primers, for five representative targets of each type as indicated. Band intensities are quantitated below. *M* indicates markers.

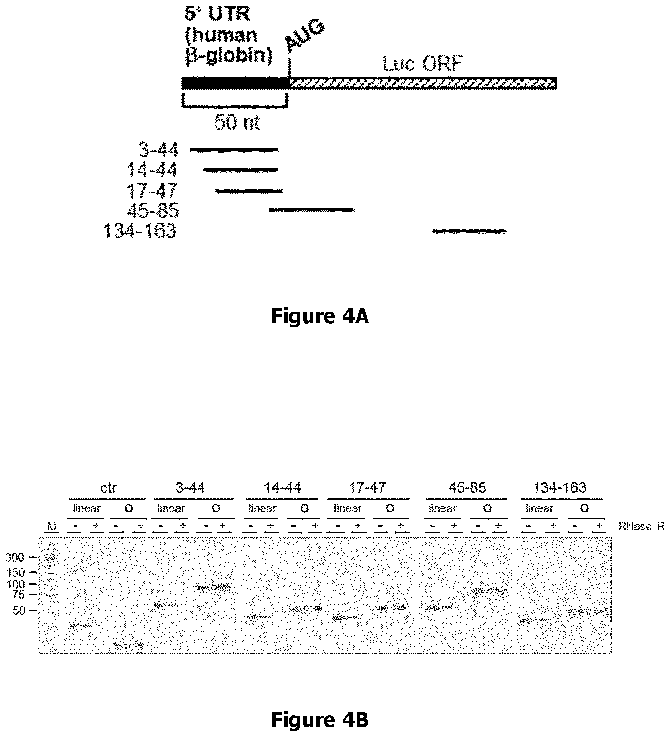

Figure 4A is a schematic representation of a luciferase reporter (luc) construct comprising the β-globin 5'-UTR (nucleotides 1-50), targeted by AS-circRNAs. Target regions of AS-circRNAs are represented as black lines with nucleotide coordinates.
Figure 4B shows the results of antisense RNAs synthesis targeting the 5'-UTR of human β-globin (HBB) mRNA. AS-RNA synthesis was performed by *in vitro* T7 transcription and circularization by T4 RNA ligase. Gel-purified linear and circular RNAs were mock-treated or treated with RNase R (-/+), and analyzed by denaturing PAGE and ethidium bromide staining. Mobilities of circular (o) and linear (-) forms are marked. *M* indicates markers (sizes in nucleotides).

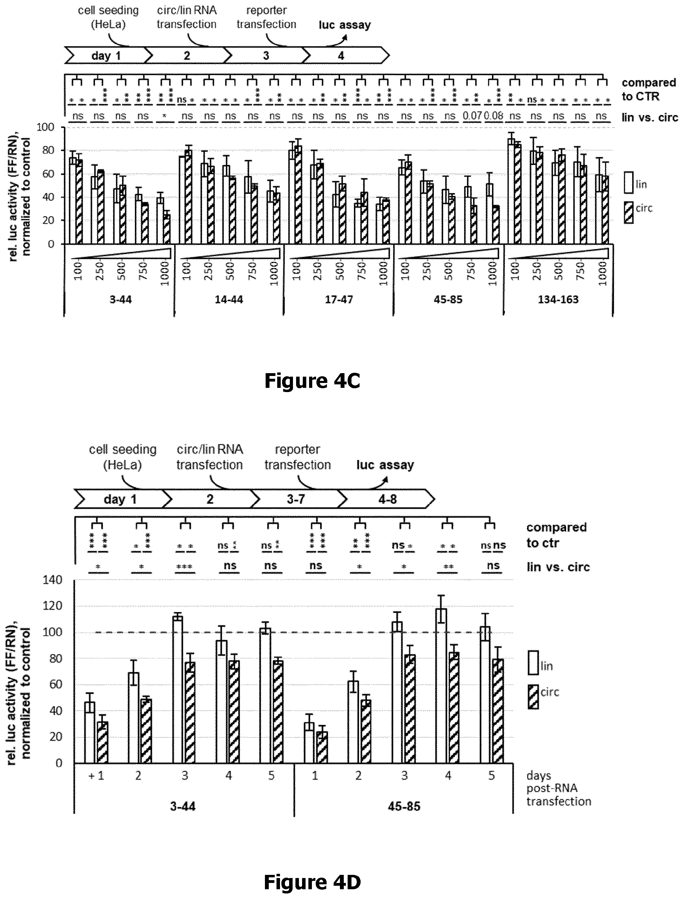

Figure 4C shows the results of translation-inhibitory activities of AS-RNAs (AS-HBB 3-44, AS-HBB 14-44, AS-HBB 14-47, AS-HBB/Luc 45-85) targeting the 5'-UTR of human β-globin (HBB) mRNA. Luciferase reporter assays in HeLa cells transfected with synthetic RNAs (linear or circular) were performed as indicated in the workflow shown at the top of the figure. In particular, HeLa cells were transfected with increasing amounts (100 - 1000 ng per assay) of circRNAs (hatched bars), or their linear counterparts (white bars). After 24 hours, the reporter constructs were transfected, and relative luciferase activities (Firefly/Renilla expression ratios) were measured, normalized to control circRNA (CTR; mean and standard deviations of three replicates, ^{∗}*P<* 0.05, ^{∗∗}*P* < 0.005, ^{∗∗∗}*P* < 0.001, ns = not significant, two-sided *t*-test). For nearly significant samples, the P-value is given instead.
Figure 4D shows durability of the translation-inhibition activities of AS-RNAs (AS-HBB 3-44 and AS-HBB/Luc 45-85). HeLa cells were transfected with the AS-circRNAs (hatched bars) or their linear counterparts (white bars), followed by transfection of the reporter constructs after one to five days. The experimental workflow is indicated at the top of the figure. Relative luciferase activities (Firefly/Renilla expression ratios) were measured, normalized to control circRNA (CTR; mean and standard deviations of three replicates, ^{∗}*P* < 0.05, ^{∗∗}*P* < 0.005, ^{∗∗∗}*P* < 0.001, ns = not significant, two-sided *t*-test).

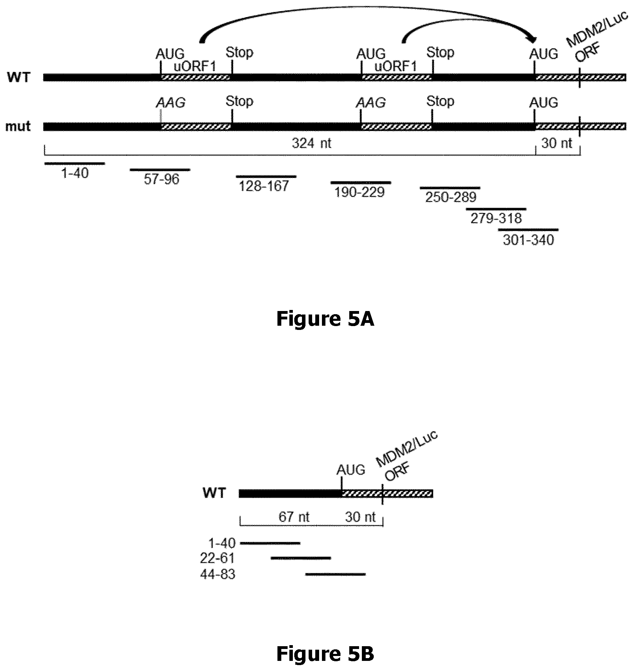

Figure 5A is a schematic representation of two luciferase reporter (luc) constructs comprising 5'-UTR sequences of MDM2 isoforms 1-2, targeted by AS-RNAs. The long 5'-UTR (324 nucleotides), harbouring two uORFs, was used in wildtype (WT) and mutated (mut) versions. In addition, 30 nucleotides of the MDM2 ORF, including the AUG start codon, were fused in-frame with the luciferase ORF. Arrows indicate potential effects of the uORFs on the translational output. Target regions of AS-circRNAs are represented as black lines with nucleotide coordinates.
Figure 5 B is a schematic representation of a third luciferase reporter construct comprising 5'-UTR sequences of MDM2 isoforms 3-6, targeted by AS-RNAs. The short 5'-UTR (67 nucleotides (nt)) of the MDM2 isoforms 3-6 is followed by 30 nucleotides of the MDM2 ORF, including the AUG start codon, fused in-frame with the luciferase ORF.

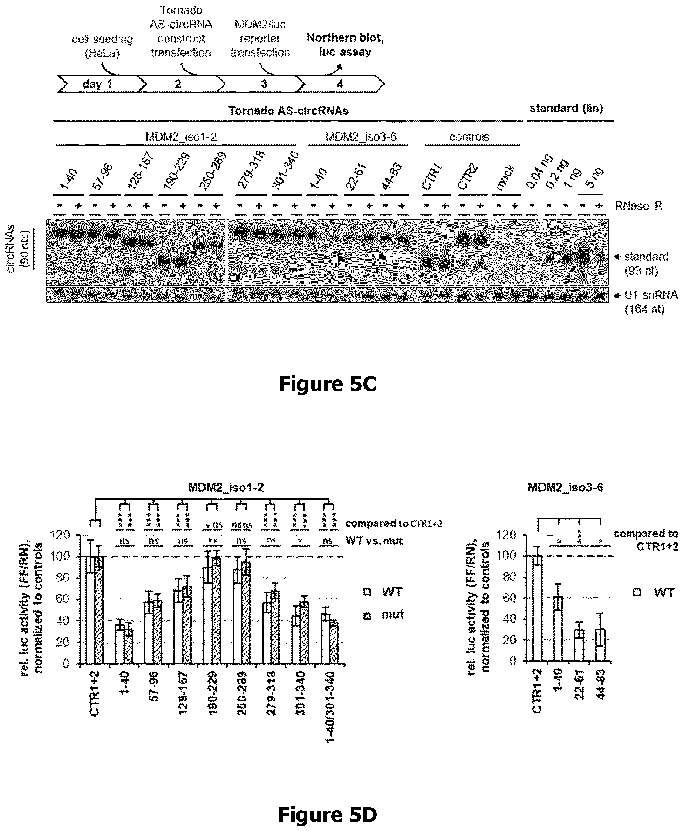

Figure 5C shows expression results of Tornado-based AS-circRNAs targeting the 5'-UTR of human MDM2 mRNA isoforms. In particular, Tornado constructs were transfected in HeLa cells, followed one day later by transfection of controls (CTR1, CTR2, mock treatment). AS-circRNA overexpression was detected by Northern blot, using a circular-junction specific probe. Circularity was confirmed by RNase R treatment (-/+). As an input control, U1 snRNA was detected by a U1-specific Northern probe (bottom). Note that U1 snRNA is partially resistent to degradation by RNase R due to its strong secondary structure. In addition, a linear standard RNA (93 nucleotides; 0.04 to 5 ng) containing the circular junction served to quantitate Northern signals.
Figure 5D shows the results of translation-inhibitory activities of AS-circRNAs targeting the 5'-UTR of human MDM2 mRNA isoforms. Luciferase reporter assays were performed in HeLa cells transfected with Tornado AS-circRNA overexpression constructs, as indicated, and MDM2_iso1-2 wildtype (WT) version (white bars) or MDM2_iso1-2 mutant version (hatched bars), or MDM2_iso3-6 wildtype (WT) reporters. Relative luciferase activities (Firefly/Renilla expression ratios) were measured, and normalized to control circRNA (CTR; mean and standard deviations of three replicates, ^{∗}*P<* 0.05, ^{∗∗}*P* < 0.005, ^{∗∗∗}*P* < 0.001, ns = not significant, two-sided ttest).

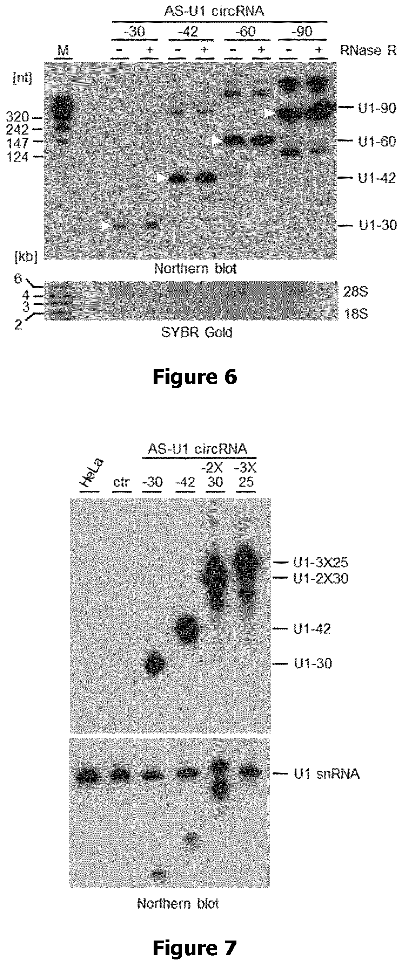

Figure 6 shows the results of Tornado-based overexpression of AS-U1-30, -42, -60, and -90 circRNAs. HeLa cells were transfected with Tornado-based circRNA constructs (AS-U1-30, AS-U1-42, AS-U1-60, AS-U1-90). One day later, total RNA was prepared, followed by mock or RNase R treatment (-/+), and detection of circRNAs (indicated by the arrows) by Northern blotting (top panel; denaturing PAGE). Ethidium-bromide staining of 18S and 28S rRNAs (bottom panel; agarose gel) served as input and RNase R control. *M*, markers for either PAGE (sized in nucleotides) or agarose gel electrophoresis (sizes in kb).
Figure 7 shows the results of the detection of transfected AS-circRNAs by Northern blotting. HeLa cells were transfected with purified AS-U1 circRNAs -30, -42, -2x30, -3x25, or control circRNA. Mock-transfected cells (HeLa) were included as a control. One day later, circRNAs were detected by Northern blotting (top panel), followed by reprobing with a U1-specific probe (bottom panel).

### The disclosure in particular relates to the following items:

1. A circular polynucleotide comprising a nucleotide sequence, which sequence comprises a targeting region comprising or consisting of a nucleotide sequence complementary to a target structure which is involved in shaping the cellular proteome, wherein
   the target structure is involved in pre-mRNA splicing and the polynucleotide is capable of at least one of:
      (i) inhibiting pre-mRNA splicing;
      (ii) resulting in intron retention;
      (iii) resulting in alternative 5' splice site use; and/or
      (iv) resulting in single-exon skipping, and
   the nucleotide sequence comprises a targeting region comprising or consisting of a nucleotide sequence complementary to a U1 snRNA or to a continuous or discontinuous portion of said U1 snRNA.
2. The polynucleotide of item 1, wherein the nucleotide sequence comprises a targeting region comprising or consisting of a nucleotide sequence complementary to a single-stranded region in the secondary structure, *e.g.,* in a loop sequence, of a U1 snRNA or to a continuous or discontinuous portion of said U1 snRNA.
3. The polynucleotide of item 1 or 2, wherein the nucleotide sequence of the targeting region is complementary to at least nucleotides 1 to 20, starting from the 5' end of the U1 snRNA nucleotide sequence.
4. The polynucleotide of item 3, wherein the nucleotide sequence of the targeting region complementary to at least the nucleotides 1 to 20 is flanked 5' by one or more additional nucleotides for targeting the nucleotide at position 21 up to at most to the nucleotide at position 100 or 70 of the U1 snRNA nucleotide sequence.
5. The polynucleotide of item 3, wherein the nucleotide sequence of the targeting region complementary to at least the nucleotides 1 to 20 is flanked 5' by one or more additional nucleotides for targeting the nucleotide at position 21 up to at most to the nucleotide at position 50 of the U1 snRNA nucleotide sequence.
6. The polynucleotide of item 3, wherein the nucleotide sequence of the targeting region complementary to at least the nucleotides 1 to 20 is flanked 5' by one or more additional nucleotides for targeting the nucleotide at position 21 up to at most to the nucleotide at position 42 of the U1 snRNA nucleotide sequence.
7. The polynucleotide of item 3, wherein the nucleotide sequence of the targeting region complementary to at least the nucleotides 1 to 20 is flanked 5' by one or more additional nucleotides for targeting the nucleotide at position 21 up to at most to the nucleotide at position 30 of the U1 snRNA nucleotide sequence.
8. The polynucleotide of any one of items 1 to 7, wherein the nucleotide sequence complementary to a U1 snRNA or to a continuous or discontinuous portion of said U1 snRNA is complementary to at least one continuous portion of the U1 snRNA, the continuous portion being selected from the group consisting of:
   (i) nucleotides 1 to 20 of the nucleotide sequence of the U1 snRNA;
   (ii) nucleotides 1 to 22 of the nucleotide sequence of the U1 snRNA;
   (iii) nucleotides 1 to 25 of the nucleotide sequence of the U1 snRNA;
   (iv) nucleotides 1 to 26 of the nucleotide sequence of the U1 snRNA;
   (v) nucleotides 1 to 30 of the nucleotide sequence of the U1 snRNA;
   (vi) nucleotides 1 to 40 of the nucleotide sequence of the U1 snRNA;
   (vii) nucleotides 1 to 42 of the nucleotide sequence of the U1 snRNA; and
   (viii) nucleotides 1 to 50 of the nucleotide sequence of the U1 snRNA.
9. The polynucleotide of any one of items 1 to 8, wherein the nucleotide sequence of the targeting region is depicted in any one of SEQ ID NO: 3 to SEQ ID: 6.
10. The polynucleotide of any one of items 1 to 9, wherein the targeting region of the polynucleotide comprises two or more copies of the nucleotide sequence complementary to a U1 snRNA or to a continuous or discontinuous portion of said U1 snRNA.
11. The polynucleotide of item 10, wherein the targeting region of the polynucleotide comprises two or three copies of the nucleotide sequence complementary to a U1 snRNA or to a continuous or discontinuous portion of said U1 snRNA.
12. The polynucleotide of item 10 or 11, wherein the nucleotide sequence comprises a targeting region comprising or consisting of a nucleotide sequence complementary to a single-stranded region in the secondary structure, *e.g.,* in a loop sequence, of a U1 snRNA or to a continuous or discontinuous portion of said U1 snRNA.
13. The polynucleotide of any one of items 10 to 12, wherein the nucleotide sequence complementary to a U1 snRNA or to a continuous or discontinuous portion of said U1 snRNA comprises a nucleotide sequence which is complementary to at least nucleotides 1 to 20 of the U1 snRNA nucleotide sequence.
14. The polynucleotide of item 13, wherein the nucleotide sequence complementary to at least the nucleotides 1 to 20 is flanked 5' by one or more additional nucleotides for targeting the nucleotide at position 21 up to at most to the nucleotide at position 50 of the U1 snRNA nucleotide sequence.
15. The polynucleotide of any one of items 10 to 12, wherein the nucleotide sequence complementary to a U1 snRNA or to a continuous or discontinuous portion of said U1 snRNA comprises a nucleotide sequence which is complementary to at least nucleotides 1 to 25 of the U1 snRNA nucleotide sequence.
16. The polynucleotide of item 15, wherein the nucleotide sequence complementary to at least the nucleotides 1 to 25 is flanked 5' by one or more additional nucleotides for targeting the nucleotide at position 26 up to at most to the nucleotide at position 50 of the U1 snRNA nucleotide sequence.
17. The polynucleotide of any one of items 13 to 16, wherein the nucleotide sequence complementary to a U1 snRNA or to a continuous or discontinuous portion of said U1 snRNA is complementary to at least one continuous portion of the U1 snRNA, the continuous portion being selected from the group consisting of:
   (i) nucleotides 1 to 20 of the nucleotide sequence of the U1 snRNA;
   (ii) nucleotides 1 to 22 of the nucleotide sequence of the U1 snRNA;
   (iii) nucleotides 1 to 25 of the nucleotide sequence of the U1 snRNA;
   (iv) nucleotides 1 to 26 of the nucleotide sequence of the U1 snRNA;
   (v) nucleotides 1 to 30 of the nucleotide sequence of the U1 snRNA;
   (vi) nucleotides 1 to 40 of the nucleotide sequence of the U1 snRNA;
   (vii) nucleotides 1 to 42 of the nucleotide sequence of the U1 snRNA; and
   (viii) nucleotides 1 to 50 of the nucleotide sequence of the U1 snRNA.
18. The polynucleotide of any one of items 10 to 17, wherein the nucleotide sequence of the continuous portion is depicted in any one of SEQ ID NO: 3 to SEQ ID: 6.
19. The polynucleotide of item 17 or 18, wherein the targeting region comprises two or three copies of any of the continuous portions.
20. The polynucleotide of any one items 10 to 19, wherein between the nucleotides of one or more of the continuous portions a non-complementary sequence of two or more nucleotides is inserted.
21. The polynucleotide of any one of items 10 to 20, wherein the nucleotide sequence of the targeting region is depicted in any one of SEQ ID NO: 7 to SEQ ID: 9.
22. The polynucleotide of any one of items 1 to 21, wherein the U1 snRNA is human U1 snRNA as depicted in SEQ ID NO: 1.
23. A polynucleotide comprising a nucleotide sequence, which sequence comprises a targeting region comprising or consisting of a nucleotide sequence complementary to a target structure which is involved in shaping the cellular proteome, wherein
   the target structure is involved in mRNA translation and the polynucleotide is capable of inhibiting mRNA translation, and
   the nucleotide sequence comprises a targeting region comprising or consisting of a nucleotide sequence complementary to a 5' untranslated region (5'-UTR) or to a continuous or discontinuous portion of said 5' untranslated region of the mouse double minute 2 homolog (MDM2) mRNA.
24. The polynucleotide of item 23, wherein the polynucleotide is a circular polynucleotide.
25. The polynucleotide of item 23 or 24, wherein the nucleotide sequence of the 5'-UTR is depicted in SEQ ID NO: 10.
26. The polynucleotide of item 25, wherein the targeting region is complementary to at least the nucleotides 22 to 40 of the 5'-UTR nucleotide sequence.
27. The polynucleotide of item 26, wherein the targeting region complementary to at least the nucleotides 22 to 40 of the 5'-UTR is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.
28. The polynucleotide of item 26 or 27, wherein the nucleotide sequence of the targeting region complementary to at least nucleotides 22 to 40 of the 5'-UTR is flanked 3' by one or more additional nucleotides for targeting the nucleotide at position 21 up to at most to the nucleotide at position 1 of the 5'-UTR sequence.
29. The polynucleotide of item 26 or 27, wherein the nucleotide sequence of the targeting region complementary to at least nucleotides 22 to 40 of the 5'-UTR is flanked 5' by one or more additional nucleotides for targeting the nucleotide at position 41 up to at most to the nucleotide at position 50 of the 5'-UTR sequence.
30. The polynucleotide of item 25, wherein the targeting region is complementary to at least the nucleotides 67 to 86 of the 5'-UTR nucleotide sequence.
31. The polynucleotide of item 30, wherein the targeting region complementary to at least the nucleotides 67 to 86 of the 5'-UTR is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.
32. The polynucleotide of item 30 or 31, wherein the nucleotide sequence of the targeting region complementary to at least nucleotides 67 to 86 of the 5'-UTR is flanked 3' by one or more additional nucleotides for targeting the nucleotide at position 66 up to at most to the nucleotide at position 47 of the 5'-UTR sequence.
33. The polynucleotide of item 30 or 31, wherein the nucleotide sequence of the targeting region complementary to at least nucleotides 67 to 86 of the 5'-UTR is flanked 3' by one or more additional nucleotides for targeting the nucleotide at position 66 up to at most to the nucleotide at position 57 of the 5'-UTR sequence.
34. The polynucleotide of any one of items 30 to 33, wherein the nucleotide sequence of the targeting region complementary to at least nucleotides 67 to 86 of the 5'-UTR is flanked 5' by one or more additional nucleotides for targeting the nucleotide at position 87 up to at most to the nucleotide at position 106 of the 5'-UTR sequence.
35. The polynucleotide of any one of items 30 to 33, wherein the nucleotide sequence of the targeting region complementary to at least nucleotides 67 to 86 of the 5'-UTR is flanked 5' by one or more additional nucleotides for targeting the nucleotide at position 87 up to at most to the nucleotide at position 96 of the 5'-UTR sequence.
36. The polynucleotide of item 25, wherein the targeting region is complementary to at least the nucleotides 138 to 157 of the 5'-UTR nucleotide sequence.
37. The polynucleotide of item 36, wherein the targeting region complementary to at least the nucleotides 138 to 157 of the 5'-UTR is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.
38. The polynucleotide of item 36 or 37, wherein the nucleotide sequence of the targeting region complementary to at least nucleotides 138 to 157 of the 5'-UTR is flanked 3' by one or more additional nucleotides for targeting the nucleotide at position 137 up to at most to the nucleotide at position 118 of the 5'-UTR sequence.
39. The polynucleotide of item 36 or 37, wherein the nucleotide sequence of the targeting region complementary to at least nucleotides 138 to 157 of the 5'-UTR is flanked 3' by one or more additional nucleotides for targeting the nucleotide at position 137 up to at most to the nucleotide at position 128 of the 5'-UTR sequence.
40. The polynucleotide of any one of items 36 to 39, wherein the nucleotide sequence of the targeting region complementary to at least nucleotides 138 to 157 of the 5'-UTR is flanked 5' by one or more additional nucleotides for targeting the nucleotide at position 158 up to at most to the nucleotide at position 177 of the 5'-UTR sequence.
41. The polynucleotide of any one of items 36 to 39, wherein the nucleotide sequence of the targeting region complementary to at least nucleotides 138 to 157 of the 5'-UTR is flanked 5' by one or more additional nucleotides for targeting the nucleotide at position 158 up to at most to the nucleotide at position 167 of the 5'-UTR sequence.
42. The polynucleotide of item 25, wherein the targeting region is complementary to at least the nucleotides 301 to 318 of the 5'-UTR nucleotide sequence.
43. The polynucleotide of item 42, wherein the targeting region complementary to at least the nucleotides 301 to 318 of the 5'-UTR is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.
44. The polynucleotide of item 42 or 43, wherein the nucleotide sequence of the targeting region complementary to at least nucleotides 301 to 318 of the 5'-UTR is flanked 3' by one or more additional nucleotides for targeting the nucleotide at position 300 up to at most to the nucleotide at position 269 of the 5'-UTR sequence.
45. The polynucleotide of item 42 or 43, wherein the nucleotide sequence of the targeting region complementary to at least nucleotides 301 to 318 of the 5'-UTR is flanked 3' by one or more additional nucleotides for targeting the nucleotide at position 300 up to at most to the nucleotide at position 279 of the 5'-UTR sequence.
46. The polynucleotide of any one of items 42 to 45, wherein the nucleotide sequence of the targeting region complementary to at least nucleotides 301 to 318 of the 5'-UTR is flanked 5' by one or more additional nucleotides for targeting the nucleotide at position 319 up to at most to the nucleotide at position 324 of the 5'-UTR sequence.
47. The polynucleotide of any one of items 25 to 46, wherein the nucleotide sequence of the targeting region is complementary to at least one continuous portion of the 5' untranslated region, the continuous portion being selected from the group consisting of:
   (i) nucleotides 1 to 40 of the sequence depicted in SEQ ID NO: 10;
   (ii) nucleotides 57 to 96 of the sequence depicted in SEQ ID NO: 10;
   (iii) nucleotides 128 to 167 of the sequence depicted in SEQ ID NO: 10
   (iv) nucleotides 279 to 318 of the sequence depicted in SEQ ID NO: 10; and
   (v) nucleotides 301 to 324 of the sequence depicted in SEQ ID NO: 10.
48. The polynucleotide of any one of items 25 to 47, wherein the nucleotide sequence of the targeting region is depicted in any one of SEQ ID NO: 11 to SEQ ID NO: 31.
49. The polynucleotide of any one of items 25 to 48, wherein the nucleotide sequence of the targeting region comprises the sequences as depicted in SEQ ID NO: 31, wherein the sequence is additionally flanked 5' in a contiguous manner by one or more nucleotides for targeting nucleotide +1 up to at most nucleotide +26, preferably up to at most nucleotide +16 of the main ORF.
50. The polynucleotide of item 23 or 24, wherein the nucleotide sequence of the 5'-UTR is depicted in SEQ ID NO: 32.
51. The polynucleotide of item 50, wherein the targeting region is complementary to at least the nucleotides 22 to 40 of the 5'-UTR nucleotide sequence.
52. The polynucleotide of item 51, wherein the targeting region complementary to at least the nucleotides 22 to 40 of the 5'-UTR is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.
53. The polynucleotide of item 51 or 52, wherein the nucleotide sequence of the targeting region complementary to at least nucleotides 22 to 40 of the 5'-UTR is flanked 3' by one or more additional nucleotides for targeting the nucleotide at position 21 up to at most to the nucleotide at position 1 of the 5'-UTR sequence.
54. The polynucleotide of item 51 or 52, wherein the nucleotide sequence of the targeting region complementary to at least nucleotides 22 to 40 of the 5'-UTR is flanked 3' by one or more additional nucleotides for targeting the nucleotide at position 21 up to at most to the nucleotide at position 10 of the 5'-UTR sequence.
55. The polynucleotide of any one of items 51 to 54, wherein the nucleotide sequence of the targeting region complementary to at least nucleotides 22 to 40 of the 5'-UTR is flanked 5' by one or more additional nucleotides for targeting the nucleotide at position 41 up to at most to the nucleotide at position 61 of the 5'-UTR sequence.
56. The polynucleotide of any one of items 51 to 54, wherein the nucleotide sequence of the targeting region complementary to at least nucleotides 22 to 40 of the 5'-UTR is flanked 5' by one or more additional nucleotides for targeting the nucleotide at position 41 up to at most to the nucleotide at position 50 of the 5'-UTR sequence.
57. The polynucleotide of item 50, wherein the targeting region is complementary to at least the nucleotides 44 to 67 of the 5'-UTR nucleotide sequence.
58. The polynucleotide of item 57, wherein the targeting region complementary to at least the nucleotides 44 to 67 of the 5'-UTR is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.
59. The polynucleotide of item 57 or 58, wherein the nucleotide sequence of the targeting region complementary to at least nucleotides 44 to 67 of the 5'-UTR is flanked 3' by one or more additional nucleotides for targeting the nucleotide at position 43 up to at most to the nucleotide at position 32 of the 5'-UTR sequence.
60. The polynucleotide of any one of items 50 to 59, wherein the nucleotide sequence of the targeting region is complementary to at least one continuous portion of the 5' untranslated region, the continuous portion being selected from the group consisting of:
   (i) nucleotides 1 to 40 of the sequence depicted in SEQ ID NO: 32;
   (ii) nucleotides 22 to 61 of the sequence depicted in SEQ ID NO: 32; and
   (iii) nucleotides 44 to 67 of the sequence depicted in SEQ ID NO: 32.
61. The polynucleotide of any one of items 50 to 60, wherein the nucleotide sequence of the targeting region is depicted in any one of SEQ ID NO: 33 to SEQ ID: 39.
62. The polynucleotide of any one of items 50 to 61, wherein the nucleotide sequence of the targeting region comprises the sequences as depicted in SEQ ID NO: 38, wherein the sequence is additionally flanked 5' in a contiguous manner by one or more nucleotides for targeting nucleotide +1 up to at most nucleotide +26, preferably up to at most nucleotide +16 of the ORF.
63. The polynucleotide of any one of items 1 to 62, wherein the nucleotide sequence of the targeting region has at least 70%, at least 75%, at least 80%, at least 85%, at least 90%, at least 95%, at least 97%, or at least 99% identity to the sequence depicted in any one of SEQ ID NO: 3 to 8, 15 to 17, 20, 21, 23 to 25, respectively.
64. The polynucleotide of any one of items 1 to 63, wherein the nucleotide sequence of the targeting region is at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% complementary to the U1 snRNA or the 5' untranslated region or a continuous or discontinuous portion thereof as defined in any one of claims 22, 25, 48, 50 and 63.
65. The polynucleotide of any one of items 1 to 64, wherein the nucleotide sequence of the targeting region is 100% complementary to the U1 snRNA or the 5'-untranslated region or a continuous or discontinuous portion thereof.
66. The polynucleotide of any one of items 1 to 65, wherein the nucleotide sequence of the targeting region is at least 15 nucleotides, at least 20 nucleotides, or at least 25 nucleotides in length.
67. The polynucleotide of any one of items 1 to 66, wherein the nucleotide sequence of the targeting region is at most 500 nucleotides, at most 250 nucleotides, or at most 150 nucleotides in length.
68. The polynucleotide of any one of items 1 to 67, wherein the polynucleotide comprises at least one ribonucleotide.
69. The polynucleotide of any one of items 1 to 68, wherein the polynucleotide consists of ribonucleotides.
70. The polynucleotide of any one of items 1 to 69, wherein the polynucleotide comprises at least one deoxyribonucleotide.
71. The polynucleotide of any one of items 1 to 68 or 70, wherein the polynucleotide comprises a mixture of ribonucleotides and deoxyribonucleotides.
72. The polynucleotide of any one of items 1 to 71, wherein the polynucleotide comprises at least one modified nucleotide.
73. A pharmaceutical composition comprising one or more of the polynucleotides of any one of items 1 to 72, and a pharmaceutically acceptable carrier.
74. A pharmaceutical composition of item 73 for use as a medicament.
75. A polynucleotide of any one of items 1 to 72 or a pharmaceutical composition of item 73 for use in a method of treating or preventing a proliferative disorder, said method comprising a step of administering to a patient in need thereof one or more of the polynucleotides or the pharmaceutical composition.
76. The polynucleotide or the pharmaceutical composition for use of item 75, wherein the proliferative disorder is cancer.
77. A method of treating or preventing a proliferative disease comprising administering to a patient in need thereof one or more of the polynucleotides of any one of items 1 to 72 or the pharmaceutical composition of claim 73.
78. The method of item 77, wherein the proliferative disease is cancer.
79. The method of item 77 or 78 or the polynucleotides or the pharmaceutical composition for use of item 75, wherein the one or more polynucleotides or the pharmaceutical composition is/are administered locally or systemically.
80. The method or the polynucleotides or the pharmaceutical composition for use of item 79, wherein the one or more polynucleotides or the pharmaceutical composition is/are administered parenterally.
81. The method or the polynucleotides or the pharmaceutical composition for use of any one of items 77 to 80, wherein the one or more polynucleotides or the pharmaceutical composition is/are administered alone or in combination with any other agent.
82. The method of or the polynucleotides or the pharmaceutical composition for use of item 81, wherein the other agent is selected from a chemotherapeutic agent, an immunotherapeutic agent, a cytokine, a hormone, lipids or liposomes, or any combination thereof.
83. A method for targeting a nucleic acid comprising contacting the nucleic acid to be targeted with a polynucleotide of any one of items 1 to 72.
84. A method for forming a complex comprising a polynucleotide of any one of items 1 to 72, and a single-stranded nucleic acid, the method comprising a step of contacting the polynucleotide with the single-stranded nucleic acid.
85. The method of item 84, wherein the single-stranded nucleic acid is a nucleic acid comprising or consisting of ribonucleotides.
86. A method for interfering with genome expression comprising contacting a polynucleotide of any one of items 1 to 72 with the nucleic acid of the cell or a patient.
87. The method of any one of items 77 to 86, wherein the method is an *in vitro* or an *in vivo* method.

### EXAMPLES

### Plasmids

### Tornado-based antisense-U1 and -MDM2 circRNA expression constructs:

The Tornado-based antisense-U1 and -MDM2 circRNA expression constructs as shown in Table 27 were prepared using standard PCR and cloning methods known to the skilled person:

**Table 27:**

| **antisense-U1 circRNAs** | **antisense-MDM2 circRNAs** |
|---|---|
| Tornado U1 AS 30nt | 1-40 (iso1-2) |
| Tornado U1 AS 42nt | 57-96 (iso1-2) |
| Tornado U1 AS 60nt | 128-167 (iso1-2) |
| Tornado U1 AS 90nt | 190-229 (iso1-2) |
| Tornado Control | 250-289 (iso1-2) |
| Minx | 279-318 (iso1-2) |
| | 301-340 (iso1-2) |
| | 1-40 (iso3-6) |
| | 22-61 (iso3-6) |
| | 44-83 (iso3-6) |
| | CTR1 |
| | CTR2 |

Each of the AS-U1-30, -42, -60, and -90 constructs carry an antisense region against the human U1 snRNA. AS-U1-30 is designed to specifically target the region starting at the very 5'-terminus of U1 snRNA and extending to nucleotide 30 of the sequence as shown in SEQ ID NO: 1. AS-U1-42 is designed to specifically target the region starting at the very 5'-terminus of U1 snRNA and extending to nucleotide 42 of the sequence as shown in SEQ ID NO: 1. AS-U1-60, also referred to as AS-US-2x30 herein, is designed to specifically target the region starting at the very 5'-terminus of U1 snRNA and extending to nucleotide 30 of the sequence as shown in SEQ ID NO: 1, but comprises two copies of the 1-30 antisense-U1 region. AS-U1-90, also referred to as AS-US-3x30 herein, is designed to specifically target the region starting at the very 5'-terminus of U1 snRNA and extending to nucleotide 30 of the sequence as shown in SEQ ID NO: 1, but comprises three copies of the 1-30 antisense-U1 region.

Antisense sequences of 40 nucleotides target the 5'-UTR of MDM2, including the two uORFs in the long 5'-UTR of MDM2_iso 1-2; as shown in Figure 5A (MDM2_iso1-2) and Figure 5B (MDM2_iso3-6). The nucleotides of the respective 5'-UTR sequence to be targeted are indicated by the numbers. For example, "1-40 (iso1-2)" is designed to specifically target the region from nucleotide 1 to nucleotide 40 of the 5' UTR iso1-2 sequence of the MDM2 as shown in SEQ ID NO: 10. "57-96 (iso1-2)" is designed to specifically target the region from nucleotide 57 to nucleotide 96 of the 5' UTR iso1-2 sequence of the MDM2 as shown in SEQ ID NO: 10, and so forth.

Double-stranded DNA oligonucleotides with NotI and SacII overhangs were inserted into NotI-/SacII-digested pAV-U6+27-Tornado-Broccoli vector (obtained from Addgene; Litke, J.L., and Jaffrey, S.R., Nat. Biotechnol. 37 (2019): 667-675). To enhance flexibility, short spacers were inserted when multiple copies of antisense sequence were used. Randomized sequences of similar length were used as controls.

### Minigenes:

The MINX minigene for transfection was cloned between the KpnI/BamHI sites of pcDNA3, using a PCR product from the original SP6 MINX construct (Zillmann, M., et al., Mol. Cell. Biol. 8(1988): 814-821).

The PLCD3 minigene was cloned by PCR in pcDNA3 and contains exon 14 (102 nucleotides), exon 15 (150 nucleotides), and exon 16 (80 nucleotides), with shortened introns (intron 14 to 109 nucleotides, intron 15 to 158 nucleotides; Refseq NM_133373).

For the CD45 WT minigene, see Preuβner M., et al., Nucleic Acids Res. 40(2012): 5666-5678.

### Luciferase reporter constructs:

The 5'-UTR (nucleotides 1-50, as shown in SED ID NO: 40) sequence of human hemoglobin subunit beta (β-globin; HBB) was cloned into pcDNA5-CMV-FF (Medenbach, J., et al., Cell 145 (2011): 902-913) containing the Firefly reporter ORF, using the HindIII and BamHI restriction sites.

MDM2 comprises six main isoforms (isoform 1: NM_002392, isoform 2: NM_001145339, isoform 3: NM_001145337, isoform 4: NM_001145340, isoform 5: NM_001278462, isoform 6: NM_001367990). Isoforms 1 and 2 contain a long 5'-UTR sequence (nucleotides 1-324, as shown in SEQ ID NO: 10) with two additional uORFs. Isoforms 3-6 have a short 5'-UTR sequence (nucleotides 1-67; without uORFs, as shown in SEQ ID NO: 32). Both the long and short 5'-UTR sequences were cloned into pcDNA5-CMV-FF using BamHI-H3 sites. In addition, 30 nucleotides of the MDM2 ORF sequence, identical in all isoforms, were inserted, followed in-frame by the Firefly ORF in both reporters (as shown in Figures 5A and 5B). For the construction of a reporter with mutated uORFs, oligonucleotide cassettes with corresponding nucleotide changes (T→A) were cloned in front of the Firefly reporter ORF.

### Example 1: In vitro transcription and circularization of short designer circRNAs

RNAs were transcribed from annealed DNA-oligonucleotide templates, using the HighScribe^{™} T7 high-yield RNA synthesis kit (NEB) in the presence of ATP, CTP, UTP, and GTP (each at 7.5 mM), GMP (30 mM GMP; Merck), and RNaseOut (Thermo Fisher Scientific) for 2 h at 37°C. The DNA template was digested by the addition of RQ1 DNase (2 U per 20 µl-reaction, Promega), and incubation for 30 min at 37°C. Transcripts were purified using the Monarch RNA purification kit (NEB). If used for antisense-affinity selection, RNA transcripts were internally labeled by incorporation of 2.5 mM (33% of total UTP) azide-modified UTP analog (Jena Bioscience), and - after circularization- biotinylated with copper-free Click chemistry (DABCO-containing biotin; Jena Bioscience).

For circularization, 100 µg transcribed RNA were incubated with 100 U of T4 RNA ligase (Thermo Fisher Scientific) in T4 RNA ligase buffer, supplemented with 0.1 mg/ml BSA and RNaseOut (Thermo Fisher Scientific), overnight at 16°C in a final volume of 200 µl. RNA was recovered by phenol/chloroform extraction (Roth) and ethanol precipitation or by using the Monarch RNA Cleanup Kit (NEB).

Gel purification was performed as described (Breuer, J., and Rossbach, O., Methods Protoc. 3 (2020): 42). To validate the circular configuration, 250 ng of gel-purified circular or linear RNA were incubated with or without 2 U of RNase R (Lucigen) per 5-µl reaction (30 min at 37°C). After digestion, 200 ng of RNAs were separated by 10% (as shown in Figure 1B) or 12% (as shown in Figure 4B) denaturing PAGE and visualized by ethidium bromide staining.

### Example 2: Antisense-circRNAs (AS-circRNAs) directed against the human U1 snRNA

A series of small AS-U1 circular RNAs (circRNAs) were designed to specifically target regions, starting at the very 5'-terminus of U1 snRNA and extending to various lengths, ranging from 11 to 42 perfect base pairs (AS-U1-11, -22, -30, and 42; as shown in Figure 1A). In addition, AS-U1 circRNAs with further extended base-pairing potential were tested. AS-U1-2x30, with two copies of the 1-30 antisense-U1 region, and AS-U1-3x25, with three copies of a 1-25 antisense-U1 region, the 1-25 antisense-U1 region is designed to specifically target the region, starting at the very 5'-terminus of U1 snRNA up to nucleotide 25 of the U1 snRNA.
Circularizable polynucleotide (CP) AS-U1-11 (SEQ ID NO: 48):
   GGGAGTAAGC**CAGGTAAGTAT**GCTTACAGTA
Circularizable polynucleotide (CP) AS-U1-22 (SEQ ID NO: 49):
   GGGAGTAAGC**ATCTCCCCTGCCAGGTAAGTAT**GCTTACAGTA
Circularizable polynucleotide (CP) AS-U1-30 (SEQ ID NO: 50):
   GGGAGTAAGC**ATCATGGTATCTCCCCTGCCAGGTAAGTAT**GCTTACAGTA
Circularizable polynucleotide (CP) AS-U1-42 (SEQ ID NO: 51):
   GGGAGTAAGC**ACCACCTTCGTGATCATGGTATCTCCCCTGCCAGGTAAGTAT**GCTTACAGTA
Circularizable polynucleotide (CP) AS-U1-2x30 (SEQ ID NO: 52): 
GGGAGTAAGCATCATGGTATCTCCCCTGCCAGGTAAGTATAATTAAATCATGGTATCTCCCCTG
CCAGGTAAGTATGCTTACAGTA
Circularizable polynucleotide (CP) AS-U1-3x25 (SEQ ID NO: 53): 
GGGAGTAAGCGGTATCTCCCCTGCCAGGTAAGTATATATGGTATCTCCCCTGCCAGGTAAGTAT
ATATGGTATCTCCCCTGCCAGGTAAGTATGCTTACAGTA
Circularizable polynucleotide (CP) CTR (SEQ ID NO: 54):
   GGGAGTAAGC**CCTGCCTGTCTATTGATGTC**GCTTACAGTA

Each circular RNA was designed such that the targeting sequence (shown in bold letters in the sequences shown in this example) was linked to a common short stem-loop comprising 6 perfect base-pairs (underlined letters in the above sequences) with two overhanging ends that were joined by ligation. The circular RNAs were generated by *in vitro* T7-transcription and ligation, followed by gel purification. The circular configuration of the purified circRNAs was confirmed by their resistance to the exonuclease RNase R, as well by their unusual mobility in denaturing PAGE (as shown in Figure 1B).

### Example 3: Pulldown with biotinylated transcripts and silver staining

Biotin-labeled circular or linear RNAs were incubated with HeLa nuclear extract, followed by affinity selection. For *in vitro* binding assays, 1 µg of biotinylated linear or circular RNA was incubated with 20 µl nuclear extract (CILBiotech, Belgium) and 180 µl WB100 buffer (100 mM KCI, 10 mM MgCl₂, 0.01% NP-40, 20 mM Hepes pH 7.5, 1 mM DTT) for one hour (at room temperature, rotating), followed by binding to 20 µl blocked Neutravidin agarose resin (Thermo Scientific, rotating for two hours at 4°C). Beads were washed five times with WB150 buffer (150 mM KCI, 10 mM MgCl₂, 0.01% NP-40, 20 mM Hepes pH 7.5, 1 mM DTT). Selected RNAs were eluted by incubating with 200 µl proteinase K buffer (150 mM NaCl, 12.5 mM EDTA, 1% SDS, 100 mM Tris pH 8.0) for 10 min at 80°C, followed by phenol extraction and ethanol precipitation. Isolated RNA was analyzed by 10% denaturing PAGE and silver staining (Bio-Rad), whereby all the spliceosomal snRNAs, 7SL RNA, and tRNA can be detected by silver staining (as shown in Figure 1D).

Comparing RNA from depleted extracts and the affinity-selected RNA, it can be seen that both AS-U1 circRNA with 22 and 30 base-pair regions were efficient and specific for the selection of U1 snRNPs from extract.

### Example 4: In vitro splicing

As a first functional assessment, the effect of AS-U1-circRNA on *in vitro* splicing of a model twoexon pre-mRNA, MINX, in HeLa nuclear extract, was assayed focussing on the AS-U1-30 circRNA (as shown in Figure 1E). AS-U1 circRNA (or a control circRNA of similar length, as shown in SEQ ID NO: 54), were added to nuclear extract, for comparison also in linear configuration, and titrating amounts between 100 and 1000 ng per 25-µl splicing reaction.

In particular, BamHI-digested MINX plasmid DNA was transcribed in the presence of m⁷GpppG cap analog (NEB). *In vitro* splicing assays were performed in HeLa nuclear extract (CILBiotech, Belgium), preincubating 5 ng of MINX pre-mRNA for 15 min in a 25-µl reaction, followed by the addition of 100, 200, 500, or 1000 ng (as shown in Figure 1E), or one or four µg (as shown in Figure 1F) of circular or linear AS-U1 RNA, and incubation for 60 min. Splicing activity was detected by RT-PCR analysis with gene-specific primers, detecting unspliced pre-mRNA and spliced mRNA.

The control RNA (CTR; SEQ ID NO: 54), either circular or linear, had no significant effect on splicing efficiencies; in contrast, the AS-U1-30 circRNA showed the strongest splicing-inhibitory effects, in a dose-dependent manner [splicing efficiencies at the highest concentration reduced from 78% (control) to 47% (anti-U1-30)]. The linear counterpart was less effective at all concentrations, with efficiencies reduced to levels between 69 and 55%).

AS-U1 circRNAs with further extended base-pairing potential were also tested in these *in vitro* splicing inhibition assays (as shown in Figure 1C): AS-U1-42, AS-U1-2x30, and AS-U1-3x25, all in comparison to the AS-U1-30 and the negative control circRNA. In this series of assays, one and four µg of each circRNA per 25-µl splicing reaction were tested, and for each of them, dose dependence was observed. Extending the antisense-U1 region further strongly increases splicing inhibition by AS-circRNAs, at the highest concentration from 75% (control) to 36% (AS-U1-30), 27% (AS-U1-42), 29% (AS-U1-2x30), and 8% (AS-U1-3x25), respectively. In sum, AS-U1 circRNAs efficiently target U1 snRNAs in extracts, can be applied for highly specific affinity selection or functional depletion of U1 snRNA, resulting in splicing inhibition.

### Example 5: Cotransfection of Tornado constructs and minigenes, splicing assays

Next *in vivo* activity of AS-U1 circRNAs was assayed, using the Tornado system for circRNA overexpression. The Tornado system relies on ribozyme-mediated excision of the sequence to be circularized from a construct, followed by circularization catalyzed by the endogenous tRNA ligase RtcB. Four different circRNAs AS-U1-30, AS-U1-42, AS-U1-60 (=AS-U1-2×30) and AS-U1-90 (=AS-U1-3x30), and a control antisense sequence (see SEQ ID NOs: 56 and 57) were overexpressed (as shown in Figure 2A and Figure 2B).

Each of these Tornado constructs was transfected in HeLa cells, in combination with splice reporter minigene constructs. For detecting splicing effects by overexpressed AS-U1 circRNAs, HeLa cells were plated on 6-well plates (4 × 10⁵ cells per well) and cultured in Dulbecco's modified Eagle's medium (DMEM, Invitrogen). Both AS-U1-Tornado constructs (AS-U1-30, AS-U1-42, AS-U1-60 (2x30), or AS-U1-90 (3x30)) and the minigene constructs (MINX, PLCD3, or CD45) were reverse-transfected together into cells, using 2 µg of the antisense-U1-Tornado constructs, 2 µg of minigenes, and 8 µl Turbofect (Thermo Fisher Scientific) per well (in 500 µl Opti-MEM). Cells were stored in a CO₂ incubator (5%) at 37°C (Excella Eco-170). The medium was changed after 4 h, and the cells were harvested after 48 h. In the case of the MINX minigene (as shown in Figure 2), the Tornado construct was reverse-transfected for 24 hours, followed by forwardtransfection of MINX for four more hours.

RNA was isolated using TRIzol (Ambion), isopropanol precipitation and treatment with RQ1 DNase (Promega), followed by reverse transcription (qScript cDNA Synthesis Kit, Quanta) and splicing assays by PCR with gene-specific primers.

The following splice reporter minigene constructs were used:
- MINX, a minimal splicing construct with two exons;
- PLCD3, an alternative-splicing minigene with three exons (Rösel-Hillgärtner, T.D., et al., PLoS Genet. 9(2013): e1003856); or
- a more complex, CD45-derived alternative-splicing minigene (Preussner, M., et al., Nucleic Acids Res. 40(2012): 5666-5678), with two constant flanking exons 3 and 7, and three variable exon 4, 5, and 6 in between, that are included in various combinations.

All five expected circRNAs were overexpressed in the cells co-transfected with either splice reporter (for Northern blot analysis, see Figure 2B; for confirmation of circularity by RNase R treatment, see Figure 6). To detect whether this series of AS-U1 circRNAs affected general splicing activity and alternative splicing patterns, RT-PCR analysis for the three splice reporter minigenes was performed. The results are shown in Figure 2C.

MINX pre-mRNA splicing efficiency was reduced from the control level of 77% to levels between 53 and 57% for each of the AS-U1 circRNAs. Splicing of the three-exon PLCD3 minigene was analyzed for both efficiency (of exon 15-16 splicing) as well as for inclusion of exon 15. The splicing efficiency was reduced by AS-U1 circRNAs -42, -60 (2x30), and -90 (3x30) from 81% (control) to levels between 56 and 65%. PLCD3 exon 15 inclusion was reduced by all four AS-U1 circRNAs from 42% (control) to between 18 and 36%.

The splicing efficiency and the complex alternative splicing pattern of CD45 was assayed, using a five-exon minigene derivative with the three variable exons 4, 5, and 6. The splicing efficiency of exons 5 and 6 (75% for the control) was reduced to 57% by AS-U1-30 circRNA, and to levels between 12 and 22% by the AS-U1 circRNAs with longer complementarity (-42, -60 (2x30), and -90 (3x30). Analyzing alternative splicing of exons 4-5-6 (measuring the R0/R45 isoform ratio: complete skipping, R0, to exons 4-5 inclusion, R45) indicated also strong effects, in particular for the longer antisense-circRNAs AS-U1-42, AS-U1-60 (2x30) and AS-U1-90 (3x30) from 49% to 19-26%. In summary, for each of the three minigenes it was shown that overexpressed antisense-U1 circRNAs can modulate splicing *in vivo.* The effect increases with extended complementarity.

### Example 6: Transfected antisense-U1 circRNAs modulate alternative splicing of cellular genes.

As an alternative way to the Tornado system, *in-vitro-*transcribed and gel-purified circRNAs were transfected into HeLa cells. With this approach also dosis-dependent effects could be studied.

For circRNA transfection, 3 × 10⁵ HeLa cells were seeded on 6-well plates one day before, and 5 µg circular RNA were transfected using Lipofectamine 2000. After 24 h, RNA was isolated (TRIzol, Ambion), followed by reverse transcription (qscript cDNA Synthesis Kit; Quanta) and alternative splicing assays by PCR with gene-specific primers. PCR products were quantified by densitometry, using the Image3 software, and are based on three biological replicates.

For quantitative analysis of intron retention, real-time qPCR was carried out, using the StepOne Plus Real-Time PCR System (Thermo Fisher Scientific) as well as the Luna qPCR Reaction Mix (NEB), according to the manufacturer's instructions. All reactions were performed in biological triplicates. The evaluation was carried out, using the ΔΔCt method with average cycle threshold (Ct) values (Pfaffl, M.W., Nucleic Acids Res, 29 (2001): e45). Normalization was performed with Ct values of housekeeping gene ACTB in corresponding samples, relative to the control samples.

Four different AS-U1 circRNAs were generated and compared: AS-U1-30 and AS-U1-42, each with a single copy of sequence perfectly complementary to nucleotides 1-30 and 1-42 of U1 snRNA, respectively; AS-U1-2x30 and AS-U1-3x25, with two or three copies of antisense sequence to the first 30 or 25 nucleotides, respectively, of U1 snRNA (for details see SEQ ID NOs: 50 to 53). As a negative control, a circRNA was used with a random-generated 20-nucleotide sequence (SEQ ID NO: 54) inserted instead of the antisense sequence.

The four different, gel-purified AS-U1 circRNAs (or the control circRNA) were transfected in HeLa cells, and 24 h later, alternative splicing (intron retention, alternative 5'ss use, and exon skipping; see Figure 3A) was analyzed by RT-PCR and gene-specific primers. For Northern blot analysis of circRNA expression, see Figure 7).

To identify endogenous target genes, that are particularly sensitive to antisense-U1 circRNAs, RNA-seq analysis was applied with total RNA from HeLa cells transfected with the AS-U1-42 circRNA (or control) to analyze alternative splicing patterns. Since U1 snRNAs is essential for 5' splice site definition, three modes of alternative splicing were analyzed: intron retention, alternative 5' splice site use, and single-exon skipping. Five alternative splicing targets with most significant effects were selected from each of the three alternative-splicing modes for RT-PCR validation, using all four AS-U1 circRNA samples (Figure 3B and Figure 3C), allowing direct comparison of the activities of the different AS-U1 circRNAs. The positions of all exons of these target genes is shown in Table 28, below:

**Table 28: Alternative splicing targets**

| **A. Intron retention targets** | | | |
|---|---|---|---|
| gene_id | chromosome (strand) | RefSeq | target intron position (intron #) |
| RPL24 | chr3 (-) | NM_000986 | 101405402..101405515 (i1) |
| RBM34 | chr1 (-) | NM_015014 | 235323963..235324207 (i2) |
| TMA7 | chr3 (+) | NM_001329417 | 48481869..48481950 (i2) |
| RPS21 | chr20 (+) | NM_001024 | 60963421..60963512 (i5) |
| RPL41 | chr12 (+) | NM_021104 | 56510444..56510558 (i1) |

| **B. Alternative 5' splice site targets** | | | |
|---|---|---|---|
| gene_id | chromosome (strand) | RefSeq | target exon position (exon #) |
| WDR26 | chr1 (-) | NM_001115113 | 224619227..224619283 (e3) |
| | | | 224619179..224619283 |
| HNRNPC | chr14 (-) | NM_004500 | 21699156..21699231 (e4) |
| | | | 21699117..21699231 |
| SRSF5 | chr14 (+) | NM_001320214 | 70233804..70233972 (e1) |
| | | | 70233804..70234097 |
| RPS15A | chr16 (-) | NM_001030009 | 18801632..18801656 (e1) |
| | | | 18801566..18801656 |
| UQCR10 | chr22 (+) | NM_013387 | 30163358..30163537 (e1) |
| | | | 30163358..30163596 |

| **C. Exon skipping targets** | | | |
|---|---|---|---|
| gene_id | chromosome (strand) | RefSeq | target exon position (exon #) |
| RPL5 | chr1 (+) | NM_000969 | 93298946..93299015 (e2) |
| EIF4G2 | chr11 (-) | NM_001418 | 10823208..10823321 (e14) |
| EIF4G2 | chr11 (-) | NM_001418 | 10828367..10828432 (e3) |
| UBE2L3 | chr22 (+) | NM_003347 | 21947150..21947245 (e2) |
| CHRAC1 | chr8 (+) | NM_017444 | 141524468..141524594 (e2) |

For each of the intron retention cases, in parallel flanking-exon and exon-intron primer combinations were used for validation, producing consistent results: Transfection of AS-U1 circRNAs increased intron retention levels (Figure 3B). Regarding alternative 5' splice site usage, transfection of AS-U1 circRNAs strongly enhanced usage of the proximal (downstream) 5' splice site (Figure 3C, left panel). The strongest effects were observed for the exon skipping examples: In each case tested, skipping of the alternative exon was enhanced after transfection of AS-U1 circRNAs. For example, in the case of two different exons of EIF4G1, skipping levels increased from very low levels (1 and 12) to around 60% (Figure 3C, right panel). In summary, all these 15 examples showed the expected strong splicing effects, and consistently for all of them, the splicing effects correlated with the extent of complementarity, *i.e.,* increased for AS-U1 circRNAs in this order: AS-U1-30, AS-U1-42, AS-U1-2×30, and AS-U1-3×25. Therefore, AS-U1 circRNAs can be applied as a new means to modulate alternative splicing patterns *in vivo.*

### Example 7: Antisense-circRNAs targeting the 5'-UTR of human β-globin mRNA: design, synthesis, transfection, and translation-inhibitory activities.

For testing the applicability of designer AS-circRNAs for modulating gene expression, mRNA translation inhibitory effects by small circRNAs were analysed. AS-circRNAs with RNA sequences directed against the human β-globin (HBB) 5'-UTR were developed. In order to search for optimal β-globin target sequences in the 5'-UTR, a luciferase reporter system was used (as shown in Figure 4A). For this purpose, the complete 5'-UTR sequence of 50 nucleotides (SEQ ID NO: 40) including the translation start codon was fused in-frame with the luciferase ORF.

In this context, five small AS-HBB circRNAs (50 to 62 nucleotides in length) were designed to specifically target different regions of the β-globin reporter (as shown in Figure 4A). The antisense sequences of these circRNAs were between 30 and 42 nucleotides in length.
Circularizable polynucleotide (CP) AS-HBB 3-44 (SEQ ID NO: 55):
   GGGAGTAAGC**TGTTTGAGGTTGCTAGTGAACACAGTTGTGTCAGAAGCAAAT**GCTTACAGTA
Circularizable polynucleotide (CP) AS-HBB 14-44 (SEQ ID NO: 56):
   GGGAGTAAGC**TGTTTGAGGTTGCTAGTGAACACAGTTGTGT**GCTTACAGTA
Circularizable polynucleotide (CP) AS-HBB 17-47 (SEQ ID NO: 57):
   GGGAGTAAGC**GTCTGTTTGAGGTTGCTAGTGAACACAGTTG**GCTTACAGTA
Circularizable polynucleotide (CP) AS-HBB/Luc 45-85 (SEQ ID NO: 58):
   GGGAGTAAGC**GGGCCTTTCTTTATGTTTTTGGCGTCGGATCCCATGGTGTC**GCTTACAGTA
Circularizable polynucleotide (CP) AS-Luc 134-163 (SEQ ID NO: 59):
   GGGAGTAAGC**GGAACCAGGGCGTATCTCTTCATAGCCTTA**GCTTACAGTA
Circularizable polynucleotide (CP) CTR (SEQ ID NO: 60):
   GGGAGTAAGCAGATGCGCACCGCACAGATGCGCACGCTTACAGTA

The nucleotides of the respective sequence to be targeted are indicated by the numbers. For example, "AS-HBB 3-44" is designed to specifically target the region starting from nucleotide 3 to nucleotide 44 of the 5' UTR HBB sequence as shown in SEQ ID NO: 40. Thus, the first three AS-circRNAs (AS-HBB 3-44, AS-HBB 14-44, and AS-HBB 17-47) cover almost the complete β-globin 5'-UTR, and the AS-HBB/Luc 45-85 is directed against the AUG-proximal region with flanking luciferase-ORF sequences. The AS-Luc 134-163 against an internal region of the luciferase ORF. To control and normalize luciferase activities, a non-specific circRNA (CTR) with a randomized sequence of 25 nucleotides was used instead of the specific antisense sequences. In each circRNA, the antisense sequence (shown in bold letters) was flanked by a short stem-loop-forming sequence (underlined) with two overhanging ends, to facilitate *in vitro* circularization.

The AS-circRNAs were produced by *in vitro* T7 transcription and circularization by T4 RNA ligase. Circularization was controlled by RNase R treatment (as shown in Figure 4B).

Translation-inhibitory activities of the AS-HBB circRNAs were analysed in transfected HeLa cells and compared with their respective linear counterparts. To evaluate specificity and dosis dependence of any effects, five different quantities of each of the five synthetic AS-HBB-RNAs were transfected (100-1000 ng of linear/circular RNA; day 2). Luciferase reporter transfection followed one day later (Firefly and Renilla luciferase reporters co-transfection; day 3), and after one more day, luciferase assays were performed (day 4; see flowchart, Figure 4C).

As can be taken from Figure 4, the two most effective circRNAs in the context of the β-globin 5'-UTR (AS-HBB 3-44 and AS-HBB/Luc 45-85) have the longest antisense sequence of 41 nucleotides compared to the other circRNAs tested, suggesting that a longer AS sequence has an advantage by forming a more stable duplex.

Comparing the effects of the various AS-HBB circRNAs, under assay conditions, maximal activities were achieved with 750 to 1000 ng AS-HBB circRNA. Overall, for most targets comparable effects were detected with linear and circular antisense RNAs; only for two target sequences (AS-circRNAs 3-44 and 45-85), a stronger reduction by the respective circRNA could be observed: The AS-circRNA directed against the 5'-terminal region (AS-HBB 3-44) reduced luciferase activity by up to 75% (1.2-times stronger than linear RNA at titration endpoint), and the AS-circRNA directed against the AUG-proximal region (AS-HBB/Luc 45-85) reduced by up to 68% (1.4-times stronger than linear RNA at titration endpoint).

Based on these results, next the question was addressed whether the observed effects of AS-HBB circRNAs 3-44 and 45-85 persist for a longer time compared to linear RNA (as shown in Figure 4D). For this purpose, luciferase assays were performed with cells transfected with linear or circular AS-HBB circRNAs 3-44 and 45-85 (together with the corresponding control), measuring thereafter for five days every 24 hours. In this experimental workflow, DMEM medium with 10% FBS was replaced by DMEM medium with only 2% FBS.

Comparing the durability of the translation-inhibitory effects for circRNA versus linear counterpart showed that the effects of circRNAs persist significantly longer (Figure 4D). Specifically, between one and two days after reporter transfection, the effect was strongest (circRNA: reduction to 31-48% for AS-HBB 3-44 and to 23-47% for AS-HBB/Luc 45-85; linear RNA: reduction to 46-69% for AS-HBB 3-44 and 31-62% for AS-HBB/Luc 45-85). After three days, the effect of the circular RNA leveled off at a reduction by about 20%, whereas the linear RNA had completely lost its effectiveness. This effect was specific for the circular conformation and indicates a durability of the effect over at least three days under our conditions. In summary, it could be concluded that for the human β-globin 5'-UTR that in particular both the 5'-terminal as well as the AUG startcodon proximal regions are accessible to small synthetic AS-circRNAs, resulting in strong translational inhibition of up to 75%.

### Example 8: Design, overexpresssion, and translation-inhibitory activities of antisense-circRNAs targeting the 5'-UTR of human MDM2 mRNA isoforms.

To further confirm that small AS-circRNAs can function in translation regulation, the concept was applied to another human gene, MDM2, which is complex due to at least six different protein isoforms and a long 5'-UTR with two uORFs. Therefore, two luciferase reporter constructs were designed for the respective isoforms, which differ in their 5'-UTR (MDM2_iso1-2, with the two uORFs in a 324 nucleotide 5'-UTR (SEQ ID NO: 10), and MDM2_iso3-6, with a short, 67 nucleotide 5'-UTR (SEQ ID NO: 32) without the uORFs; as shown in Figure 5A (MDM2_iso1-2) and Figure 5B (MDM2_iso3-6), respectively). To test whether the main ORF could be regulated by blocking the uORFs with a corresponding AS-circRNA, also a mutant reporter of MDM2_iso1-2 was included, with mutations in the respective start codons (AUG to AAG) of the first and second uORF. The start codon of the first uORF is located at positions 76 to 78 of the sequence as depicted in SEQ ID NO: 10, and the start codon of the second uORF is located at positions 210 to 212 of the sequence as depicted in SEQ ID NO: 10. The 5'-UTR sequence of the mutant reporter of MDM2_isol-2 is as depicted in SEQ ID NO: 73. All three constructs contain the ORF translation start codon and the first 30 nucleotides of the MDM2 open reading frame (ORF), fused in-frame with the luciferase ORF.

In this context, a series of ten short AS-circRNAs, each 90 nt long and with a 40-nt antisense sequence, which specifically targets the 5'-UTR regions of MDM2 (as shown in Figure 5A and Figure 5B) was designed.
Circularizable polynucleotide (CP) AS-MDM2 1-40 (iso1-2) (SEQ ID NO: 61): 
AACCATGCCGAGTGCGGCCGCAACAGCCAAGCTCGCCGCGGTGCCTCGGTGCGCGCCCCCTACA
AAAACCGCGGTCGGCGTGGACTGTAG
Circularizable polynucleotide (CP) AS-MDM2 57-96 (iso1-2) (SEQ ID NO: 62): 
AACCATGCCGAGTGCGGCCGCAAGGGCTCGGCTTCATGCTCCATCTTTCCGACACACAGGGCCA
AAAACCGCGGTCGGCGTGGACTGTAG
Circularizable polynucleotide (CP) AS-MDM2 128-167 (iso1-2) (SEQ ID NO: 63): 
AACCATGCCGAGTGCGGCCGCAATCCGGGGAGGGACGGTGCTCCTGGCTGCGAAAGCAGCAGGA
AAAACCGCGGTCGGCGTGGACTGTAG
Circularizable polynucleotide (CP) AS-MDM2 190-229 (iso1-2) (SEQ ID NO: 64): 
AACCATGCCGAGTGCGGCCGCAACCCTGGGCCTCGGGGATCATTCCACTCTCCGGGCCAGGGCA
AAAACCGCGGTCGGCGTGGACTGTAG
Circularizable polynucleotide (CP) AS-MDM2 250-289 (iso1-2) (SEQ ID NO: 65): 
AACCATGCCGAGTGCGGCCGCAAGGAGTCGGGGGTCCCTCAAGACTCCCCAGTATCCTTCACGA
AAAACCGCGGTCGGCGTGGACTGTAG
Circularizable polynucleotide (CP) AS-MDM2 279-318 (iso1-2) (SEQ ID NO: 66): 
AACCATGCCGAGTGCGGCCGCAAGCTCCTCACCATCCGGGGTTATCGCGCTTGGAGTCGGGGGA
AAAACCGCGGTCGGCGTGGACTGTAG
Circularizable polynucleotide (CP) AS-MDM2 301-340 (iso1-2) (SEQ ID NO: 67): 
AACCATGCCGAGTGCGGCCGCAATGTTGGTATTGCACATTTGCCTGCTCCTCACCATCCGGGGA
AAAACCGCGGTCGGCGTGGACTGTAG
Circularizable polynucleotide (CP) AS-MDM2 1-40 (iso3-6) (SEQ ID NO: 68): 
AACCATGCCGAGTGCGGCCGCAACGTGCGTCCGTGCCCACAGGTCTACCCTCCAATCGCCACTA
AAAACCGCGGTCGGCGTGGACTGTAG
Circularizable polynucleotide (CP) AS-MDM2 22-61 (iso3-6) (SEQ ID NO: 69): 
AACCATGCCGAGTGCGGCCGCAAGGATCAGCAGAGAAAAAGTGGCGTGCGTCCGTGCCCACAGA
AAAACCGCGGTCGGCGTGGACTGTAG
Circularizable polynucleotide (CP) AS-MDM2 44-83 (iso3-6) (SEQ ID NO: 70): 
AACCATGCCGAGTGCGGCCGCAATGTTGGTATTGCACATTTGCCTGGATCAGCAGAGAAAAAGA
AAAACCGCGGTCGGCGTGGACTGTAG
Circularizable polynucleotide (CP) CTR1 (SEQ ID NO: 71): 
AACCATGCCGAGTGCGGCCGCAAAGATGCGCACCGCACAGATGCGCACCACGCCACGCGTAGAA
AAAACCGCGGTCGGCGTGGACTGTAG
Circularizable polynucleotide (CP) CTR2 (SEQ ID NO: 72):

AACCATGCCGAGTGCGGCCGCAAAGTCAGTGAGTCAGTGTAATACGGGAGGATACCCGCTGTCA
AAAACCGCGGTCGGCGTGGACTGTAG

Each circular RNA was designed such that the targeting sequence (shown in bold letters in the sequences shown in this example) was linked to a common Tornado stem loop. The targeting region and the Tornado stem loop are connected by two flexible linkers (shown in cursive letters in the sequences of this example). In circular RNA AS-MDM2 57-96 (iso1-2) the nucleotide at position 36 of the nucleotide sequence as depicted in SEQ ID NO: 62 is A, and in circular RNA AS-MDM2 279-318 (iso1-2) the nucleotide at position 44 of the nucleotide sequence as depicted in SEQ ID NO: 66 is A for avoiding longer stretches of T nucleotides in order to increase transcription efficiency of polymerase III. Each of CTR1 and CTR2 contains a central 40-nucleotide randomized sequence.

The nucleotides of the target sequence to be targeted are indicated by the numbers. For example, "AS-MDM2 1-40 (iso1-2)" is designed to specifically target the region starting from nucleotide 1 to nucleotide 40 of the 5' UTR sequence of MDM2 isoforms 1 and 2, as shown in SEQ ID NO: 10. Accordingly, both AS-MDM2 1-40 (iso1-2 as well as iso3-6) target the 5'-terminal 40 nucleotides of the respective 5'-UTR. Furthermore, AS-MDM2 57-96 and AS-MDM2 190-229 cover the start codons of the first and second uORF (iso1-2), respectively. The region of the main ORF is covered by AS-MDM2 301-340 (for MDM2_iso1-2) and AS-MDM2 44-83 (for MDM2_iso3-6). Finally, AS-MDM2 128-167 targets an internal region of MDM2_iso1-2, and AS-MDM2 250-289 (iso1-2), AS-MDM2 279-318 (iso1-2), and AS-MDM2 22-61 (iso3-6) are directed against AUG-proximal regions of either isoform. As a control and to normalize the luciferase activities, two non-specific circRNAs (CTR1 and 2) were used, each containing a randomized sequence of 40 nucleotides instead of the antisense sequence.

Each of these AS-circRNAs was transiently overexpressed in HeLa cells by the Tornado system. After one day, the respective MDM2/luciferase reporter construct was transfected, followed -after one more day- by Northern blotting (to validate circRNA overexpression) and luciferase assays (to assess translation inhibition activities; for experimental workflow, as shown in Figure 5C).

Overexpression of all ten AS-circRNAs, specific for the 5'-UTRs of MDM2_iso1-2 or iso3-6, as well as the two control circRNAs, was confirmed by Northern blot analysis, including RNase R treatment, to confirm circularity (Figure 5C). In addition, cell fractionation demonstrated that overexpressed AS-circRNAs localize predominantly in the cytoplasmic compartment (data not shown).

Reporter assays with both MDM2_iso1-2 (WT and mut) and MDM2_iso3-6 5'-UTR constructs demonstrated translation-inhibition activity for the overexpressed AS-MDM2 circRNAs 1-40, 57-96, 128-167, 279-318, 301-340 (iso1-2), and AS-MDM2 circRNAs 1-40, 22-61 and 44-83 (iso3-6), based on luciferase levels reduced to 32-72% (as shown in Figure 5D, left panel).

Comparing wildtype and mutant reporters for 5'-UTR iso1-2, significant differences were observed only for AS-MDM2 190-229 and AS-MDM2 301-340, although of only low extent. Therefore, both uORFs appear not to have a strong impact on translational output in this reporter context. Interestingly, however, when comparing the activities of all AS-circRNAs, a position-dependent effect emerges: Targeting the 5'-terminal region (AS-MDM2 1-40) and the main-ORF proximal region (AS-MDM2 301-340) resulted in the strongest effects (down to 36 and 46% residual level, respectively). The effects of the remaining AS-circRNAs become smaller with increasing distance to the 5'-terminal region (AS-MDM2 57-96 with 57%, AS-MDM2 128-167 with 68% residual level); the effects increase again when moving closer to the AUG-proximal region of the main ORF (AS-MDM2 279-318 with 57% residual level). Combining the two most effective AS-circRNAs (AS-MDM2 1-40 and AS-MDM2 301-340) did not further increase the translation-inhibitory effect above their separate effects.

Regarding the MDM2_iso3-6 reporter, reductions to 60-29% were measured (as shown in Figure 5D, right panel). The AS-circRNA 1-40 had an intermediate effect (60% residual level), whereas the two circRNAs covering the AUG-proximal (AS-MDM2 22-61) and the AUG-overlapping regions (AS-MDM2 44-83) showed the strongest effects with reductions to 29% residual level.

In conclusion, based on two separate MDM2 reporters and a series of ten AS-circRNAs, the results indicate that in particular both the 5'-terminal as well as the AUG-proximal regions can be efficiently targeted by AS-circRNAs, resulting in strong translational inhibition down to residual levels of approximately 30%.

### Example 9: Northern Blot and RNase R digestion

For Northern blot detection of the expression of Tornado-derived circRNAs, 500 ng of total RNA were analyzed by 10% denaturing PAGE (Figures 2B, 5C, and Figure 6). For transfected circRNAs, 2 µg were loaded (Figure 7). Transfer to nylon membrane (Carl Roth) was performed for one hour at 15 V (0.3 mA/cm²). After UV-crosslinking (254 nm; 120 mJ/cm²), the membrane was hybridized with a DIG-UTP-labeled riboprobe (DIG RNA labeling Mix, Roche) in NorthernMax hybridization buffer (Thermo Fisher Scientific) overnight at 60°C. For expressed antisense-U1 circRNAs, a U1 probe (against nucleotides 1-30) was used (Figure 7), for expressed antisense-MDM2 circRNAs, a circular-junction-specific probe (Figure 5C). Further steps were performed following the standard procedure for Northern Blot (Schneider, T., et al., Methods Mol Biol. 1724 (2018): 119-133), using alkaline phosphatase-conjugated anti-DIG Fab fragments, and CDP-Star chemiluminescence (Roche). As a loading control, endogenous U1 snRNA was detected by an antisense-riboprobe.

To confirm circularity of the Tornado-expressed circRNAs (Figure 5C and Figure 6), 1 U of RNase R was used to digest 1 µg of total RNA (30 min at 37°C). 500 ng digested or control-undigested RNA were loaded onto a 10% denaturing polyacrylamide gel. For Northern blot detection, the U1-30 nucleotide antisense probe was used (20 ng/ml). As a control for RNase R, the untreated RNA was loaded on a 1 × MOPS agarose gel and ethidium bromide-stained.

### Example 10: Transfection of in vitro-transcribed RNAs and Tornado-based circRNA expression constructs; luciferase reporter assays

For luciferase reporter assays, 1 × 10⁵ HeLa cells were seeded per well (12-well plates). RNA transfections were done using Lipofectamine 2000, Tornado-plasmid transfections with Turbofect reagent, both in a total volume of 1 ml medium per well (Thermo Fisher Scientific). For titration experiments, different amounts (100, 250, 500, 750, or 1000 ng per assay) of circular or corresponding linear RNAs were used.

For durability assays, 1 × 10⁵ HeLa cells were seeded per well (12-well plates) in DMEM-medium supplemented with 10% FBS (Gibco) one day before transfection. After transfection of RNA, the normal medium was exchanged by DMEM-medium supplemented with 2% FBS (Gibco).

For Tornado-circRNA screening, cells were transfected with 1 µg of plasmid DNA. After one day, cells were co-transfected with 50 ng of 5'-UTR luciferase reporter plasmids, together with 5 ng of pRL-SCV40 Renilla-reporter (Promega). At 24 h post-transfection, cells were washed three times with PBS (Gibco), and lysed in 250 µl Lysis-Juice (PJK). Luminescence was measured for Firefly and Renilla luciferase (Beetle- and Renilla-Juice kits, respectively; PJK). Relative luciferase activities were calculated as ratios of the Firefly and Renilla raw values, with three technical replicates per sample and a total of three independent biological replicates.

**TABLE OF SEQUENCES**

| **SEQ ID NO** | **Name** | **Sequence** |
|---|---|---|
| 1 | human U1 snRNA | ATACTTACCTGGCAGGGGAGATACCATGATCACGAAGGTGGTTTTCCC AGGGCGAGGCTTATCCATTGCACTCCGGATGTGCTGACCCCTGCGATT |
| | | TCCCCAAATGTGGGAAACTCGACTGCATAATTTGTGGTAGTGGGGGAC TGCGTTCGCGCTTTCCCCTG |
| 2 | AS-U1-11 | CAGGTAAGTAT |
| 3 | AS-U1-20 | CTCCCCTGCCAGGTAAGTAT |
| 4 | AS-U1-22 | ATCTCCCCTGCCAGGTAAGTAT |
| 5 | AS-U1-30 | ATCATGGTATCTCCCCTGCCAGGTAAGTAT |
| 6 | AS-U1-42 | ACCACCTTCGTGATCATGGTATCTCCCCTGCCAGGTAAGTAT |
| 7 | AS-U1-3×25 | GGTATCTCCCCTGCCAGGTAAGTATATATGGTATCTCCCCTGCCAGGT AAGTATATATGGTATCTCCCCTGCCAGGTAAGTAT |
| 8 | AS-U1-2×30 | ATCATGGTATCTCCCCTGCCAGGTAAGTATAATTAAATCATGGTATCT CCCCTGCCAGGTAAGTAT |
| 9 | AS-U1-3×30 | ATCATGGTATCTCCCCTGCCAGGTAAGTATAATTAAATCATGGTATCT CCCCTGCCAGGTAAGTATAATTAAATCATGGTATCTCCCCTGCCAGGT AAGTAT |
| 10 | 5' UTR iso1-2 of the MDM2 (mouse double minute 2 homolog) mRNA | GTAGGGGGCGCGCACCGAGGCACCGCGGCGAGCTTGGCTGCTTCTGGG GCCTGTGTGGCCCTGTGTGTCGGAAAGATGGAGCAAGAAGCCGAGCCC GAGGGGCGGCCGCGACCCCTCTGACCGAGATCCTGCTGCTTTCGCAGC CAGGAGCACCGTCCCTCCCCGGATTAGTGCGTACGAGCGCCCAGTGCC CTGGCCCGGAGAGTGGAATGATCCCCGAGGCCCAGGGCGTCGTGCTTC CGCGCGCCCCGTGAAGGAAACTGGGGAGTCTTGAGGGACCCCCGACTC CAAGCGCGAAAACCCCGGATGGTGAGGAGCAGGCAA |
| 11 | AS-MDM2 22-40 (iso1-2) | CAGCCAAGCTCGCCGCGGT |
| 12 | AS-MDM2 10-40 (iso1-2) | CAGCCAAGCTCGCCGCGGTGCCTCGGTGCGC |
| 13 | AS-MDM2 1-40 (iso1-2) | CAGCCAAGCTCGCCGCGGTGCCTCGGTGCGCGCCCCCTAC |
| 14 | AS-MDM2 22-50 (iso1-2) | GCCCCAGAAGCAGCCAAGCTCGCCGCGGT |
| 15 | AS-MDM2 22-60 (iso1-2) | GGCCACACAGGCCCCAGAAGCAGCCAAGCTCGCCGCGGT |
| 16 | AS-MDM2 67-86 (iso1-2) | TCNTGCTCCATCTTTCCGAC |
| | | wherein N is A or C or G or T/U, preferably A or T/U, more preferably A |
| 17 | AS-MDM2 57-86 (iso1-2) | TCNTGCTCCATCTTTCCGACACACAGGGCC |
| | | wherein N is A or C or G or T/U, preferably A or T/U, more preferably A |
| 18 | AS-MDM2 47-86 (iso1-2) | TCNTGCTCCATCTTTCCGACACACAGGGCCACACAGGCCC |
| | | wherein N is A or C or G or T/U, preferably A or T/U, more preferably A |
| 19 | AS-MDM2 67-96 (iso1-2) | GGGCTCGGCTTCNTGCTCCATCTTTCCGAC |
| | | wherein N is A or C or G or T/U, preferably A or T/U, more preferably A |
| 20 | AS-MDM2 67-106 (isol-2) | GCCGCCCCTCGGGCTCGGCTTCNTGCTCCATCTTTCCGAC |
| | | wherein N is A or C or G or T/U, preferably A or T/U, more preferably A |
| 21 | AS-MDM2 57-96 (iso1-2) | GGGCTCGGCTTCNTGCTCCATCTTTCCGACACACAGGGCC |
| | | wherein N is A or C or G or T/U, preferably A or T/U, more preferably A |
| 22 | AS-MDM2 138-157 (iso1-2) | GACGGTGCTCCTGGCTGCGA |
| 23 | AS-MDM2 128-157 (iso1-2) | GACGGTGCTCCTGGCTGCGAAAGCAGCAGG |
| 24 | AS-MDM2 118-157 (iso1-2) | GACGGTGCTCCTGGCTGCGAAAGCAGCAGGATCTCGGTCA |
| 25 | AS-MDM2 138-157 (iso1-2) | TCCGGGGAGGGACGGTGCTCCTGGCTGCGA |
| 26 | AS-MDM2 138-177 (iso1-2) | TACGCACTAATCCGGGGAGGGACGGTGCTCCTGGCTGCGA |
| 27 | AS-MDM2 128-167 (iso1-2) | TCCGGGGAGGGACGGTGCTCCTGGCTGCGAAAGCAGCAGG |
| 28 | AS-MDM2 301-318 (iso1-2) | GCTCCTCACCATCCGGGG |
| 29 | AS-MDM2 279-318 (iso1-2) | GCTCCTCACCATCCGGGGTTNTCGCGCTTGGAGTCGGGGG |
| | | wherein N is A or C or G or T/U, preferably A or T/U, more preferably A |
| 30 | AS-MDM2 269-318 (iso1-2) | GCTCCTCACCATCCGGGGTTNTCGCGCTTGGAGTCGGGGGTCCCTCAA GA |
| | | wherein N is A or C or G or T/U, preferably A or T/U, more preferably A |
| 31 | AS-MDM2 301-324 (iso1-2) | TTGCCTGCTCCTCACCATCCGGGG |
| 32 | 5' UTR iso3-6 of the MDM2 (mouse double minute 2 homolog) mRNA | AGTGGCGATTGGAGGGTAGACCTGTGGGCACGGACGCACGCCACTTTT TCTCTGCTGATCCAGGCAA |
| 33 | AS-MDM2 22-40 (iso3-6) | CGTGCGTCCGTGCCCACAG |
| 34 | AS-MDM2 10-40 (iso3-6) | CGTGCGTCCGTGCCCACAGGTCTACCCTCCA |
| 35 | AS-MDM2 1-40 (iso3-6) | CGTGCGTCCGTGCCCACAGGTCTACCCTCCAATCGCCACT |
| 36 | AS-MDM2 22-50 (iso3-6) | GAAAAAGTGGCGTGCGTCCGTGCCCACAG |
| 37 | AS-MDM2 22-61 (iso3-6) | GGATCAGCAGAGAAAAAGTGGCGTGCGTCCGTGCCCACAG |
| 38 | AS-MDM2 44-67 (iso3-6) | TTGCCTGGATCAGCAGAGAAAAAG |
| 39 | AS-MDM2 32-67 (iso3-6) | TTGCCTGGATCAGCAGAGAAAAAGACCGCACGCA |
| 40 | 5' UTR of the human beta-globin (HBB) mRNA | ACATTTGCTTCTGACACAACTGTGTTCACTAGCAACCTCAAACAGACA CC |
| 41 | AS-HBB 17-44 | TGTTTGAGGTTGCTAGTGAACACAGTTG |
| 42 | AS-HBB 10-44 | TGTTTGAGGTTGCTAGTGAACACAGTTGTGTCAGA |
| 43 | AS-HBB 1-44 | TGTTTGAGGTTGCTAGTGAACACAGTTGTGTCAGAAGCAAATGT |
| 44 | AS-HBB 17-50 | GGTGTCTGTTTGAGGTTGCTAGTGAACACAGTTG |
| 45 | AS-HBB 3-44 | TGTTTGAGGTTGCTAGTGAACACAGTTGTGTCAGAAGCAAAT |
| 46 | AS-HBB 14-44 | TGTTTGAGGTTGCTAGTGAACACAGTTGTGT |
| 47 | AS-HBB 17-47 | GTCTGTTTGAGGTTGCTAGTGAACACAGTTG |
| 48 | CP AS-U1-11 | GGGAGTAAGC**CAGGTAAGTAT**GCTTACAGTA |
| 49 | CP AS-U1-22 | GGGAGTAAGC**ATCTCCCCTGCCAGGTAAGTAT**GCTTACAGTA |
| 50 | CP AS-U1-30 | GGGAGTAAGCATCATGGTATCTCCCCTGCCAGGTAAGTATGCTTACAG TA |
| 51 | CP AS-U1-42 | GGGAGTAAGCACCACCTTCGTGATCATGGTATCTCCCCTGCCAGGTAA GTATGCTTACAGTA |
| 52 | CP AS-U1-2x30 | GGGAGTAAGCATCATGGTATCTCCCCTGCCAGGTAAGTATAATTAAAT CATGGTATCTCCCCTGCCAGGTAAGTATGCTTACAGTA |
| 53 | CP AS-U1-3x25 | GGGAGTAAGCGGTATCTCCCCTGCCAGGTAAGTATATATGGTATCTCC CCTGCCAGGTAAGTATATATGGTATCTCCCCTGCCAGGTAAGTATGCT TACAGTA |
| 54 | CP CTR | GGGAGTAAGC**CCTGCCTGTCTATTGATGTC**GCTTACAGTA |
| 55 | CP AS-HBB 3-44 | GGGAGTAAGCTGTTTGAGGTTGCTAGTGAACACAGTTGTGTCAGAAGC AAATGCTTACAGTA |
| 56 | CP AS-HBB 14-44 | GGGAGTAAGCTGTTTGAGGTTGCTAGTGAACACAGTTGTGTGCTTACA GTA |
| 57 | CP AS-HBB 17-47 | GGGAGTAAGCGTCTGTTTGAGGTTGCTAGTGAACACAGTTGGCTTACA GTA |
| 58 | CP AS-HBB/Luc 45-85 | GGGAGTAAGCGGGCCTTTCTTTATGTTTTTGGCGTCGGATCCCATGGT GTCGCTTACAGTA |
| 59 | CP AS-Luc 134-163 | GGGAGTAAGCGGAACCAGGGCGTATCTCTTCATAGCCTTAGCTTACAG TA |
| 60 | CP CTR | GGGAGTAAGC**AGATGCGCACCGCACAGATGCGCAC**GCTTACAGTA |
| 61 | CP AS-MDM2 1-40 (iso1-2) | AACCATGCCGAGTGCGGCCGCAACAGCCAAGCTCGCCGCGGTGCCTCG GTGCGCGCCCCCTACAAAAACCGCGGTCGGCGTGGACTGTAG |
| 62 | CP AS-MDM2 57-96 (iso1-2) | AACCATGCCGAGTGCGGCCGCAAGGGCTCGGCTTCATGCTCCATCTTT CCGACACACAGGGCCAAAAACCGCGGTCGGCGTGGACTGTAG |
| 63 | CP AS-MDM2 128-167 (iso1-2) | AACCATGCCGAGTGCGGCCGCAATCCGGGGAGGGACGGTGCTCCTGGC TGCGAAAGCAGCAGGAAAAACCGCGGTCGGCGTGGACTGTAG |
| 64 | CP AS-MDM2 190-229 (iso1-2) | AACCATGCCGAGTGCGGCCGCAACCCTGGGCCTCGGGGATCATTCCAC TCTCCGGGCCAGGGCAAAAACCGCGGTCGGCGTGGACTGTAG |
| 65 | CP AS-MDM2 250-289 (iso1-2) | AACCATGCCGAGTGCGGCCGCAAGGAGTCGGGGGTCCCTCAAGACTCC CCAGTATCCTTCACGAAAAACCGCGGTCGGCGTGGACTGTAG |
| 66 | CP AS-MDM2 279-318 (iso1-2) | AACCATGCCGAGTGCGGCCGCAAGCTCCTCACCATCCGGGGTTATCGC GCTTGGAGTCGGGGGAAAAACCGCGGTCGGCGTGGACTGTAG |
| 67 | CP AS-MDM2 301-340 (iso1-2) | AACCATGCCGAGTGCGGCCGCAATGTTGGTATTGCACATTTGCCTGCT CCTCACCATCCGGGGAAAAACCGCGGTCGGCGTGGACTGTAG |
| 68 | CP AS-MDM2 1-40 (iso3-6) | AACCATGCCGAGTGCGGCCGCAACGTGCGTCCGTGCCCACAGGTCTAC CCTCCAATCGCCACTAAAAACCGCGGTCGGCGTGGACTGTAG |
| 69 | CP AS-MDM2 22-61 (iso3-6) | AACCATGCCGAGTGCGGCCGCAAGGATCAGCAGAGAAAAAGTGGCGTG CGTCCGTGCCCACAGAAAAACCGCGGTCGGCGTGGACTGTAG |
| 70 | CP AS-MDM2 44-83 (iso3-6) | AACCATGCCGAGTGCGGCCGCAATGTTGGTATTGCACATTTGCCTGGA TCAGCAGAGAAAAAGAAAAACCGCGGTCGGCGTGGACTGTAG |
| 71 | CP CTR1 | AACCATGCCGAGTGCGGCCGCAAAGATGCGCACCGCACAGATGCGCAC CACGCCACGCGTAGAAAAAACCGCGGTCGGCGTGGACTGTAG |
| 72 | CP CTR2 | AACCATGCCGAGTGCGGCCGCAAAGTCAGTGAGTCAGTGTAATACGGG AGGATACCCGCTGTCAAAAACCGCGGTCGGCGTGGACTGTAG |
| 73 | 5' UTR iso1-2 of the MDM2 mRNA mutated | GTAGGGGGCGCGCACCGAGGCACCGCGGCGAGCTTGGCTGCTTCTGGG GCCTGTGTGGCCCTGTGTGTCGGAAAGAAGGAGCAAGAAGCCGAGCCC GAGGGGCGGCCGCGACCCCTCTGACCGAGATCCTGCTGCTTTCGCAGC CAGGAGCACCGTCCCTCCCCGGATTAGTGCGTACGAGCGCCCAGTGCC CTGGCCCGGAGAGTGGAAAGATCCCCGAGGCCCAGGGCGTCGTGCTTC CGCGCGCCCCGTGAAGGAAACTGGGGAGTCTTGAGGGACCCCCGACTC CAAGCGCGAAAACCCCGGATGGTGAGGAGCAGGCAA |
| 74 | human U1 snRNA; partial sequence | AUACUUACCUGGCAGGGGAGAUACCAUGAUCACGAAGGUGGUUUUCC |

## Claims

1. A circular polynucleotide comprising a nucleotide sequence, which sequence comprises a targeting region comprising or consisting of a nucleotide sequence complementary to a target structure which is involved in shaping the cellular proteome, wherein
the target structure is involved in pre-mRNA splicing and the circular polynucleotide is capable of at least one of:
(i) inhibiting pre-mRNA splicing;
(ii) resulting in intron retention;
(iii) resulting in alternative 5' splice site use; and/or
(iv) resulting in single-exon skipping, and
the nucleotide sequence comprises a targeting region comprising or consisting of a nucleotide sequence complementary to a U1 snRNA or to a continuous or discontinuous portion of said U1 snRNA.

2. The polynucleotide of claim 1, wherein the nucleotide sequence of the targeting region is complementary to at least nucleotides 1 to 20, starting from the 5' end of the U1 snRNA nucleotide sequence,

3. The polynucleotide of claim 2, wherein
(i) the nucleotide sequence of the targeting region complementary to at least the nucleotides 1 to 20 is flanked 5' by one or more additional nucleotides for targeting the nucleotide at position 21 up to at most to the nucleotide at position 100, up to at most to the nucleotide at position 50, up to at most to the nucleotide at position 42, or up to at most to the nucleotide at position 30 of the U1 snRNA nucleotide sequence; and/or
(ii) the nucleotide sequence of the targeting region is depicted in any one of SEQ ID NO: 3 to SEQ ID: 6.

4. The polynucleotide of any one of claims 1 to 3, wherein the targeting region of the polynucleotide comprises two or more copies, preferably two or three copies, of the nucleotide sequence complementary to a U1 snRNA or to a continuous or discontinuous portion of said U1 snRNA.

5. The polynucleotide of claim 4, wherein the nucleotide sequence complementary to a U1 snRNA or to a continuous or discontinuous portion of said U1 snRNA comprises
(i) a nucleotide sequence which is complementary to at least nucleotides 1 to 20 of the U1 snRNA nucleotide sequence,
wherein preferably the nucleotide sequence complementary to at least the nucleotides 1 to 20 is flanked 5' by one or more additional nucleotides for targeting the nucleotide at position 21 up to at most to the nucleotide at position 50 of the U1 snRNA nucleotide sequence;
(ii) a nucleotide sequence which is complementary to at least nucleotides 1 to 25 of the U1 snRNA nucleotide sequence,
wherein preferably the nucleotide sequence complementary to at least the nucleotides 1 to 25 is flanked 5' by one or more additional nucleotides for targeting the nucleotide at position 26 up to at most to the nucleotide at position 50 of the U1 snRNA nucleotide sequence;
(iii) the nucleotide sequence of the continuous portion is depicted in any one of SEQ ID NO: 3 to SEQ ID: 6; and/or
(iv) the nucleotide sequence of the targeting region is depicted in any one of SEQ ID NO: 7 to SEQ ID: 9.

6. The polynucleotide of any one of claims 1 to 5, wherein the nucleotide sequence complementary to a U1 snRNA or to a continuous or discontinuous portion of said U1 snRNA is complementary to at least one continuous portion of the U1 snRNA, the continuous portion being selected from the group consisting of:
(i) nucleotides 1 to 20 of the nucleotide sequence of the U1 snRNA;
(ii) nucleotides 1 to 22 of the nucleotide sequence of the U1 snRNA;
(iii) nucleotides 1 to 25 of the nucleotide sequence of the U1 snRNA;
(iv) nucleotides 1 to 26 of the nucleotide sequence of the U1 snRNA;
(v) nucleotides 1 to 30 of the nucleotide sequence of the U1 snRNA;
(vi) nucleotides 1 to 40 of the nucleotide sequence of the U1 snRNA;
(vii) nucleotides 1 to 42 of the nucleotide sequence of the U1 snRNA; and
(viii) nucleotides 1 to 50 of the nucleotide sequence of the U1 snRNA.

7. A linear or circular polynucleotide comprising a nucleotide sequence, which sequence comprises a targeting region comprising or consisting of a nucleotide sequence complementary to a target structure which is involved in shaping the cellular proteome, wherein
the target structure is involved in mRNA translation and the polynucleotide is capable of inhibiting mRNA translation, and
the nucleotide sequence comprises a targeting region comprising or consisting of a nucleotide sequence complementary to a 5' untranslated region (5'-UTR) or to a continuous or discontinuous portion of said 5' untranslated region of the mouse double minute 2 homolog (MDM2) mRNA.

8. The polynucleotide of claim 7, wherein the nucleotide sequence of the 5'-UTR is depicted in SEQ ID NO: 10, and the targeting region is complementary to
(i) at least the nucleotides 22 to 40 of the 5'-UTR nucleotide sequence,
wherein preferably the nucleotide sequence of the targeting region complementary to at least nucleotides 22 to 40 of the 5'-UTR depicted in SEQ ID NO: 10 is flanked 3' by one or more additional nucleotides for targeting the nucleotide at position 21 up to at most to the nucleotide at position 1 of the 5'-UTR sequence;
(ii) at least the nucleotides 67 to 86 of the 5'-UTR nucleotide sequence, wherein preferably the nucleotide sequence of the targeting region complementary to at least nucleotides 67 to 86 of the 5'-UTR depicted in SEQ ID NO: 10 is flanked 3' by one or more additional nucleotides for targeting the nucleotide at position 66 up to at most to the nucleotide at position 57 of the 5'-UTR sequence, and/or
wherein preferably the nucleotide sequence of the targeting region complementary to at least nucleotides 67 to 86 of the 5'-UTR depicted in SEQ ID NO: 10 is flanked 5' by one or more additional nucleotides for targeting the nucleotide at position 87 up to at most to the nucleotide at position 96 of the 5'-UTR sequence;
(iii) at least the nucleotides 138 to 157 of the 5'-UTR nucleotide sequence, wherein preferably the nucleotide sequence of the targeting region complementary to at least nucleotides 138 to 157 of the 5'-UTR depicted in SEQ ID NO: 10 is flanked 3' by one or more additional nucleotides for targeting the nucleotide at position 137 up to at most to the nucleotide at position 128 of the 5'-UTR sequence, and/or
wherein preferably the nucleotide sequence of the targeting region complementary to at least nucleotides 138 to 157 of the 5'-UTR depicted in SEQ ID NO: 10 is flanked 5' by one or more additional nucleotides for targeting the nucleotide at position 158 up to at most to the nucleotide at position 167 of the 5'-UTR sequence;
(iv) at least the nucleotides 301 to 318 of the 5'-UTR nucleotide sequence, wherein preferably the nucleotide sequence of the targeting region complementary to at least nucleotides 301 to 318 of the 5'-UTR depicted in SEQ ID NO: 10 is flanked 3' by one or more additional nucleotides for targeting the nucleotide at position 300 up to at most to the nucleotide at position 279 of the 5'-UTR sequence, and/or
wherein preferably the nucleotide sequence of the targeting region complementary to at least nucleotides 301 to 318 of the 5'-UTR depicted in SEQ ID NO: 10 is flanked 5' by one or more additional nucleotides for targeting the nucleotide at position 319 up to at most to the nucleotide at position 324 of the 5'-UTR sequence; or
(v) at least one continuous portion of the 5' untranslated region, the continuous portion being selected from the group consisting of:
- nucleotides 1 to 40 of the sequence depicted in SEQ ID NO: 10;
- nucleotides 57 to 96 of the sequence depicted in SEQ ID NO: 10;
- nucleotides 128 to 167 of the sequence depicted in SEQ ID NO: 10
- nucleotides 279 to 318 of the sequence depicted in SEQ ID NO: 10; and
- nucleotides 301 to 324 of the sequence depicted in SEQ ID NO: 10.

9. The polynucleotide of claim 8, wherein the nucleotide sequence of the targeting region is depicted in any one of SEQ ID NO: 11 to SEQ ID NO: 31, or wherein the nucleotide sequence of the targeting region comprises the sequences as depicted in SEQ ID NO: 31, wherein the sequence is additionally flanked 5' in a contiguous manner by one or more nucleotides for targeting nucleotide +1 up to at most nucleotide +26, preferably up to at most nucleotide +16 of the main ORF.

10. The polynucleotide of claim 7, wherein the nucleotide sequence of the 5'-UTR is depicted in SEQ ID NO: 32, and wherein the targeting region is complementary to
(i) at least the nucleotides 22 to 40 of the 5'-UTR nucleotide sequence,
wherein preferably the nucleotide sequence of the targeting region complementary to at least nucleotides 22 to 40 of the 5'-UTR depicted in SEQ ID NO: 32 is flanked 3' by one or more additional nucleotides for targeting the nucleotide at position 21 up to at most to the nucleotide at position 1 or up to at most the nucleotide at position 10 of the 5'-UTR sequence, and/or
wherein preferably the nucleotide sequence of the targeting region complementary to at least nucleotides 22 to 40 of the 5'-UTR depicted in SEQ ID NO: 32 is flanked 5' by one or more additional nucleotides for targeting the nucleotide at position 41 up to at most to the nucleotide at position 61 of the 5'-UTR sequence;
(ii) at least the nucleotides 44 to 67 of the 5'-UTR nucleotide sequence; or
(iii) at least one continuous portion of the 5' untranslated region, the continuous portion being selected from the group consisting of:
- nucleotides 1 to 40 of the sequence depicted in SEQ ID NO: 32;
- nucleotides 22 to 61 of the sequence depicted in SEQ ID NO: 32; and
- nucleotides 44 to 67 of the sequence depicted in SEQ ID NO: 32.

11. The polynucleotide of claim 10, wherein the nucleotide sequence of the targeting region is depicted in any one of SEQ ID NO: 33 to SEQ ID: 39, and/or wherein the nucleotide sequence of the targeting region comprises the sequences as depicted in SEQ ID NO: 38, wherein the sequence is additionally flanked 5' in a contiguous manner by one or more nucleotides for targeting nucleotide +1 up to at most nucleotide +26, preferably up to at most nucleotide +16 of the ORF.

12. The polynucleotide of any one of claims 1 to 11, wherein
(i) the nucleotide sequence of the targeting region has at least 70%, at least 75%, at least 80%, at least 85%, at least 90%, at least 95%, at least 97%, or at least 99% identity to the sequence depicted in any one of SEQ ID NO: 3 to 8, 15 to 17, 20, 21, 23 to 25, respectively;
(ii) the nucleotide sequence of the targeting region is at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% complementary to the U1 snRNA or the 5' untranslated region or a continuous or discontinuous portion thereof as defined in any one of claims 22, 25, 48, 50 and 63;
(iii) the nucleotide sequence of the targeting region is 100% complementary to the U1 snRNA or the 5'-untranslated region or a continuous or discontinuous portion thereof;
(iv) the nucleotide sequence of the targeting region is at least 15 nucleotides, at least 20 nucleotides, or at least 25 nucleotides in length;
(v) the nucleotide sequence of the targeting region is at most 500 nucleotides, at most 250 nucleotides, or at most 150 nucleotides in length;
(vi) the polynucleotide comprises at least one ribonucleotide, or the polynucleotide consists of ribonucleotides;
(vii) the polynucleotide comprises at least one deoxyribonucleotide;
(viii) the polynucleotide comprises a mixture of ribonucleotides and deoxyribonucleotides; and/or
(ix) the polynucleotide comprises at least one modified nucleotide.

13. A pharmaceutical composition comprising one or more of the polynucleotides of any one of claims 1 to 12, and a pharmaceutically acceptable carrier.

14. A pharmaceutical composition of claim 13 for use as a medicament.

15. A polynucleotide of any one of claims 1 to 12 or a pharmaceutical composition of claim 14 for use in a method of treating or preventing a proliferative disorder, preferably cancer, said method comprising a step of administering to a patient in need thereof one or more of the polynucleotides or the pharmaceutical composition.
